# EUROPEAN PATENT APPLICATION

(11) **EP 4 265 620 A1**
(43) Date of publication of application: **25.10.2023**
(21) Application number: 21902686.1
(22) Date of filing: 09.12.2021
(51) Int. Cl.: C07D 495/22, C07D 491/22, A61K 31/4743, A61K 31/4745, A61K 39/395, A61P 35/00

(54) **NOVEL CAMPTOTHECIN DERIVATIVE, COMPOSITION CONTAINING SAME, AND USE THEREOF**

(30) Priority: 11.12.2020 CN 202011463704
(71) Applicant: Wigen Biomedicine Technology (Shanghai) Co., Ltd., Shanghai 201203 (CN)
(72) Inventor: XIE, Yuli, Shanghai 201203 (CN); CAO, Gang, Shanghai 201203 (CN); FAN, Houxing, Shanghai 201203 (CN); QIAN, Lihui, Shanghai 201203 (CN)
(74) Representative: Lipscombe, Martin John
(86) International application number: PCT/CN2021/136769
(87) International publication number: WO 2022/121981

(57) **Abstract**

The present invention provides a camptothecin derivative, a composition comprising the same and use thereof. Specifically, the present invention provides a compound of formula (1) and a preparation method therefor, and use of the compound of general formula (1) and optical isomers, crystalline forms and pharmaceutically acceptable salts thereof in the preparation of a medicament for treating cancer.

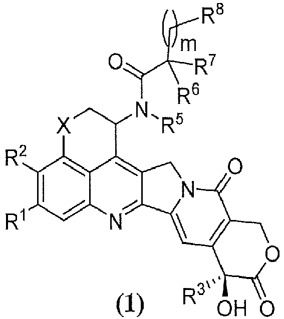

## Description

The present application claims priority to Chinese Patent Application No. 2020114637044 filed on Dec. 11, 2020, which is incorporated herein by reference in its entirety.

### TECHNICAL FIELD

The present invention relates to the field of pharmaceutical chemistry, and in particular to a novel camptothecin derivative, a composition comprising the same and use thereof.

### BACKGROUND

DNA topoisomerase is located in the nucleus of the cell with DNA as a substrate, and is then involved in the replication, transcription and mitosis of the cell. The main role of topoisomerase is to break down the supercoiled structure of DNA. Topoisomerases are classified into topoisomerase I (Topo I) and topoisomerase II (Topo II). The inhibition of topoisomerases leads to the accumulation of large amounts of broken DNA in tumor cells, thereby inducing tumor cell death. The DNA topoisomerase I inhibitor comprises camptothecin and derivatives thereof, and is clinically used for the treatment of malignant tumors.

Camptothecin is firstly separated from Camptotheca acuminata Decne (Nyssaceae). It has relatively strong cytotoxicity, and has good therapeutic effects on malignant tumors such as digestive system tumors (gastric cancer, colon cancer, and rectal cancer), liver cancer, breast cancer, bladder cancer and leukemia. Camptothecin has the main disadvantages of relatively poor solubility and stability and high toxicity, so that its use in clinical application is limited. The camptothecin derivatives can increase water solubility by introducing a water-soluble group or preparing a prodrug, thereby improving the druggability. A number of camptothecin derivatives with greatly enhanced solubility have been marketed, such as topotecan and its carbamate prodrug irinotecan.

In addition to being used as chemotherapeutic drugs to treat tumors, camptothecin derivatives are also used to be conjugated to antibodies as small molecule toxins (payload) for antibody-drug conjugates (ADCs). The ADCs conjugate the antibodies with small molecule toxins. They have both the specificity of the antibodies for binding to the surface antigen of the tumor cells and the high activity of the cytotoxic drugs for inhibiting and killing the tumor cells. Compared with the traditional chemotherapy drugs, the ADCs can kill the tumor cells more accurately, and thus the influence on normal cells is reduced. In recent years, ADCs that select camptothecin derivatives as small molecule toxins have been developed greatly. DS-8201a (Trastuzumab Deruxtecan) is the first ADC developed by Daiichi Sankyo in Japan and has been approved for marketing. The ADC selects a camptothecin derivative deruxtecan as a small molecule toxin, and takes GGFG tetrapeptide, which can be hydrolyzed by cathepsin B and a self-cleavage structure as linkers.

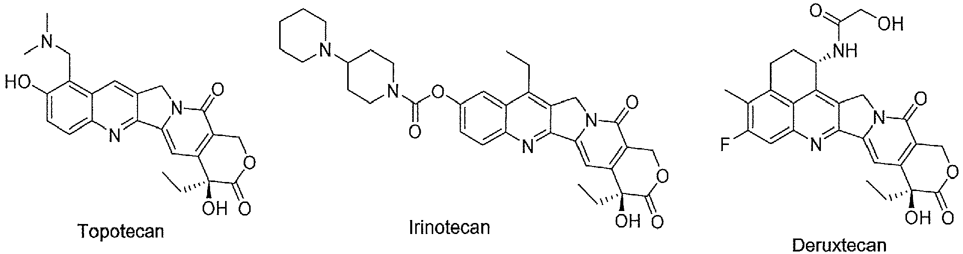

However, ADCs that use camptothecin derivatives as small molecule toxins typically require a relatively large drug-to-antibody ratio (DAR), are difficult to produce, and are prone to instability. Therefore, novel camptothecin derivatives with a higher activity have a wide application prospect as anti-tumor drugs or small molecule toxins of ADCs. Compared with the known compounds such as deruxtecan, the novel camptothecin derivative provided in the present invention has greatly improved cell activity, which has important significance for the development of novel anti-tumor drugs and ADCs.

### SUMMARY

The present invention provides a camptothecin derivative compound of general formula (1) or optical isomers, crystalline forms, pharmaceutically acceptable salts, hydrates or solvates thereof: 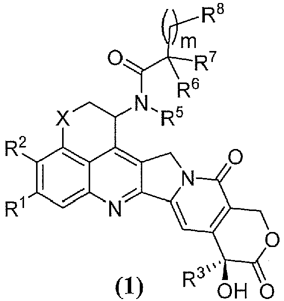 wherein, in general formula (1):
m is an integer of 0, 1 or 2;
X is selected from -O-, -S-, -S(O)-, -S(O₂)-, and -N(R⁴)-;
R¹ and R² are independently selected from H, halogen, OH, C₁₋₆ alkyl, C₁₋₆ alkoxy, C₁₋₆ haloalkyl,
C₃₋₆ cycloalkyl, NH₂, NO₂, and CN, or R¹ and R², together with the phenyl ring attached thereto, form 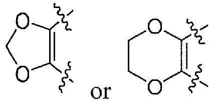 by cyclization;
R³ is selected from C₁₋₆ alkyl, C₂₋₆ alkenyl, C₁₋₃ alkoxy-substituted C₁₋₃ alkyl, and C₁₋₆ haloalkyl;
R⁴ is selected from H, C₁₋₆ alkyl and C₁₋₆ haloalkyl;
R⁵ is selected from H, C₁₋₆ alkyl and C₃₋₆ cycloalkyl;
R⁶ and R⁷ are independently selected from H, C₁₋₆ alkyl, C₁₋₆ haloalkyl, and C₃₋₆ cycloalkyl; or
R⁶ and R⁷, together with the carbon atom attached thereto, form C₃₋₆ cycloalkyl or 4-7 membered heterocycloalkyl by cyclization; or R⁶ and R⁵ are linked to form a 5-7 membered lactam ring, and R⁷ is selected from H, C₁₋₆ alkyl, C₁₋₆ haloalkyl, and C₃₋₆ cycloalkyl;
R⁸ is selected from OH and NR⁹R¹⁰, and R⁹ and R¹⁰ are independently selected from H, C₁₋₆ alkyl and C₃₋₆ cycloalkyl; or R⁹ and R¹⁰, together with the N atom attached thereto, form 4-7 membered heterocycloalkyl by cyclization, and the 4-7 membered heterocycloalkyl is unsubstituted or substituted with 1-3 groups selected from the following: C₁₋₆ alkyl, halogen, OH, CN, and NH₂.

In another preferred embodiment, in the formula (1), R⁸ is OH.

In another preferred embodiment, in the formula (1), R¹ and R² are independently selected from H, halogen, OH, Me, Et, OMe, OEt, CF₃, NH₂, NO₂, and CN; R¹ and R² are independently preferably H, F, Cl, Me, Et, OMe, OEt or CF₃; R¹ and R² are independently more preferably H, F, Me, Et or OMe; R¹ and R² are independently more preferably F or Me; or R¹ and R², together with the phenyl ring attached thereto, form 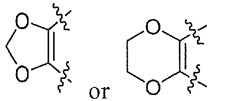 by cyclization.

In another preferred embodiment, in the formula (1), R³ is selected from Me, Et, 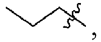 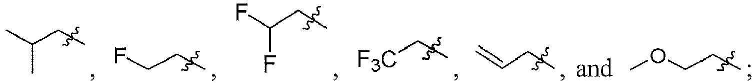 R³ is preferably Et, 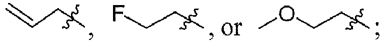 R³ is more preferably Et.

In another preferred embodiment, in the formula (1), X is selected from -O-, -S-, -S(O)-, -S(O₂)-, -N(H)-, and -N(Me)-; X is preferably -O-, -S-, -S(O)-, -S(O₂)- or -N(Me)-; X is more preferably -S-.

In another preferred embodiment, in the formula (1), R⁵ is selected from H, Me, Et, and 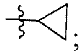 R⁵ is preferably H or Me; R⁵ is more preferably H.

In another preferred embodiment, in the formula (1), R⁶ and R⁷ are independently selected from II, Me, Et, CHF₂, CF₃, CH₂CF₃, , and 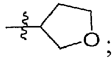 R⁶ and R⁷ are independently preferably H, Me, CF₃, 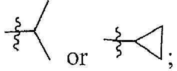 R⁶ and R⁷ are independently more preferably H or 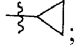 R⁶ and R⁷ are independently more preferably H; R⁶ and R⁷ are independently more preferably

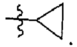

In another preferred embodiment, in the formula (1), 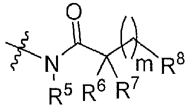 is selected from 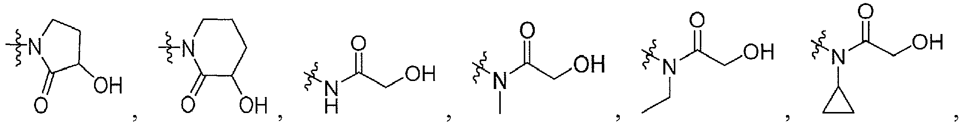 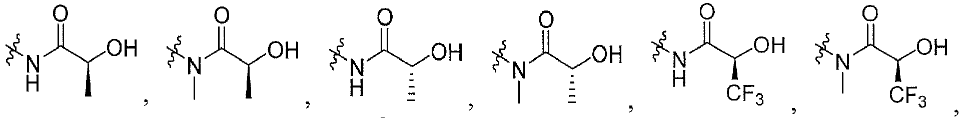 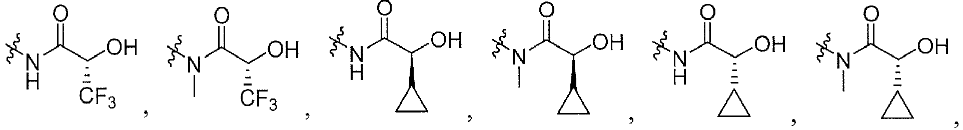 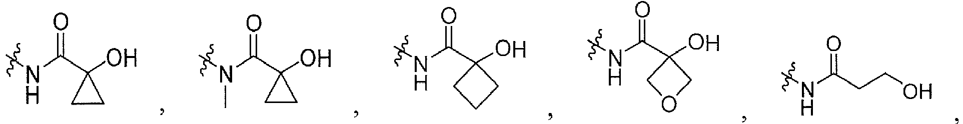 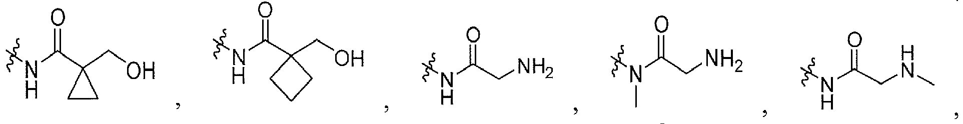 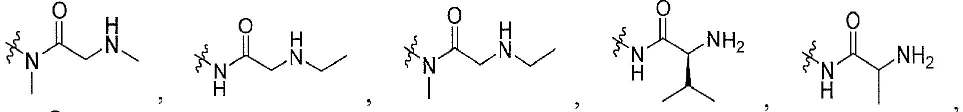 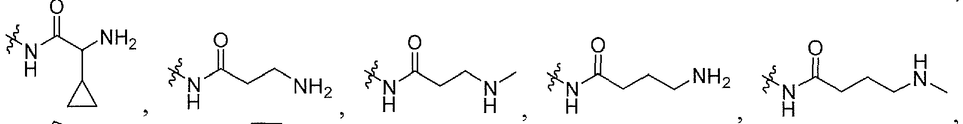 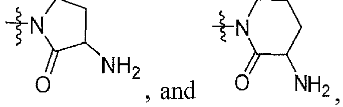 and is preferably 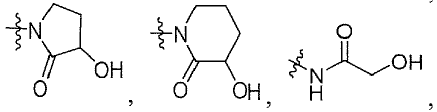 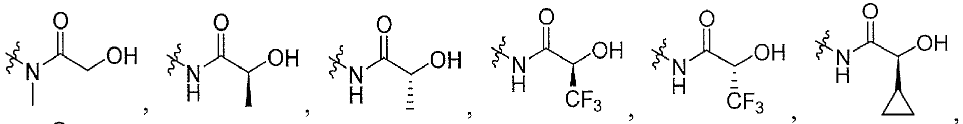 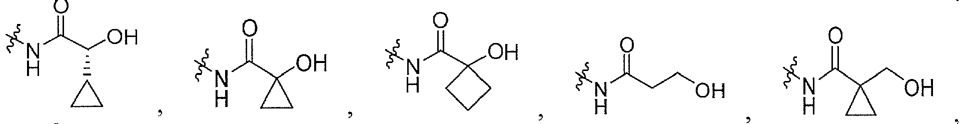 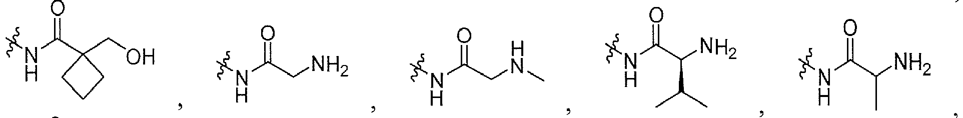 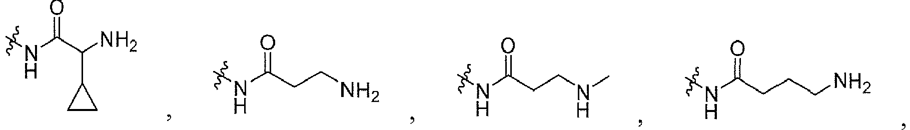 or 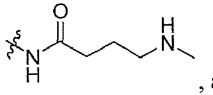 and more preferably

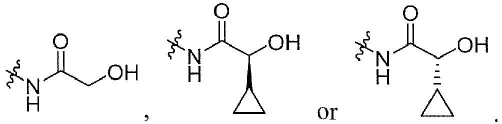

In another preferred embodiment, in the formula (1), R⁸ is OH, and 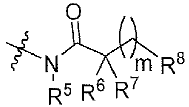 is selected from 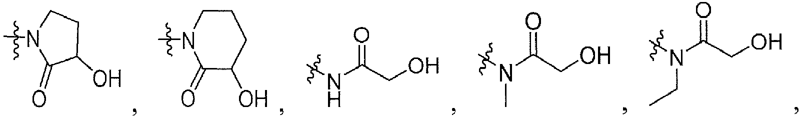 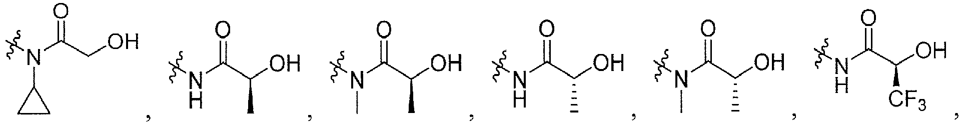 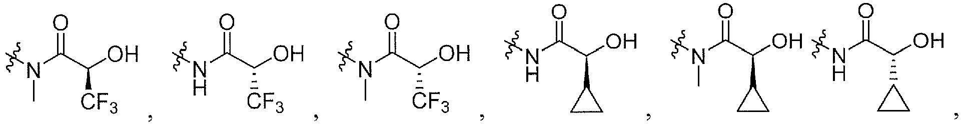 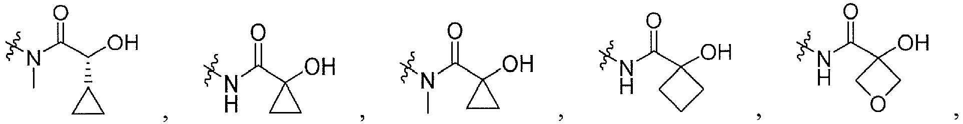 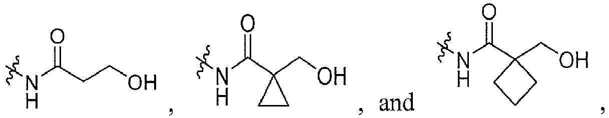 and is preferably 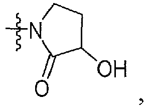 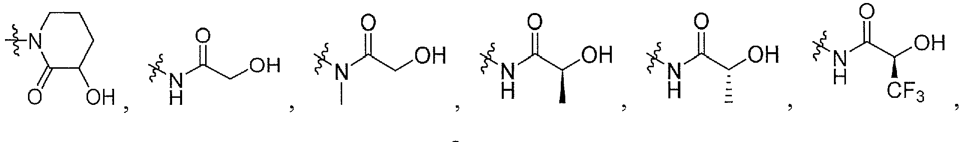 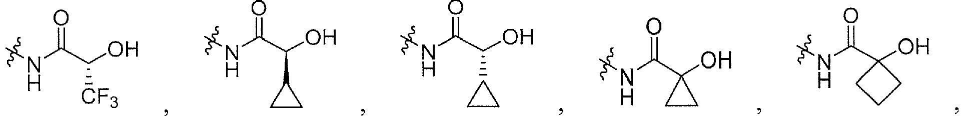 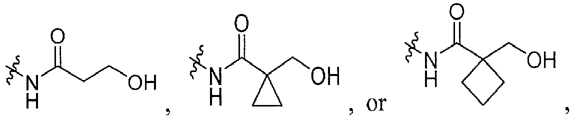 and more preferably

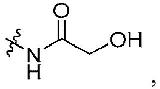

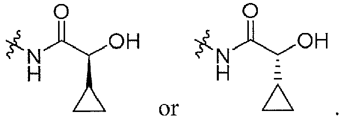

In some embodiments of the present invention, provided is a camptothecin derivative compound or a pharmaceutically acceptable salt thereof, wherein the compound has one of the following structures:

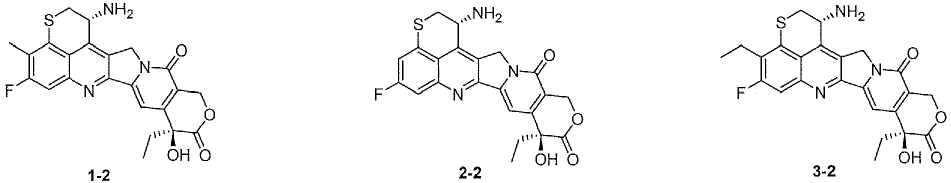

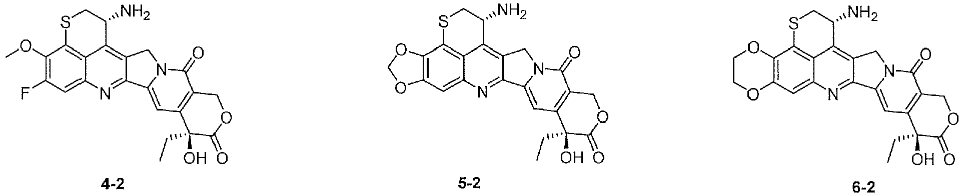

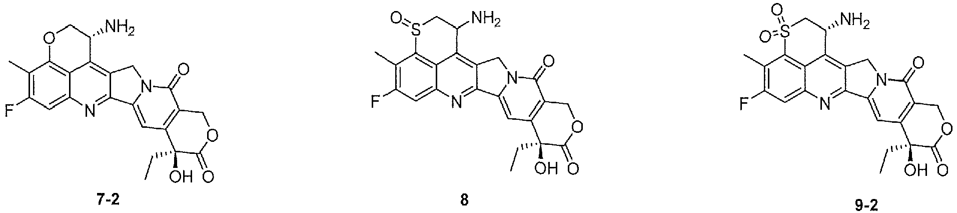

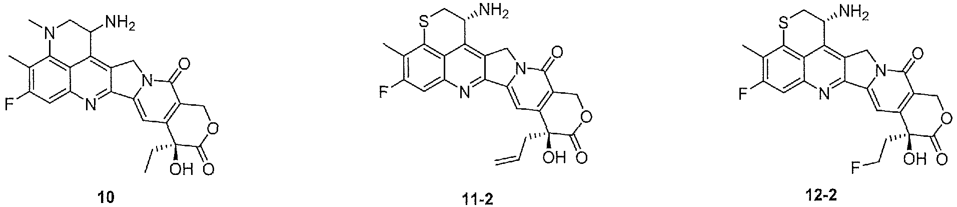

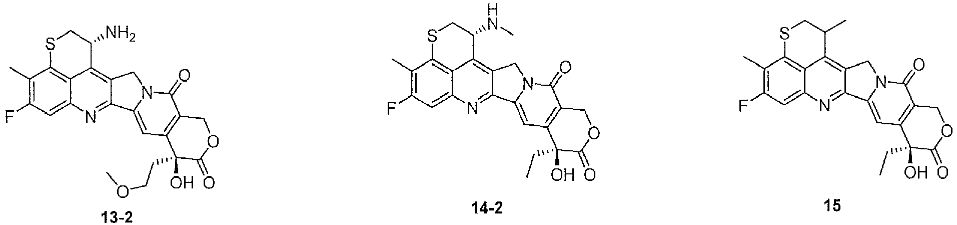

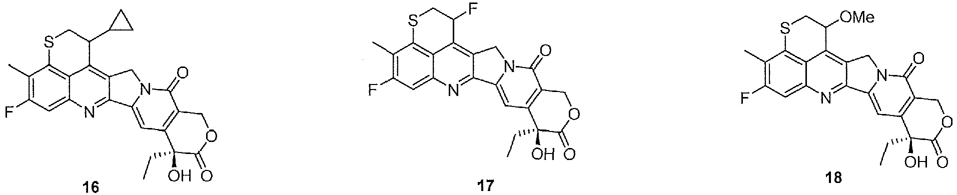

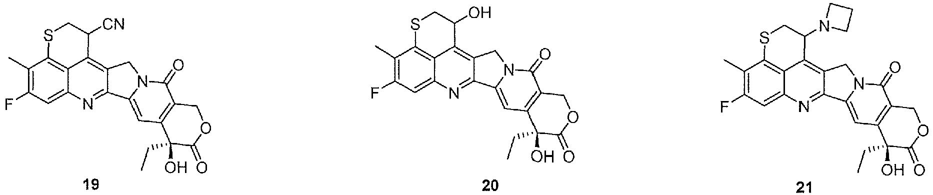

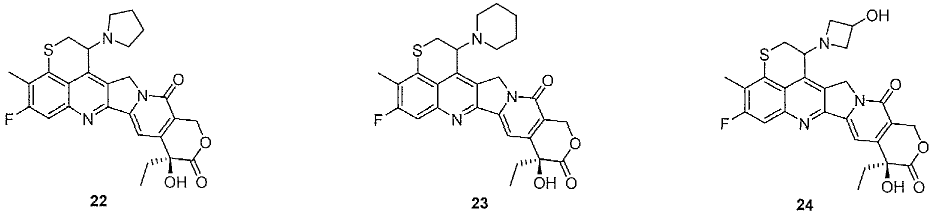

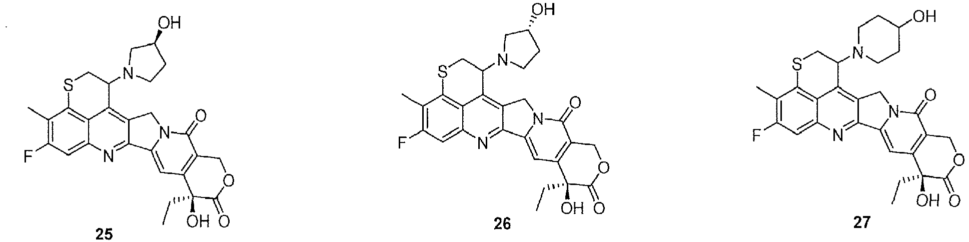

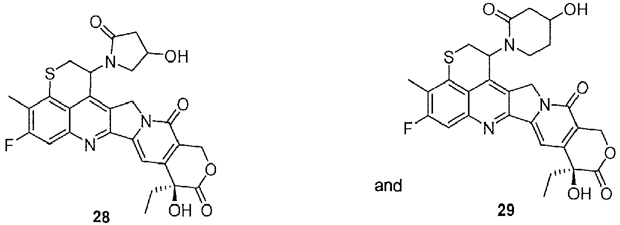

In some embodiments of the present invention, provided is a camptothecin derivative compound or a pharmaceutically acceptable salt thereof, wherein the compound has one of the following structures:

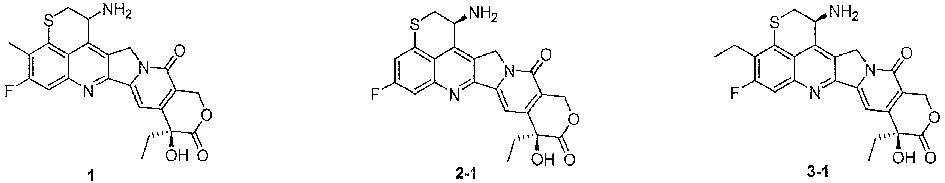

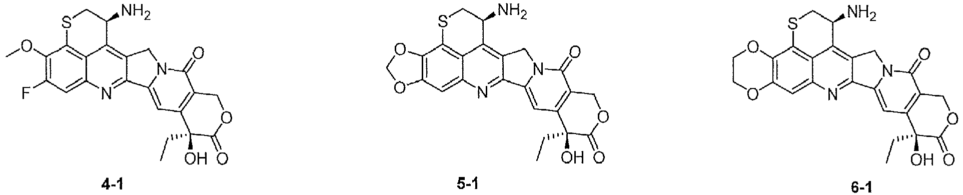

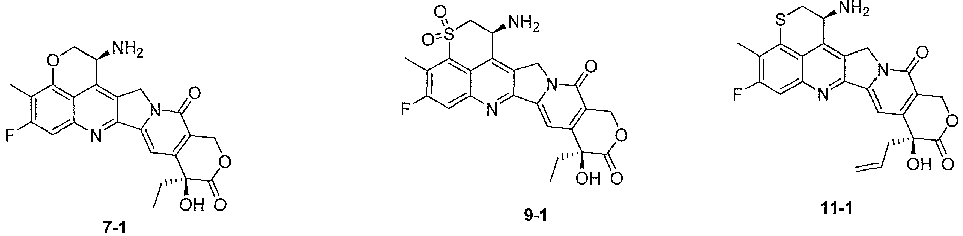

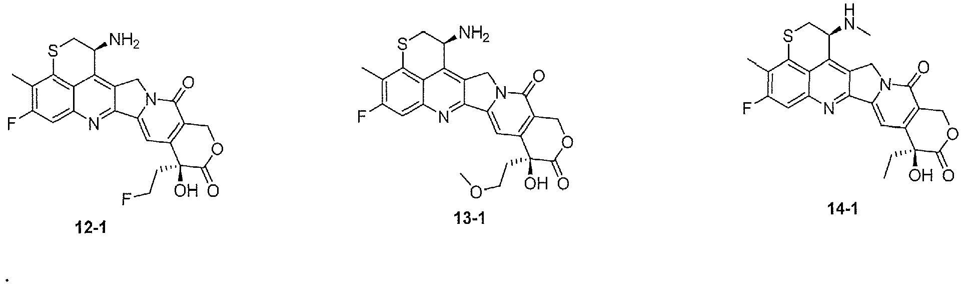

In some embodiments of the present invention, the compound of general formula (1) or isomers or pharmaceutically acceptable salts thereof has one of the following structures:

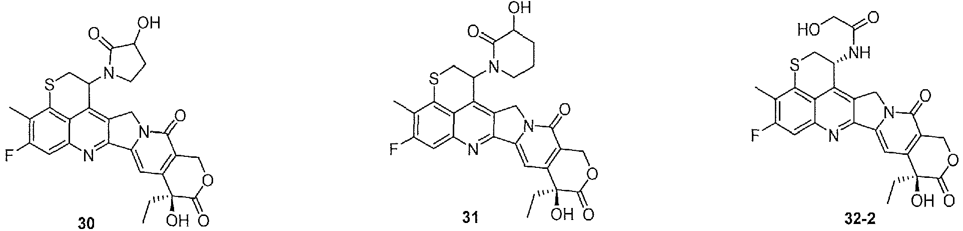

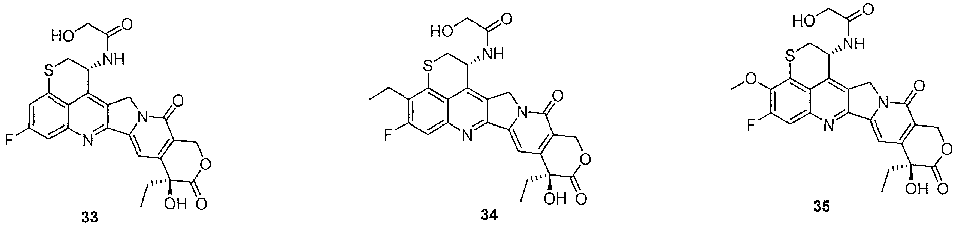

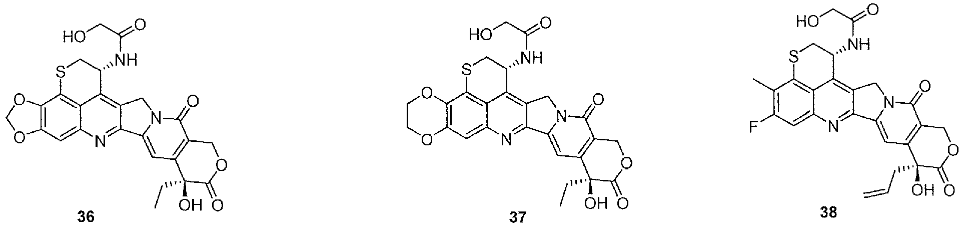

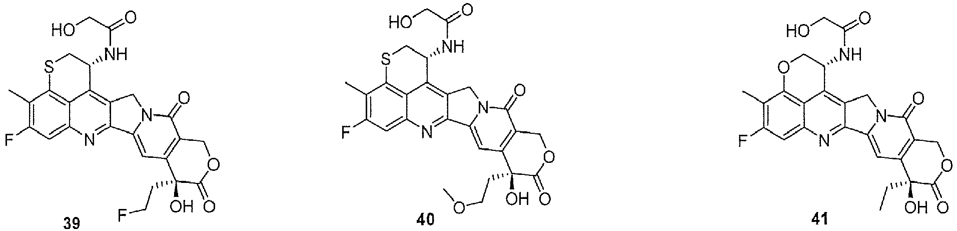

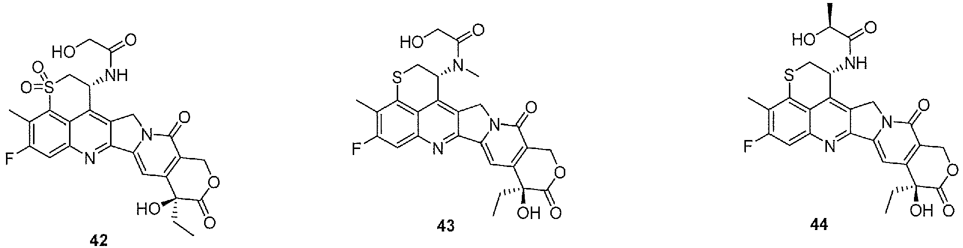

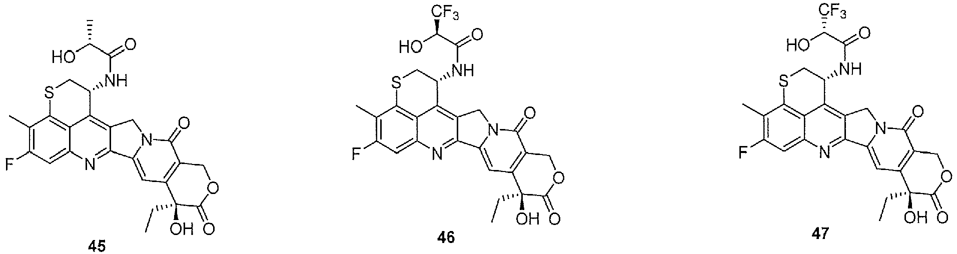

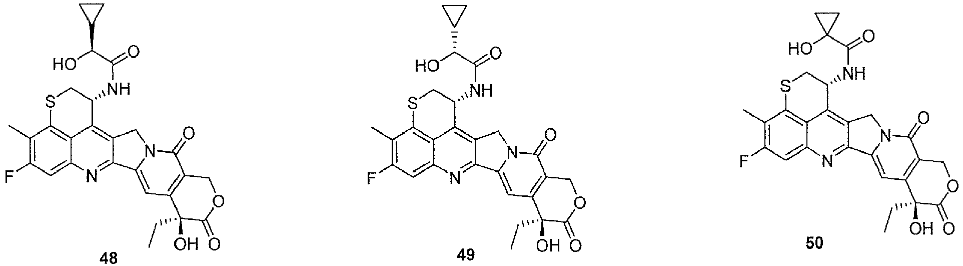

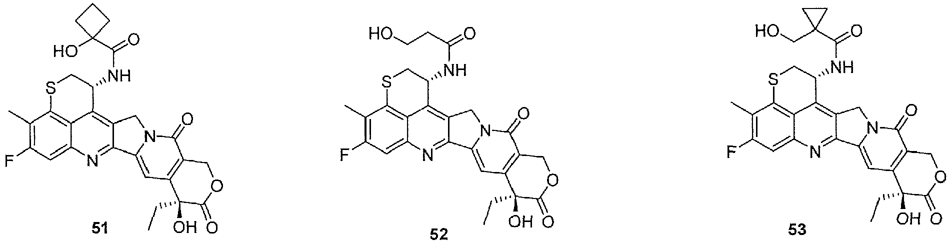

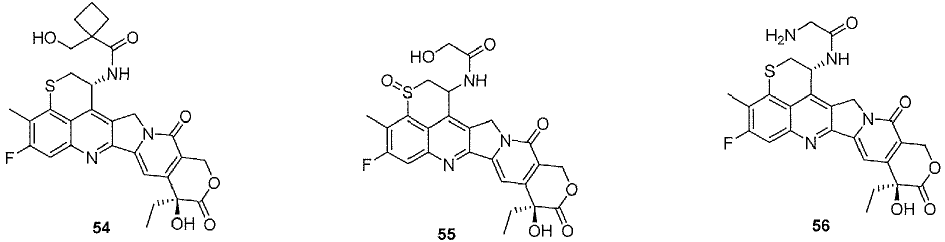

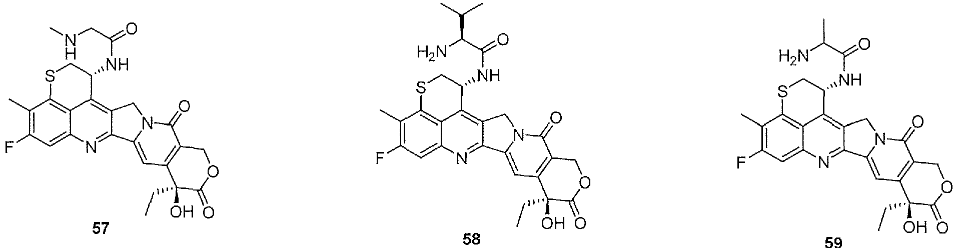

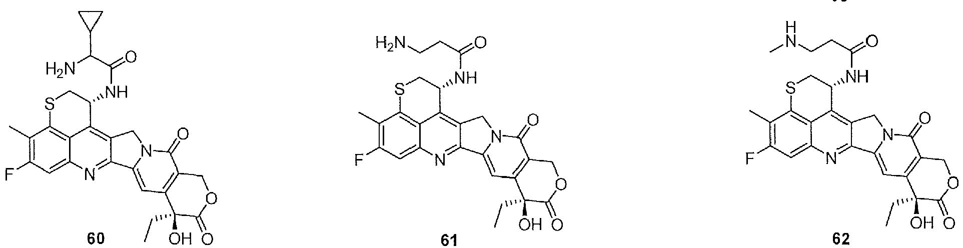

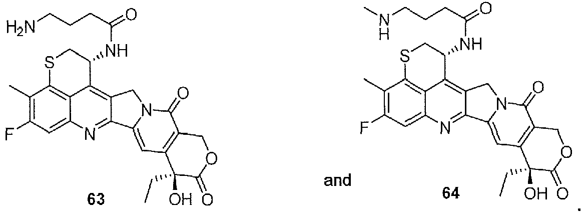

In some embodiments of the present invention, the compound of general formula (1) or isomers or pharmaceutically acceptable salts thereof has one of the following structures:

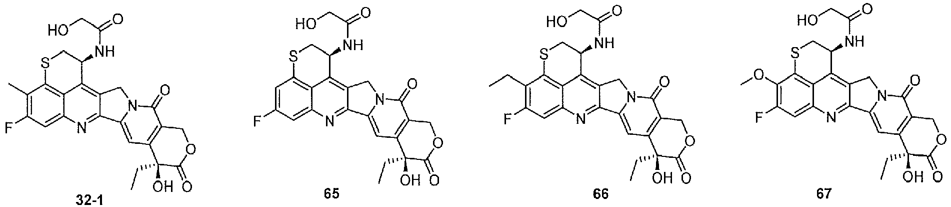

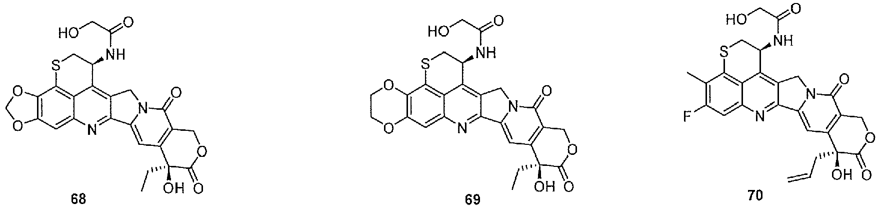

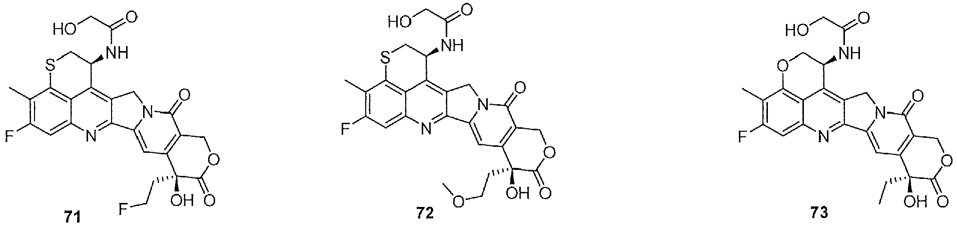

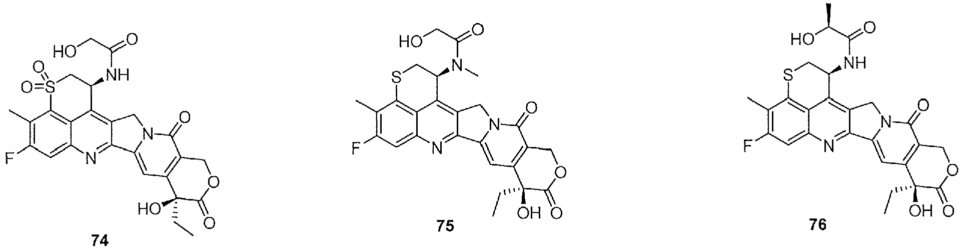

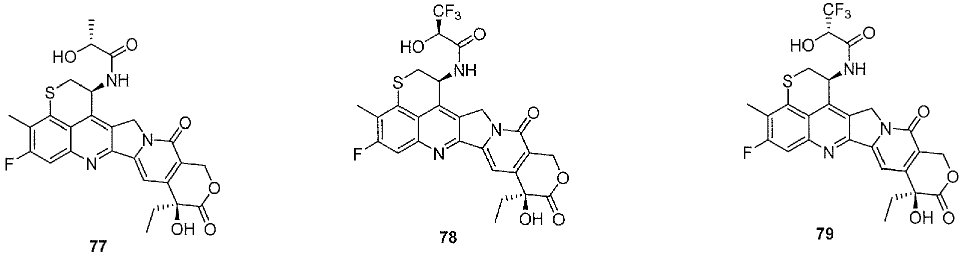

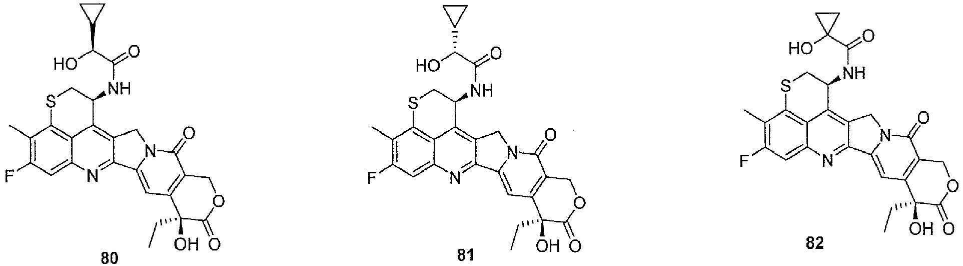

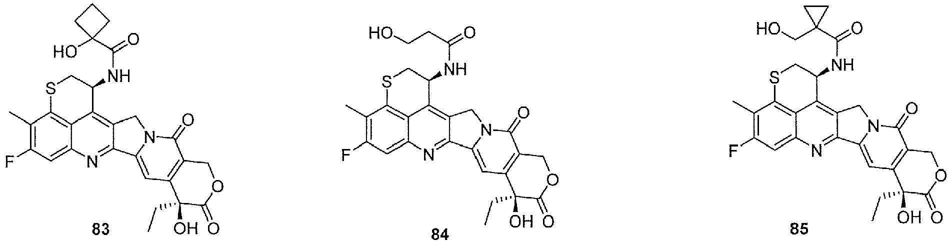

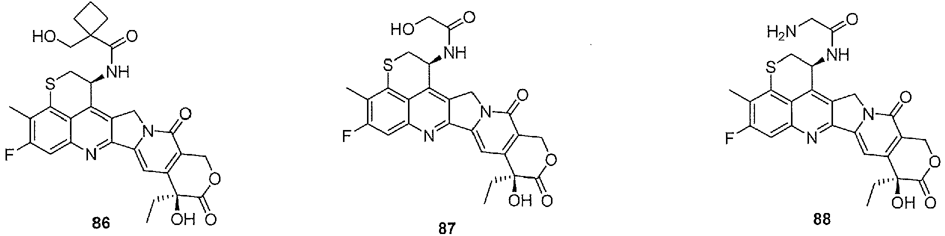

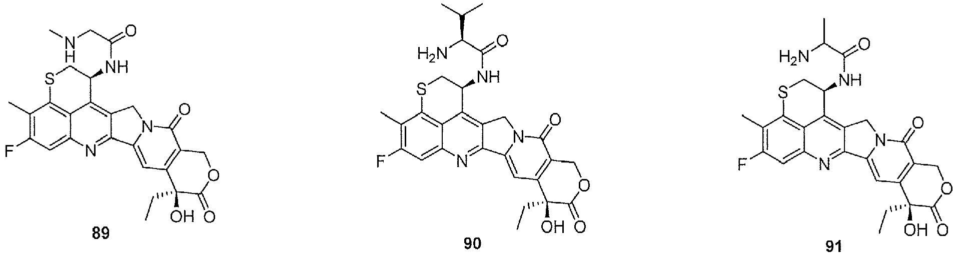

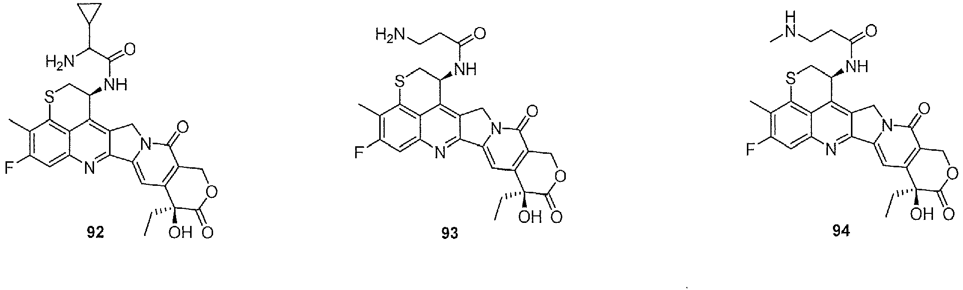

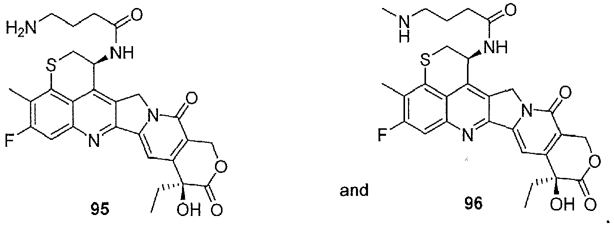

In some embodiments of the present invention, the present invention provides an antibody-drug conjugate, wherein the antibody-drug conjugate has one of the following structures: 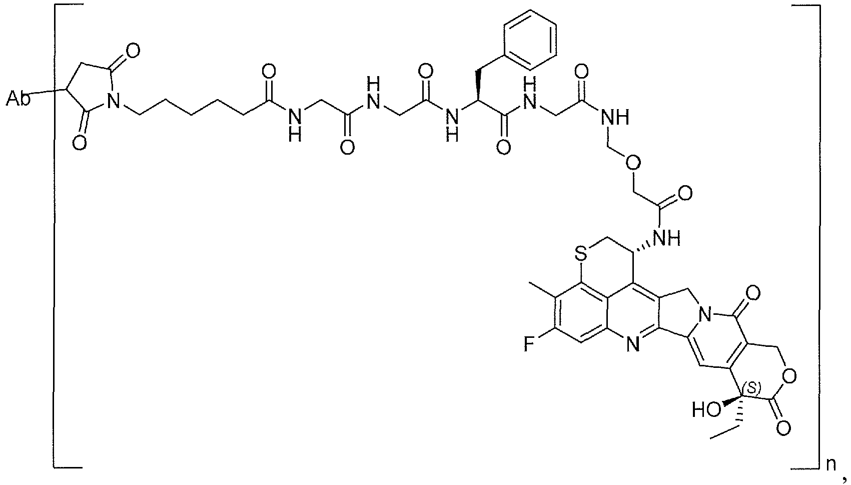 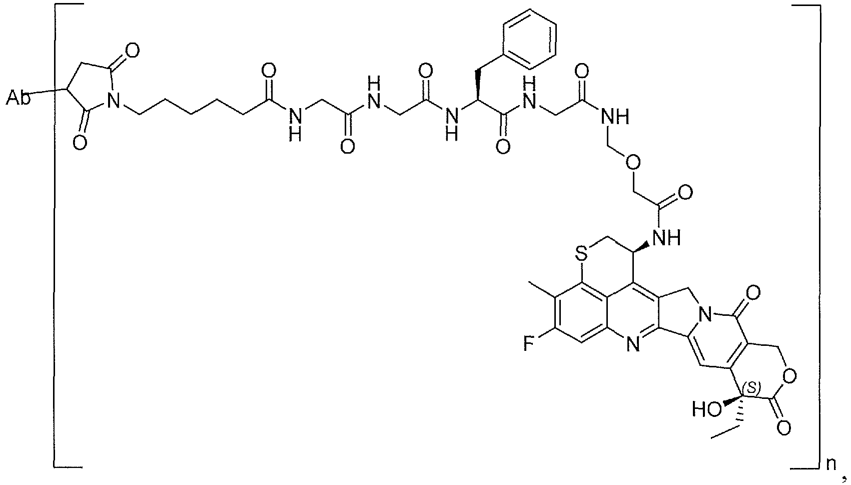 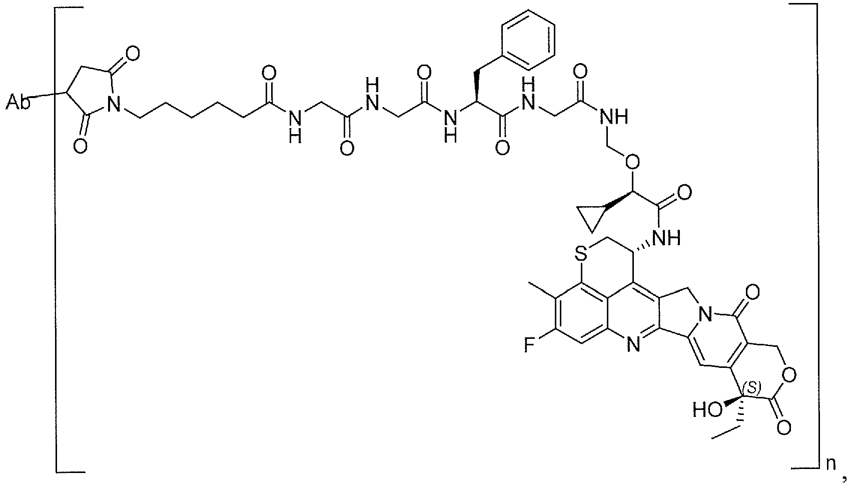 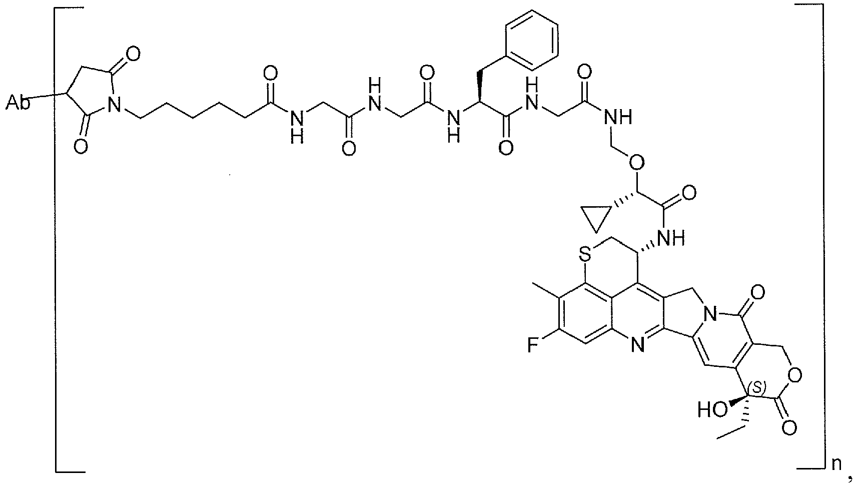 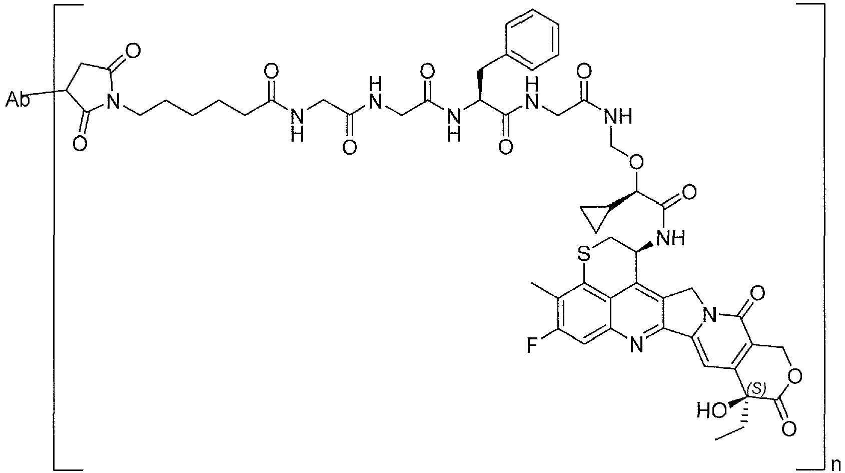 and 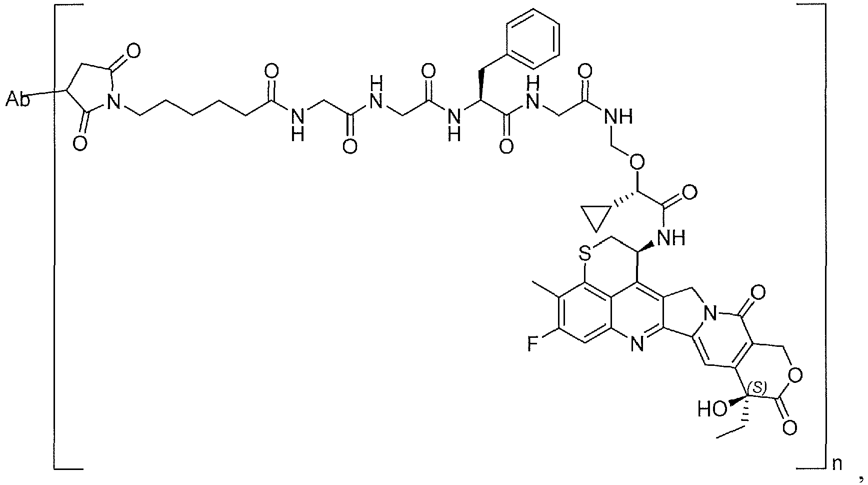 wherein
Ab represents a monoclonal antibody, preferably an anti-her2 antibody, and more preferably trastuzumab; n is a number from 2 to 8, preferably from 4 to 8, and more preferably from 7 to 8, e.g., 7.2 or 7.3.

An objective of the present invention is to provide use of the compound of the present invention or the optical isomers, the crystalline forms, the pharmaceutically acceptable salts, the hydrates or the solvates thereof as a small molecule toxin in the preparation of an antibody-drug conjugate (ADC).

Another objective of the present invention is to provide a pharmaceutical composition comprising a pharmaceutically acceptable excipient or carrier and the compound of the present invention or the optical isomers, the pharmaceutically acceptable inorganic or organic salts thereof as an active ingredient.

Still another objective of the present invention is to provide use of the compound of the present invention or the optical isomers or the pharmaceutically acceptable inorganic or organic salt thereof or the pharmaceutical composition in the preparation of a medicament for treating tumors and related diseases.

Yet another objective of the present invention is to provide use of the compound of the present invention or the optical isomers or the pharmaceutically acceptable inorganic or organic salt thereof in the preparation of a medicament for treating tumors and related diseases.

Still yet another objective of the present invention is to provide an antibody-drug conjugate (ADC), wherein the antibody-drug conjugate comprises an antibody, a small molecule toxin and a linker; the small molecule toxin is the compound of the present invention, and the linker links the antibody and the small molecule toxin via a covalent bond.

It should be understood that both the above general description and the following detailed description of the present invention are exemplary and explanatory, and are intended to provide further explanation of the present invention claimed.

### Synthesis of Compound

Methods for preparing the compound of general formula (1) disclosed herein is specifically described below, but these specific methods do not limit the present invention in any way.

The compound of general formula (1) described above may be synthesized using standard synthetic techniques or well-known techniques in combination with the methods described herein. In addition, solvents, temperatures and other reaction conditions mentioned herein may vary. Starting materials for the synthesis of the compounds may be obtained synthetically or commercially. The compounds described herein and other related compounds having different substituents may be synthesized using well-known techniques and starting materials, including the methods found in March, ADVANCED ORGANIC CHEMISTRY, 4th Ed., (Wiley 1992); Carey and Sundberg, ADVANCED ORGANIC CHEMISTRY, 4th Ed., Vols. A and B (Plenum 2000, 2001), and Green and Wuts, PROTECTIVE GROUPS IN ORGANIC SYNTHESIS, 3rd Ed., (Wiley 1999). General methods for preparing a compound can be changed by using appropriate reagents and conditions for introducing different groups into the formulas provided herein.

In one aspect, the compounds described herein are prepared according to methods well known in the art. However, the conditions involved in the methods, such as reactants, solvent, base, amount of the compound used, reaction temperature and time required for the reaction are not limited to the following explanation. The compounds of the present invention can also be conveniently prepared by optionally combining various synthetic methods described herein or known in the art, and such combinations can be easily determined by those skilled in the art to which the present invention pertains. In one aspect, the present invention also provides a method for preparing the compound of general formula (1), which is prepared using general reaction scheme 1 below. 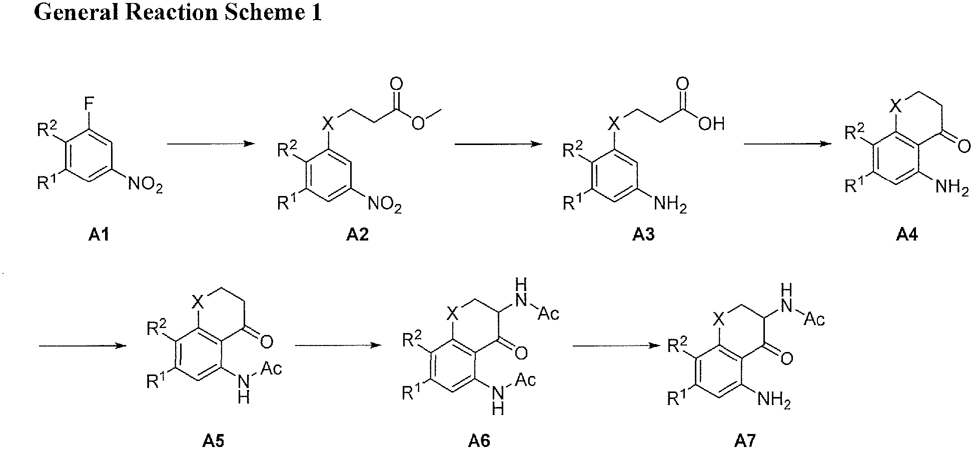 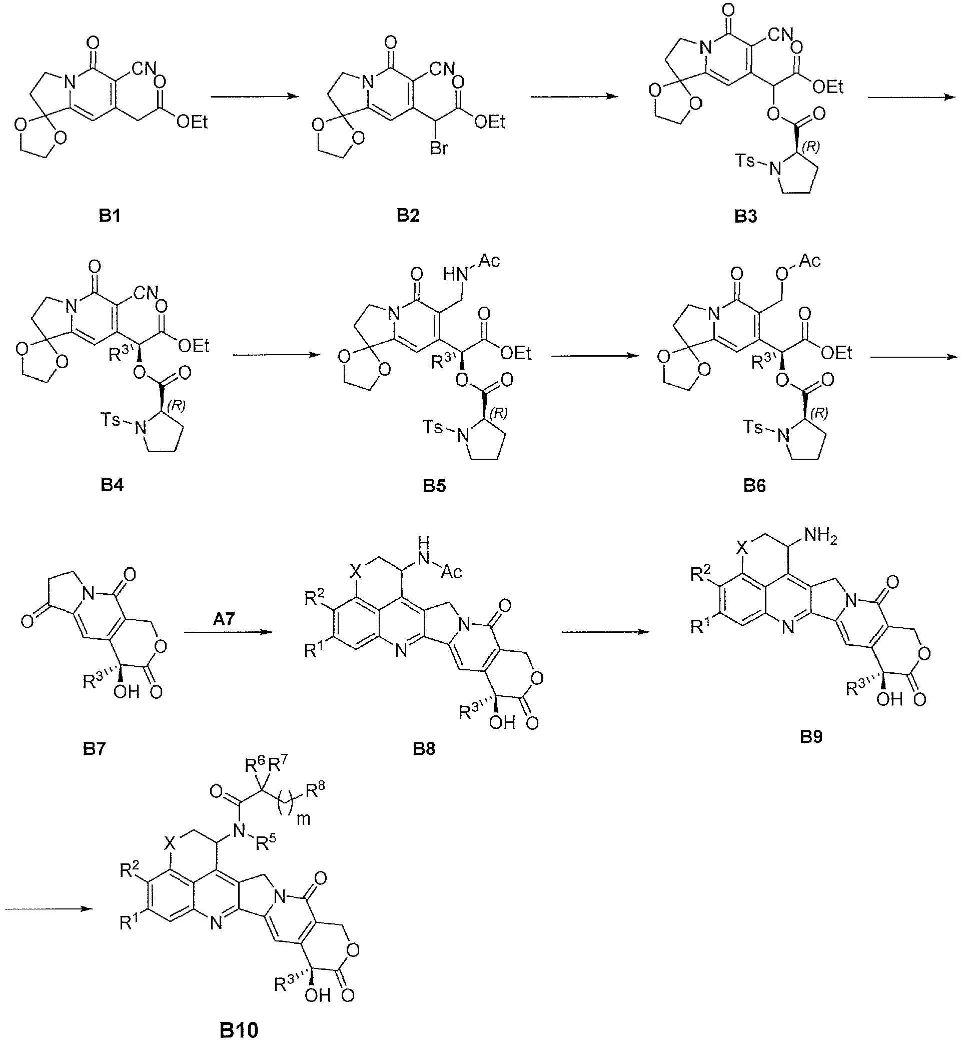 wherein R¹, R², R³, R⁵, R⁶, R⁷, R⁸ and X are as defined above.

Compound A1 is used as a starting material and subjected to a nucleophilic substitution reaction to obtain compound A2, compound A2 is subjected to nitro reduction under the condition of iron powder and hydrochloric acid and then hydrolyzed to obtain compound A3, compound A3 is subjected to a Friedel-Crafts acylation reaction to obtain compound A4, compound A4 is acetylated to obtain compound A5, compound A5 is subjected to nitrosation, reduction and acetylation to obtain compound A6, and compound A6 removes off the acetyl on the aniline to obtain intermediate compound A7.

Compound B1 is used as a starting material and subjected to bromination to obtain compound B2, compound B2 reacts with chiral amino acid to obtain compound B3, compound B3 is subjected to an affinity substitution reaction to obtain compound B4, compound B4 is subjected to cyano reduction to obtain compound B5, compound B5 is subjected to diazotization and a nucleophilic reaction to obtain compound B6, and compound B6 is finally cyclized to obtain intermediate compound B7.

Intermediate compound A7 and intermediate compound B7 are cyclized to obtain intermediate compound B8, intermediate compound B8 is deacetylated to obtain compound B9, and compound B9 is finally subjected to amino modification or functional group conversion to obtain the target compound of the present invention.

### Further Forms of Compounds

"Pharmaceutically acceptable" herein refers to a substance, such as a carrier or diluent, which will not cause a compound to lose its biological activity or properties. It is relatively non-toxic; for example, when an individual is given a substance, it will not cause unwanted biological effects or interact with any component contained therein in a deleterious manner.

The term "pharmaceutically acceptable salt" refers to a form of a compound that does not cause significant irritation to the organism for drug administration or eliminate the biological activity and properties of the compound. In certain specific aspects, pharmaceutically acceptable salts are obtained by subjecting the compound of general formula (1) to a reaction with acids, e.g., inorganic acids such as hydrochloric acid, hydrobromic acid, hydrofluoric acid, sulfuric acid, phosphoric acid, nitric acid, phosphoric acid and the like, organic acids such as formic acid, acetic acid, propionic acid, oxalic acid, trifluoroacetic acid, malonic acid, succinic acid, fumaric acid, maleic acid, lactic acid, malic acid, tartaric acid, citric acid, picric acid, methanesulfonic acid, benzenesulfonic acid, p-toluenesulfonic acid and the like, and acidic amino acids such as aspartic acid, glutamic acid and the like.

It should be understood that references to pharmaceutically acceptable salts include solvent addition forms or crystalline forms, especially solvates or polymorphs. A solvate contains either stoichiometric or non-stoichiometric amount of solvent and is selectively formed during crystallization with pharmaceutically acceptable solvents such as water and ethanol. Hydrates are formed when the solvent is water, or alcoholates are formed when the solvent is ethanol. The solvates of the compound of general formula (1) are conveniently prepared or formed according to methods described herein. For example, the hydrates of the compound of general formula (1) are conveniently prepared by recrystallization from a mixed solvent of water/organic solvent, wherein the organic solvent used includes, but is not limited to, tetrahydrofuran, acetone, ethanol or methanol. Furthermore, the compounds mentioned herein can exist in both non-solvated and solvated forms. In general, the solvated forms are considered equivalent to the non-solvated forms for purposes of the compounds and methods provided herein.

In other specific examples, the compound of general formula (1) is prepared in different forms, including but not limited to amorphous, pulverized and nanoparticle forms. In addition, the compound of general formula (1) includes crystalline forms, and may also be polymorphs. Polymorphs include different lattice arrangements of the same elements of a compound. Polymorphs usually have different X-ray diffraction spectra, infrared spectra, melting points, density, hardness, crystalline forms, optical and electrical properties, stability and solubility. Different factors such as recrystallization solvent, crystallization rate and storage temperature may lead to monocrystalline form being dominant.

In another aspect, the compound of general formula (1) may have a chiral center and/or axial chirality, and thus may be present in the form of a racemate, a racemic mixture, a single enantiomer, a diastereomeric compound, a single diastereomer and a cis-trans isomer. Each chiral center or axial chirality will independently produce two optical isomers, and all possible optical isomers, diastereomeric mixtures and pure or partially pure compounds are included within the scope of the present invention. The present invention is meant to include all such isomeric forms of these compounds.

The compound of the present invention may contain unnatural proportions of atomic isotopes at one or more of the atoms that constitute the compound. For example, the compound may be labeled with radioactive isotopes, such as tritium (³H), iodine-125 (¹²⁵I) and C-14 (¹⁴C). For another example, deuterium can be used to substitute a hydrogen atom to form a deuterated compound, the bond formed by deuterium and carbon is stronger than that formed by common hydrogen and carbon, and compared with an undeuterated medicament, the deuterated medicament generally has the advantages of reducing toxic and side effects, increasing medicament stability, enhancing curative effect, prolonging *in vivo* half-life period of the medicament and the like. All isotopic variations of the compound of the present invention, whether radioactive or not, are intended to be encompassed within the scope of the present invention.

### Terminology

Unless otherwise stated, the terms used in the present application, including those in the specification and claims, are defined as follows. It must be noted that in the specification and the appended claims, the singular forms "a" and "an" include plural meanings unless clearly indicated otherwise. Unless otherwise stated, conventional methods for mass spectrometry, nuclear magnetic resonance spectroscopy, HPLC, protein chemistry, biochemistry, recombinant DNA technology and pharmacology are used. As used herein, "or" or "and" refers to "and/or" unless otherwise stated.

Unless otherwise specified, "alkyl" refers to a saturated aliphatic hydrocarbon group, including linear and branched groups containing 1 to 6 carbon atoms. Lower alkyls containing 1 to 4 carbon atoms, such as methyl, ethyl, propyl, 2-propyl, n-butyl, isobutyl or tert-butyl, are preferred. As used herein, "alkyl" includes unsubstituted and substituted alkyl, particularly alkyl substituted with one or more halogens. Preferred alkyl is selected from CH₃, CH₃CH₂, CF₃, CHF₂, CF₃CH₂, CF₃(CH₃)CH, ⁱPr, ⁿPr, ⁱBu, ⁿBu and ^{t}Bu.

Unless otherwise specified, "alkylene" refers to a divalent alkyl as defined above. Examples of alkylene include, but are not limited to, methylene and ethylene.

Unless otherwise specified, "alkenyl" refers to an unsaturated aliphatic hydrocarbon group containing carbon-carbon double bonds, including linear or branched groups containing 1 to 14 carbon atoms. Lower alkenyls containing 1 to 4 carbon atoms, such as vinyl, 1-propenyl, 1-butenyl or 2-methylpropenyl, are preferred.

Unless otherwise specified, "alkynyl" refers to an unsaturated aliphatic hydrocarbon group containing carbon-carbon triple bonds, including linear and branched groups containing 1 to 14 carbon atoms. Lower alkynyls containing 1 to 4 carbon atoms, such as ethynyl, 1-propynyl or 1-butynyl, are preferred.

Unless otherwise specified, "cycloalkyl" refers to a non-aromatic hydrocarbon ring system (monocyclic, bicyclic, or polycyclic), and partially unsaturated cycloalkyl may be referred to as "cycloalkenyl" if the carbocyclic ring contains at least one double bond, or "cycloalkynyl" if the carbocyclic ring contains at least one triple bond. Cycloalkyl may include monocyclic or polycyclic groups and spiro rings (e.g., having 2, 3, or 4 fused rings). In some embodiments, cycloalkyl is monocyclic. In some embodiments, cycloalkyl is monocyclic or bicyclic. The ring carbon atoms of cycloalkyl may optionally be oxidized to form an oxo or sulfido group. Cycloalkyl further includes cycloalkylene. In some embodiments, cycloalkyl contains 0, 1 or 2 double bonds. In some embodiments, cycloalkyl contains 1 or 2 double bonds (partially unsaturated cycloalkyl). In some embodiments, cycloalkyl may be fused with aryl, heteroaryl, cycloalkyl, and heterocycloalkyl. In some embodiments, cycloalkyl may be fused with aryl, cycloalkyl, and heterocycloalkyl. In some embodiments, cycloalkyl may be fused with aryl and heterocycloalkyl. In some embodiments, cycloalkyl may be fused with aryl and cycloalkyl. Examples of cycloalkyl include cyclopropyl, cyclobutyl, cyclopentyl, cyclohexyl, cycloheptyl, cyclopentenyl, cyclohexenyl, cyclohexadienyl, cycloheptatrienyl, norcamphanyl, norpinanyl, norcamyl, bicyclo[1.1.1]pentyl, bicyclo[2.1.1]hexyl, and the like.

Unless otherwise specified, "alkoxy" refers to an alkyl group that bonds to the rest of the molecule through an ether oxygen atom. Representative alkoxy groups are those having 1-6 carbon atoms, such as methoxy, ethoxy, propoxy, isopropoxy, butoxy, isobutoxy, sec-butoxy and tert-butoxy. As used herein, "alkoxy" includes unsubstituted and substituted alkoxy, particularly alkoxy substituted with one or more halogens. Preferred alkoxy is selected from OCH₃, OCF₃, CHF₂O, CF₃CH₂O, ⁱ⁻PrO, ⁿ⁻PrO, ⁱ⁻BuO, ⁿ⁻BuO and ^{t-}BuO.

Unless otherwise specified, "aryl" refers to an aromatic hydrocarbon group, and it is monocyclic or polycyclic; for example, a monocyclic aryl ring may be fused with one or more carbocyclic aromatic groups. Examples of aryl include, but are not limited to, phenyl, naphthyl, and phenanthryl.

Unless otherwise specified, "heterocycloalkyl" refers to a non-aromatic ring or ring system, which may optionally contain one or more alkenylene as a moiety of the ring structure, having at least one heteroatom ring member independently selected from boron, phosphorus, nitrogen, sulfur, oxygen, and phosphorus. Partially unsaturated heterocycloalkyl may be referred to as "heterocycloalkenyl" if heterocycloalkyl contains at least one double bond, or "heterocycloalkynyl" if heterocycloalkyl contains at least one triple bond. Heterocycloalkyl may include monocyclic, bicyclic, spiro ring, or polycyclic systems (e.g., having two fused or bridged rings). In some embodiments, heterocycloalkyl is a monocyclic group having 1, 2 or 3 heteroatoms independently selected from nitrogen, sulfur and oxygen. The ring carbon atoms and heteroatoms of heterocycloalkyl may optionally be oxidized to form oxo or sulfido groups or other oxidized bonds (e.g., C(O), S(O), C(S) or S(O)₂, N-oxides, etc.), or the nitrogen atoms may be quaternized. Heterocycloalkyl may be attached via a ring carbon atom or a ring heteroatom. In some embodiments, heterocycloalkyl contains 0 to 3 double bonds. In some embodiments, heterocycloalkyl contains 0 to 2 double bonds. Also included in the definition of heterocycloalkyl are moieties having one or more aromatic rings fused to (i.e., sharing a bond with) the heterocycloalkyl ring, for example, benzo-derivatives of piperidine, morpholine, azepin, thienyl, or the like. Heterocycloalkyl containing a fused aromatic ring may be attached via any ring atom, including ring atoms of the fused aromatic ring. Examples of heterocycloalkyl include, but are not limited to, azetidinyl, azepinyl, dihydrobenzofuryl, dihydrofuryl, dihydropyranyl, N-morpholinyl, 3-oxa-9-azaspiro[5.5]undecyl, 1-oxa-8-azaspiro[4.5]decyl, piperidinyl, piperazinyl, oxopiperazinyl, pyranyl, pyrrolidinyl, quininyl, tetrahydrofuryl, tetrahydropyranyl, 1,2,3,4-tetrahydroquinolinyl, tropanyl, 4,5,6,7-tetrahydrothiazolo[5,4-c]pyridinyl, 4,5,6,7-tetrahydro-1*H*-imidazo [4,5-c]pyridine, *N*-methylpiperidinyl, tetrahydroimidazolyl, pyrazolidinyl, butyrolactam, valerolactam, imidazolidinonyl, hydantoinyl, dioxolanyl, phthalimidyl, pyrimidine-2,4(1*H*,3*H*)-dione, 1,4-dioxanyl, morpholinyl, thiomorpholinyl, thiomorpholinyl-*S*-oxide, thiomorpholinyl-*S,S*-oxide, piperazinyl, pyranyl, pyridonyl, 3-pyrrolinyl, thiopyranyl, pyronyl, tetrahydrothienyl, 2-azaspiro[3.3]heptanyl, indolinyl,

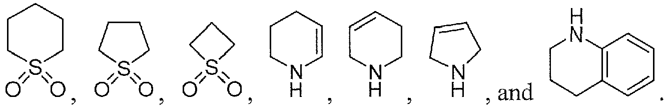

Unless otherwise specified, "halogen" (or halo) refers to fluorine, chlorine, bromine, or iodine. The term "halo" (or "halogenated") before a group name indicates that the group is partially or fully halogenated, that is, substituted in any combination by F, Cl, Br or I, preferably by For Cl. "Optional" or "optionally" means that the subsequently described event or circumstance may, but does not necessarily, occur, and the description includes instances where the event or circumstance occurs and instances where it does not.

The substituent "-O-CH₂-O-" means that two oxygen atoms in the substituent are linked to two adjacent carbon atoms in the heterocycloalkyl, aryl or heteroaryl, for example:

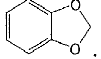

When the number of a linker group is 0, such as -(CH₂)₀-, it means that the linker group is a single bond.

When one of the variables is selected from a chemical bond, it means that the two groups linked by this variable are linked directly. For example, when L in X-L-Y represents a chemical bond, it means that the structure is actually X-Y.

Unless otherwise stated, the absolute configuration of a stereogenic center is represented by a wedged solid bond ( 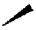 ) and a wedged dashed bond ( 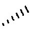 ), and the relative configuration of a stereogenic center is represented by a straight solid bond ( 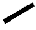 ) and a straight dashed bond ( 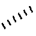 ). A wavy line ( 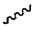 ) represents a wedged solid bond ( 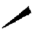 ) or a wedged dashed bond ( 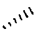 ), or a wavy line ( 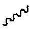 ) represents a straight solid bond ( 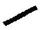 ) or a straight dashed bond ( 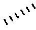 ).

Unless otherwise stated, a single bond or a double bond is represented by -̅ -̅ -̅.

### Specific Pharmaceutical and Medical Terminology

The term "acceptable", as used herein, means that a formula component or an active ingredient does not unduly adversely affect a general therapeutic target's health.

The terms "treatment," "treatment course," or "therapy", as used herein, include alleviating, inhibiting, or ameliorating a symptom or condition of a disease; inhibiting the development of complications; ameliorating or preventing underlying metabolic syndrome; inhibiting the development of a disease or symptom, e.g., controlling the progression of a disease or condition; alleviating a disease or symptom; causing a disease or symptom to subside; alleviating a complication caused by a disease or symptom, or preventing or treating a sign caused by a disease or symptom. As used herein, a compound or pharmaceutical composition, when administered, can ameliorate a disease, symptom, or condition, particularly meaning ameliorating the severity, delaying the onset, slowing the progression, or reducing the duration of the disease. Fixed or temporary administration, or continuous or intermittent administration, may be attributed to or associated with the administration.

The "active ingredient" refers to the compound of general formula (1), and pharmaceutically acceptable inorganic or organic salts of the compound of general formula (1). The compounds of the present invention may contain one or more asymmetric centers (chiral center or axial chirality) and thus occur in the form of a racemate, racemic mixture, single enantiomer, diastereomeric compound and single diastereomer. Asymmetric centers that may be present depend on the nature of the various substituents on the molecule. Each of these asymmetric centers will independently produce two optical isomers, and all possible optical isomers, diastereomeric mixtures and pure or partially pure compounds are included within the scope of the present invention. The present invention is meant to include all such isomeric forms of these compounds.

The terms such as "compound", "composition", "agent" or "medicine or medicament" are used interchangeably herein and all refer to a compound or composition that, when administered to an individual (human or animal), is capable of inducing a desired pharmacological and/or physiological response by local and/or systemic action.

The term "administered, administering or administration" refers herein to the direct administration of the compound or composition, or the administration of a prodrug, derivative, analog or the like of the active compound.

Although the numerical ranges and parameters defining the broad scope of the present invention are approximations, the related numerical values set forth in the specific examples have been present herein as precisely as possible. Any numerical value, however, inherently contains a standard deviation necessarily resulting from certain methods of testing. Herein, "about" generally means that the actual numerical value is within a particular numerical value or range ± 10%, 5%, 1%, or 0.5%. Alternatively, the term "about" indicates that the actual numerical value falls within the acceptable standard error of a mean, as considered by those skilled in the art. All ranges, quantities, numerical values and percentages used herein (e.g., to describe an amount of a material, a length of time, a temperature, an operating condition, a quantitative ratio and the like) are to be understood as being modified by the word "about", except in the experimental examples or where otherwise explicitly indicated. Accordingly, unless otherwise contrarily stated, the numerical parameters set forth in the specification and the appended claims are all approximations that may vary as desired. At the very least, these numerical parameters should be understood as the significant digits indicated or the numerical value obtained using conventional rounding rules.

Unless otherwise defined in the specification, the scientific and technical terms used herein have the same meaning as commonly understood by those skilled in the art. Furthermore, the singular nouns used in the specification encompass their plural forms, unless contradicted by context; the plural nouns used also encompass their singular forms.

### Therapeutic use

The present invention provides a method for treating diseases, including but not limited to cancer, with the compound, the antibody-drug conjugate or the pharmaceutical composition of the present invention.

In some embodiments, provided is a method for treating cancer, comprising administering to an individual in need thereof an effective amount of a pharmaceutical composition of any of the foregoing compounds and antibody-drug conjugates. In other embodiments, the cancer is a hematologic cancer and a solid tumor, including but not limited to, leukemia, breast cancer, lung cancer, pancreatic cancer, colon cancer, bladder cancer, brain cancer, urothelial cancer, prostate cancer, liver cancer, ovarian cancer, head and neck cancer, gastric cancer, mesothelioma, or all cancer metastases.

### Route of administration

The compound and the pharmaceutically acceptable salt thereof of the present invention can be prepared into various preparations comprising a safe and effective amount of the compound or the pharmaceutically acceptable salt thereof of the present invention, and a pharmaceutically acceptable excipient or carrier, wherein the "safe and effective amount" means that the amount of the compound is sufficient to significantly improve the condition without causing serious side effects. The safe and effective amount of the compound is determined according to the age, condition, course of treatment and other specific conditions of a treated subject.

The "pharmaceutically acceptable excipient or carrier" refers to one or more compatible solid or liquid fillers or gel substances that are suitable for human use and must be of sufficient purity and sufficiently low toxicity. "Compatible" means that the components of the composition are capable of intermixing with the compound of the present invention and with each other, without significantly diminishing the pharmaceutical efficacy of the compound. Examples of pharmaceutically acceptable excipients or carriers include cellulose and its derivatives (e.g., sodium carboxymethylcellulose, sodium ethylcellulose or cellulose acetate), gelatin, talc, solid lubricants (e.g., stearic acid or magnesium stearate), calcium sulfate, vegetable oil (e.g., soybean oil, sesame oil, peanut oil or olive oil), polyols (e.g., propylene glycol, glycerol, mannitol or sorbitol), emulsifiers (e.g., Tween^{®}), wetting agents (e.g., sodium lauryl sulfate), colorants, flavoring agents, stabilizers, antioxidants, preservatives, pyrogen-free water, etc.

When the compound of the present invention is administered, it may be administered orally, rectally, parenterally (intravenously, intramuscularly or subcutaneously) or topically.

Solid dosage forms for oral administration include capsules, tablets, pills, pulvises and granules. In these solid dosage forms, the active compound is mixed with at least one conventional inert excipient (or carrier), such as sodium citrate or dicalcium phosphate, or with the following ingredients: (a) fillers or extenders, such as starch, lactose, sucrose, glucose, mannitol and silicic acid; (b) binders, such as hydroxymethyl cellulose, alginate, gelatin, polyvinylpyrrolidone, sucrose and acacia; (c) humectants, such as glycerol; (d) disintegrants, such as agar, calcium carbonate, potato or tapioca starch, alginic acid, certain complex silicates and sodium carbonate; (e) solution retarders, such as paraffin; (f) absorption accelerators, such as quaternary ammonium compounds; (g) wetting agents, such as cetyl alcohol and glycerol monostearate; (h) adsorbents, such as kaolin; and (i) lubricants, such as talc, calcium stearate, magnesium stearate, solid polyethylene glycol and sodium lauryl sulfate, or mixtures thereof. In the case of capsules, tablets and pills, the dosage forms may further comprise buffers.

Solid dosage forms such as tablets, dragees, capsules, pills and granules can be prepared using coatings and shells such as enteric coatings and other materials well known in the art. They may comprise opacifying agents, and the active compound or compound in such a composition may be released in a certain part of the digestive tract in a delayed manner. Examples of embedding components that can be used are polymeric substances and wax-based substances. If necessary, the active compound can also be in microcapsule form with one or more of the above-mentioned excipients.

Liquid dosage forms for oral administration include pharmaceutically acceptable emulsions, solutions, suspensions, syrups and elixirs. In addition to the active compound, the liquid dosage form may comprise inert diluents commonly used in the art, such as water or other solvents, solubilizers and emulsifiers, for example, ethanol, isopropanol, ethyl carbonate, ethyl acetate, propylene glycol, 1,3-butanediol, dimethylformamide, and oils, especially cottonseed oil, peanut oil, corn germ oil, olive oil, castor oil and sesame oil, or mixtures of these substances.

Besides such inert diluents, the composition may further comprise adjuvants, such as wetting agents, emulsifiers, suspending agents, sweeteners, flavoring agents, and perfuming agents. Suspensions, in addition to the active compound, may comprise suspending agents, such as ethoxylated isostearyl alcohols, polyoxyethylene sorbitol and sorbitan esters, microcrystalline cellulose, aluminum methylate and agar, or mixtures of these substances.

Compositions for parenteral injection may comprise physiologically acceptable sterile aqueous or anhydrous solutions, dispersions, suspensions or emulsions, and sterile powders for redissolving into sterile injectable solutions or dispersions. Suitable aqueous and non-aqueous carriers, diluents, solvents or excipients include water, ethanol, polyols and suitable mixtures thereof.

Dosage forms for topical administration of the compound of the present invention include ointments, pulvises, patches, sprays and inhalants. The active ingredient is mixed under sterile conditions with a physiologically acceptable carrier and any preservatives, buffers or propellants that may be required if necessary.

The compound of the present invention may be administered alone or in combination with other pharmaceutically acceptable compounds. When the pharmaceutical composition is used, a safe and effective amount of the compound of the present invention is administered to a mammal (such as a human) to be treated, wherein the administration dose is a pharmaceutically effective administration dose. For a human weighing 60 kg, the daily dose of administration is usually 1-2000 mg, preferably 50-1000 mg. In determining a specific dose, such factors as the route of administration, the health condition of the patient and the like will also be considered, which are well known to skilled physicians.

The above features mentioned in the present invention or those mentioned in the examples may be combined arbitrarily. All the features disclosed in this specification may be used with any composition form and the various features disclosed in this specification may be replaced with any alternative features that provide the same, equivalent or similar purpose. Thus, unless otherwise expressly stated, the features disclosed are merely general examples of equivalent or similar features.

### BRIEF DESCRIPTION OF THE DRAWINGS

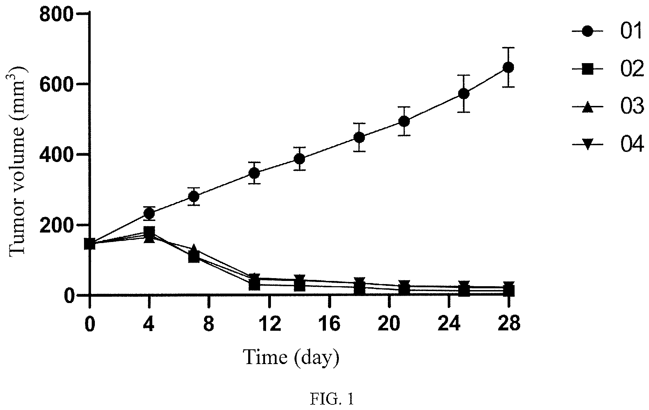

FIG. 1 shows the results of the anti-tumor activity in mice of Example 11 according to the present invention.

### DETAILED DESCRIPTION

Various specific aspects, features and advantages of the compounds, methods and pharmaceutical compositions described above will be set forth in detail in the following description, which will make the content of the present invention very clear. It should be understood that the detailed description and examples below describe specific embodiments for reference only. After reading the description of the present invention, those skilled in the art can make various changes or modifications to the present invention, and such equivalents also fall within the scope of the present invention defined herein.

In all examples, ¹H-NMR spectra were recorded with a Varian Mercury 400 nuclear magnetic resonance spectrometer, and chemical shifts are expressed in δ (ppm); silica gel for separation was 200-300 mesh silica gel if not specified, and the ratio of the eluents was volume ratio.

In the present invention, the following abbreviations are used: room temperature (RT, rt); aqueous solution (aq.); petroleum ether (PE); ethyl acetate (EA); dichloromethane (DCM); 1,4-dioxane (dioxane); methanol (MeOH); methyl tert-butyl ether (MTBE); ethanol (EtOH); tetrahydrofuran (THF); dimethylformamide (DMF); N-methylpyrrolidone (NMP); dimethyl sulfoxide (DMSO); triethylamine (TEA); diisopropylethylamine (DIPEA); 4-dimethylaminopyridine (DMAP); carbon tetrachloride (CCl₄); palladium on carbon (Pd/C); Eaton's reagent (Eaton reagent, 7.7 wt% of phosphorus pentoxide in methanesulfonic acid); iron powder (Fe); zinc powder (Zn); Raney nickel (Ranyi Ni); acetyl chloride (AcCl); acetic acid (AcOH); acetic anhydride (Ac₂O); *m*-chloroperoxybenzoic acid (m-CPBA); butyl nitrite (n-BuNO); sodium nitrite (NaNO₂); sodium hydride (NaH); magnesium sulfate (MgSO₄); *N-*bromosuccinimide (NBS); p-toluenesulfonic acid monohydrate (TsOH.H₂O); sodium carbonate (Na₂CO₃); potassium carbonate (K₂CO₃); equivalent (eq); gram/milligram (g/mg); mole/millimole (mol/mmol); liter/milliliter (L/mL); minutes (min (s)); hours (h, hr, hrs); nitrogen (N₂); nuclear magnetic resonance (NMR); liquid-mass spectrometry (LC-MS); thin-layer chromatography (TLC); preparative liquid chromatography (pre-HPLC).

### Preparation Example 1: Synthesis of N-(5-amino-7-fluoro-8-methyl-4-oxothiochroman-3-yl)acetamide (A7-a)

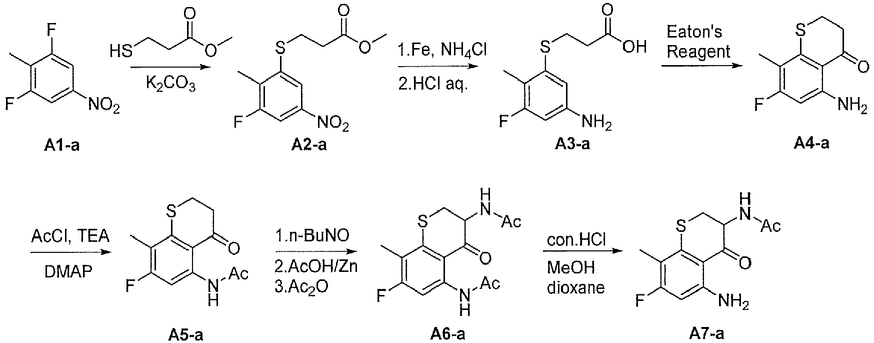

### Step 1: Synthesis of methyl 3-((3-fluoro-2-methyl-5-nitrophenyl)thio)propanoate

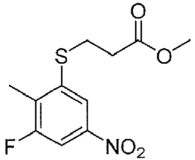

To a 50-mL three-necked flask were added **A1-a** (1.73 g, 10 mmol, 1 eq), methyl 3-mercaptopropanoate (1.8 g, 15 mmol, 1.5 eq) and NMP (10 mL), and after the system was dissolved, potassium carbonate (2 g, 15 mmol, 1.5 eq) was added and stirred at 60 °C for 8 hours under the atmosphere of argon. After the system was cooled to room temperature, water (30 mL) was added to dilute the system, and the precipitated solid was filtered and washed with water. The filter cake was separated by column chromatography (PE/EA = 1/12-1/7) to give a yellow solid **A2-a** (1.55 g, 56.8% yield), LC-MS: 274.2 [M+H]⁺.

### Step 2: Synthesis of 3-((3-fluoro-2-methyl-5-aminophenyl)thio)propionic acid

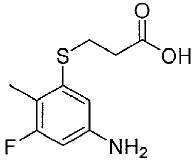

To a 250-mL three-necked flask were added **A2-a** (1.55 g, 5.67 mmol, 1 eq), iron powder (1.27 g, 22.69 mmol, 4 eq), ethanol (80 mL) and aqueous ammonium chloride (2.8 M, 28 mL, 5 eq), and the system was stirred at 90 °C for 16 hours under the atmosphere of argon. After the system was cooled to room temperature, the system was filtered through celite and washed with ethanol. The filtrate was concentrated, and the crude product was diluted with water (30 mL) and extracted with EA (30 mL × 2). The organic phase was washed with saturated brine, dried over sodium sulfate and concentrated to give the crude product (1.5 g, equivalent yield). LC-MS: 244.3 [M+H]⁺.

To a 50-mL three-necked flask were added the above crude product (1.5 g, 5.67 mmol, 1 eq) and 1,4-dioxane (15 mL), and after the system was dissolved, concentrated hydrochloric acid (37%, 10 mL) was added and stirred at 65 °C for 4 hours under the atmosphere of argon. After the system was cooled to room temperature, 3 N sodium carbonate solution was added to adjust the pH to 5. The system was extracted with EA (30 mL × 2), and the organic phase was washed with saturated brine, dried over sodium sulfate and concentrated. The crude product was slurried (EA/PE = 1/5) to give a white solid **A3-a** (985 mg, 75.8% yield over two steps), LC-MS: 228.2 [M-H]⁻.

### Step 3: Synthesis of 5-amino-7-fluoro-8-methylthiochroman-4-one

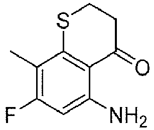

To a 50-mL three-necked flask were added **A3-a** (985 mg, 4.3 mmol, 1 eq) and Eaton's Reagent (15 mL), and the system was stirred at 60 °C for 1 hour under the atmosphere of argon. After the system was cooled to room temperature, the reaction solution was poured into ice water, and 3 N sodium carbonate solution was added to adjust the pH to 8. The system was extracted with EA (30 mL × 2), and the organic phase was washed with saturated brine, dried over sodium sulfate and concentrated to give the crude product **A4-a** (1.05 g, equivalent yield), LC-MS: 212.3 [M+H]⁺.

### Step 4: Synthesis of N-(7-fluoro-8-methyl-4-oxothiochroman-5-yl)acetamide

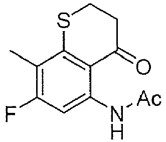

To a 50-mL three-necked flask were added **A4-a** (1.05 g, 4.3 mmol, 1 eq), DMAP (52.5 mg, 0.43 mmol, 0.1 eq) and DCM (15 mL), acetyl chloride (674 mg, 8.6 mmol, 2 eq) and triethylamine (869 mg, 8.6 mmol, 2 eq) were added in sequence under an ice bath. The system naturally returned to room temperature and was then stirred for 1 hour until the starting materials were completely consumed. The system was quenched with water (20 mL), followed by separation. The aqueous phase was extracted with DCM (20 mL × 2), and the organic phases were combined, washed with saturated brine, dried and concentrated. The residue was subjected to column chromatography (EA/PE = 1/1) to give a yellow solid **A5-a** (910 mg, 89% yield over two steps), LC-MS: 254.3 [M+H]⁺.

### Step 5: Synthesis of N,N-(7-fluoro-8-methyl-4-oxothiochromane-3,5-diyl)diacetamide

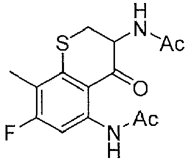

To a 50-mL three-necked flask were added potassium *tert*-butoxide (191 mg, 1.7 mmol, 1.1 eq) and anhydrous THF (5 mL), and **A5-a** (375 mg, 1.48 mmol, 1 eq) and butyl nitrite (190 mg, 1.85 mmol, 1.25 eq) were added in sequence at -20 °C. The system was heated to 5 °C and then stirred for 2 hours until the starting materials were completely consumed. The system was added with MTBE (15 mL) for dilution and filtered, and the solid was dissolved in acetic acid (5 mL), added with zinc powder (200 mg, 3.1 mmol, 2.1 eq), stirred at room temperature for 5 minutes, added with acetic anhydride (1 mL), and stirred for 2 hours. The system was washed with MeOH/DCM (3/30 mL) and concentrated, and the crude product was separated by column chromatography (EA/DCM = 1/10-1/5) to give a light brown solid **A6-a** (175 mg, 41%), LC-MS: 311.1 [M+H]⁺. Step 6: Synthesis of *N*-(5-amino-7-fluoro-8-methyl-4-oxothiochroman-3-yl)acetamide

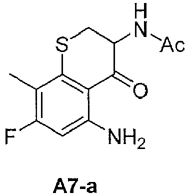

To a 50-mL three-necked flask were added **A6-a** (175 mg, 0.56 mmol, 1 eq) and methanol/1,4-dioxane (4/8 mL), and after the system was dissolved, concentrated hydrochloric acid (37%, 4 mL) was added and stirred at 40 °C for 2 hours under the atmosphere of argon. After the system was cooled to room temperature, 3 N sodium carbonate solution was added to adjust the pH to 8. The system was filtered, and the solid was dried to give **A7-a** (137 mg, 91% yield). LC-MS: 269.2 [M+H]⁺.

Similar to the synthesis of **A7**-a, the intermediates listed in the following table can be obtained:

**Table 1. Intermediates A7-b to A7-y**

| No. | **Structure** | MS [M+H]⁺ | **No.** | **Structure** | MS [M+H]⁺ |
|---|---|---|---|---|---|
| **A7-b** | 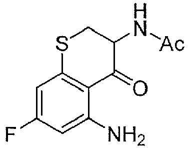 | 255.2 | **A7-e** | 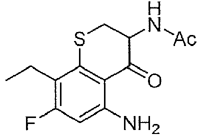 | 283.1 |
| **A7-d** | 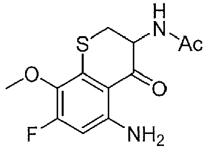 | 285.1 | **A7-e** | 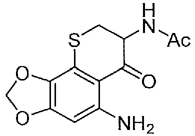 | 281.1 |
| **A7-f** | 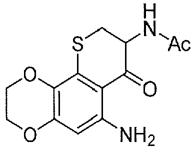 | 295.1 | **A7-g** | 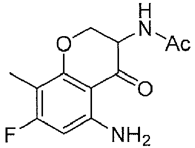 | 253.2 |
| **A7-h** | 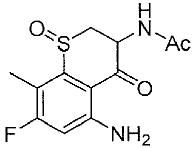 | 285.2 | **A7-i** | 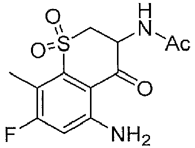 | 301.2 |
| **A7-j** | 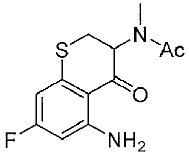 | 269.1 | **A7-k** | 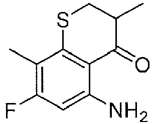 | 226.2 |
| **A7-l** | 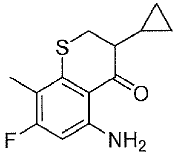 | 252.1 | **A7-m** | 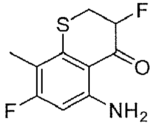 | 230.1 |
| **A7-n** | 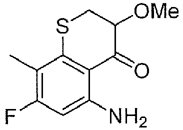 | 242.2 | **A7-o** | 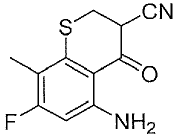 | 237.1 |
| **A7-p** | 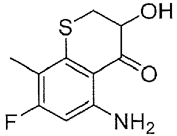 | 228.3 | **A7-q** | 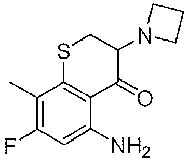 | 267.2 |
| **A7-r** | 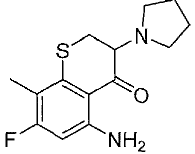 | 281.2 | **A7-s** | 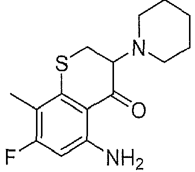 | 295.2 |
| **A7-t** | 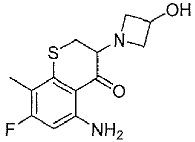 | 283.2 | **A7-u** | 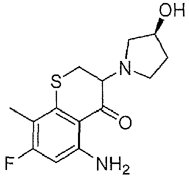 | 297.2 |
| **A7-v** | 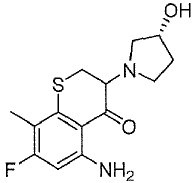 | 297.2 | **A7-w** | 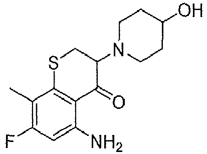 | 311.2 |
| **A7-x** | 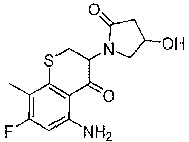 | 311.1 | **A7-y** | 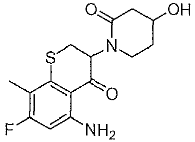 | 325.1 |

### Preparation Example 2: Synthesis of (S)-4-(2-fluoroethyl)-4-hydroxy-7,8-dihydro-1H-pyrano[3,4-f]indolizine-3,6,10(4H)-trione (B7-a)

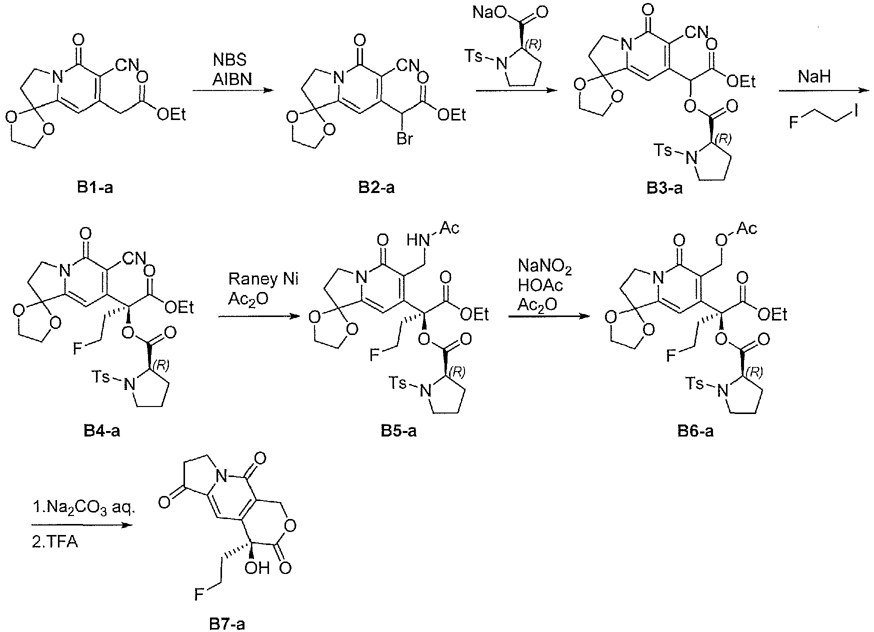

### Step 1: Synthesis of ethyl 2-bromo-2-(6-cyano-5-oxo-2,3-dihydro-5H-spiro[indolizine-1,2'-[1,3] dioxolan] -7-yl)acetate

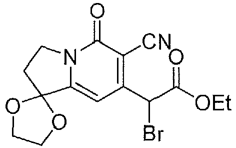

**B1-a** (3 g, 9.9 mmol, 1 eq) was dissolved in DMF (75 mL), NBS (1.5 g, 12 mmol, 1.2 eq) and m-CPBA (170 mg, 1 mmol, 0.1 eq) were added, and the system was stirred at room temperature overnight, then poured into 500 mL of ice water, and filtered. The solid was washed with water and dried to give a gray solid **B2-a** (3.8 g, equivalent yield), LC-MS: 383.2 [M+H]⁺.

### Step 2: Synthesis of 1-(6-cyano-5-oxo-2,3-dihydro-5H-spiro[indolizine-1,2'-[1,3]dioxolan]-7-yl)-2-ethoxy-2-oxoethyl tosyl-D-prolinate

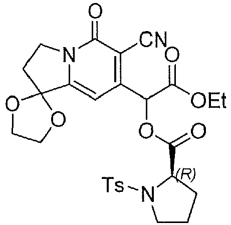

**B2-a** (1 g, 2.6 mmol, 1 eq), sodium tosyl-*D*-prolinate (1 g, 3.9 mmol, 1.5 eq) and K₂CO₃ (362 mg, 2.6 mmol, 1 eq) are dissolved in DMF (20 mL), and the system was stirred at 65 °C for 2 hours under the atmosphere of argon until the starting materials were completely consumed. The system was added with water (100 mL) for dilution and extracted with EA (100 mL × 3), and the organic phases were combined and washed twice with water, washed with saturated brine, dried and concentrated. The residue was separated by column chromatography (EA/DCM = 1/6) to give a white solid **B3-a** (1.1 g, 74% yield), LC-MS: 572.2 [M+H]⁺.

### Step 3: Synthesis of (S)-2-(6-cyano-5-oxo-2,3-dihydro-5H-spiro[indolizine-1,2'-[1,3]dioxolan-7-yl)-1 -ethoxy-4-fluoro-1 -oxobutan-2-yl tosyl-D-prolinate

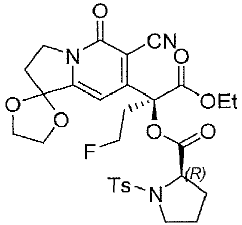

**B3-a** (1 g, 1.7 mmol, 1 eq) was dissolved in DMF (20 mL), and NaH (101 mg, 60%, 2.5 mmol, 1.5 eq) was added under an ice bath. The system returned to room temperature, stirred for 1 hour, then added with 2-fluoroiodoethane (1.5 g, 8.6 mmol, 5 eq) under an ice bath, naturally returned to room temperature, and stirred overnight. After the reaction was completed, the system was poured into ice water (100 mL) and extracted with EA (100 mL × 3), and the organic phases were combined, washed twice with water, washed with saturated saline, dried and concentrated. The residue was separated by column chromatography (EA/DCM = 1/6) to give 743 mg of crude product, which was subjected to Pre-HPLC to give a white solid **B4-a** (150 mg, 70% de, 15% yield), LC-MS: 618.2 [M+H]⁺.

### Step 4: Synthesis of (S)-2-(6-(acetamidomethyl)-5-oxo-2,3-dihydro-5H-spiro[indolizine-1,2'-[1,3]dioxolan]-7-yl)-1-ethoxy-4-fluoro-1-oxobutan-2-yl tosyl-D-prolinate

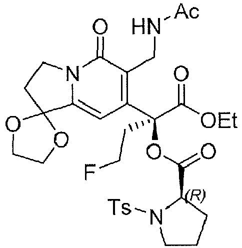

To a 50-mL three-necked flask was added Raney Ni (600 mg, water content of 50%), and the system was washed three times with HOAc, added with a solution of **B4-a** (150 mg, 0.24 mmol, 1 eq) in Ac₂O/HOAc (4/1 mL) under the atmosphere of argon, purged three times with hydrogen, reacted at 65 °C for 3 hours and filtered. The solid was washed with AcOH, and the filtrate was concentrated and separated by column chromatography (MeOH/DCM = 1/20) to give a colorless oily solution **B5-a** (130 mg, 83% yield), LC-MS: 664.2 [M+H]⁺.

### Step 5: Synthesis of (S)-2-(6-(acetoxymethyl)-5-oxo-2,3-dihydro-5H-spiro[indolizine-1,2'-[1,3]dioxolan]-7-yl)-1-ethoxy-4-fluoro-1-oxobutan-2-yl tosyl-D-prolinate

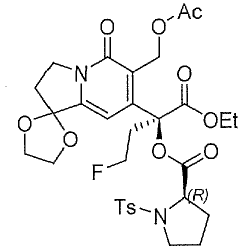

**B5-a** (130 mg, 0.2 mmol, 1 eq) was dissolved in Ac₂O/HOAc (3/1 mL), and NaNO₂ (68 mg, 1 mmol, 5 eq) was added under an ice bath. The system returned to room temperature and stirred for 1 hour. After the reaction was completed, the system was filtered, the solid was washed with AcOH, and the filtrate was concentrated, added with CCl₄ (15 mL), and stirred under reflux overnight. The system was washed with water, washed with saturated brine, dried and concentrated. The residue was separated by column chromatography (MeOH/DCM = 1/20) to give 90 mg of colorless oily solution, which was subjected to Pre-HPLC to give a white solid **B6-a** (90 mg, 69% yield), LC-MS: 665.2 [M+H]⁺.

### Step 6: Synthesis of (S)-4-(2-fluoroethyl)-4-hydroxy-7,8-dihydro-1H-pyrano[3,4-f]indolizine-3,6,10(4H)-trione

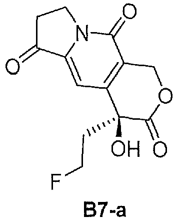

**B6-a** (90 mg, 0.14 mmol, 1 eq) was dissolved in EtOH (3 mL), and 1 N aqueous sodium carbonate (1 mL) was added. The system was stirred at room temperature for 1 hour until the starting materials were completely consumed, then concentrated at room temperature and lyophilized. The crude product was dissolved in 85% aqueous TFA (5 mL) and stirred at 85 °C for 1 hour. After the reaction was completed, the system was concentrated, and the crude product was separated by Pre-HPLC to give a white solid **B7-a** (7 mg, 17% yield, 68% de), LC-MS: 282.2 [M+H]⁺.

Similar to the synthesis of **B7-a**, the intermediates listed in the following table can be obtained:

**Table 2. Intermediates B7-b to B7-d**

| **No.** | **Structure** | MS [M+H]⁺ | **No.** | **Structure** | MS [M+H]⁺ |
|---|---|---|---|---|---|
| **B7-b** | 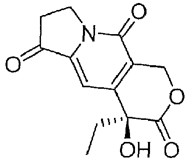 | 264.1 | **B7-c** | 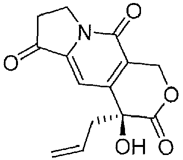 | 276.1 |
| **B7-d** | 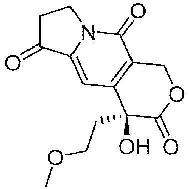 | 294.1 | | | |

### Example 1: Synthesis of (9S)-1-amino-9-ethyl-5-fluoro-9-hydroxy-4-methyl-1,9,12,15-tetrahydro-13H-pyrano[3',4':6,7]indolizino[1,2-b]thiopyrano[4,3,2-de]quinoline-10,13(2H)-dione (Compound 1)

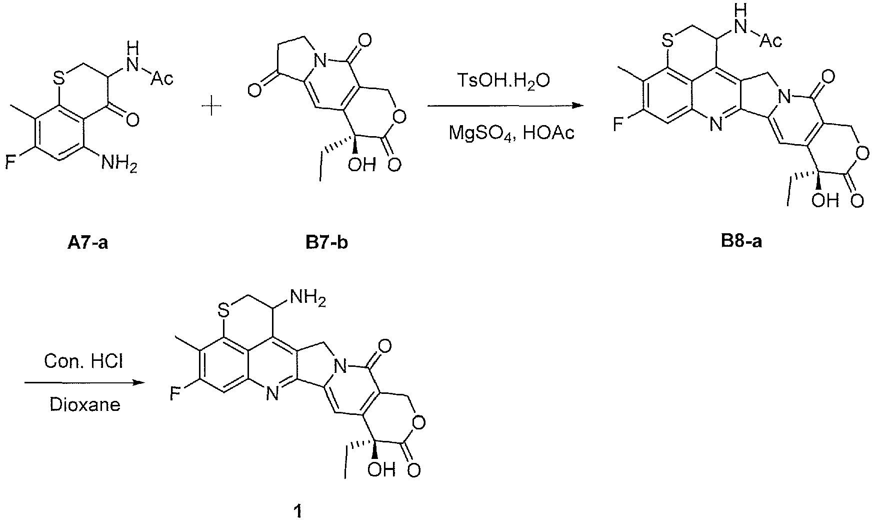

### Step 1: Synthesis of N-((9S)-9-ethyl-5-fluoro-9-hydroxy-4-methyl-10,13-dioxo-1,2,9,10,13,15-hexahydro-12H-pyrano[3',4':6,7]indolizino[1,2-b]thiopyrano[4,3,2-de]quinolin-1-yl)acetamide

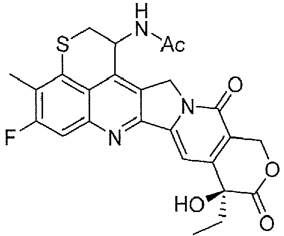

To a 50-mL three-necked flask were added **A7-a** (108 mg, 0.4 mmol, 1 eq), **B7-b** (156 mg, 0.6 mmol, 1.5 eq), *p*-toluenesulfonic acid monohydrate (45 mg, 0.24 mmol, 0.6 eq), anhydrous magnesium sulfate (1 g) and acetic acid (10 mL), and the system were stirred at 105 °C for 24 hours under the atmosphere of argon until the starting materials were completely consumed. The system was filtered, and the filter cake was washed with EA and concentrated. The crude product was separated by column chromatography (MeOH/DCM = 1/40-1/20) to give a light brown solid **B8-a** (131 mg, 67%), LC-MS: 496.2 [M+H]⁺.

### Step 2: Synthesis of (9S)-1-amino-9-ethyl-5-fluoro-9-hydroxy-4-methyl-1,9,12,15-tetrahydro-13H-pyrano [3',4':6,7]indolizino[1,2-b]thiopyrano[4,3,2-de]quinoline-10,13(2H)-dione

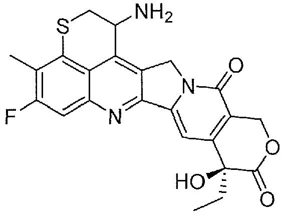

To a 50-mL three-necked flask were added **B8-a** (131 mg, 0.26 mmol, 1 eq) and 1,4-dioxane (5 mL), and after the system was dissolved, concentrated hydrochloric acid (37%, 5 mL) was added and stirred at 80 °C for 24 hours under the atmosphere of argon. The system was cooled to room temperature and then concentrated. The crude product was slurried with ACN/EA (1/1) to give a brown solid 1 (hydrochloride) (107 mg, 84.6% yield), LC-MS: 454.2 [M+H]⁺.

Similar to the synthesis of compound **1**, the compounds listed in the following table can be obtained:

**Table 3. List of compounds 2-31**

| **No.** | **Structure** | MS [M+H]⁺ | **No.** | **Structure** | MS [M+H]⁺ |
|---|---|---|---|---|---|
| **2** | 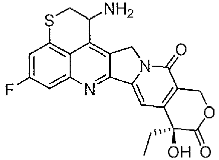 | 440.1 | **3** | 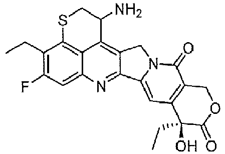 | 468.2 |
| **4** | 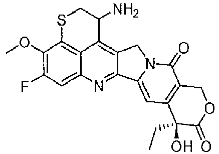 | 470.2 | **5** | 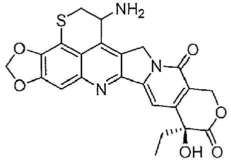 | 466.1 |
| **6** | 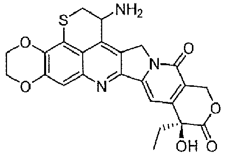 | 480.2 | **7** | 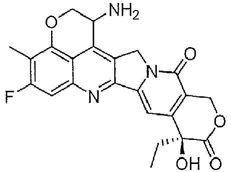 | 438.2 |
| **8** | 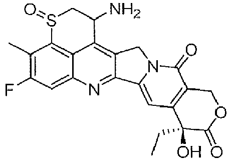 | 470.2 | **9** | 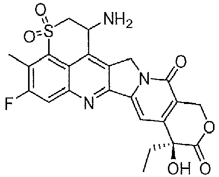 | 486.1 |
| **10** | 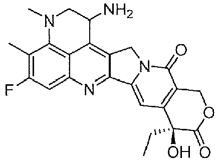 | 451.2 | **11** | 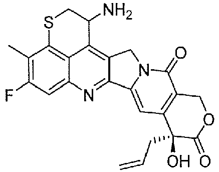 | 466.1 |
| **12** | 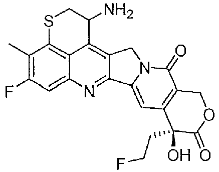 | 472.1 | **13** | 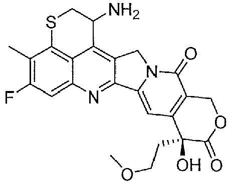 | 484.2 |
| **14** | 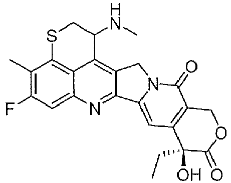 | 468.2 | **15** | 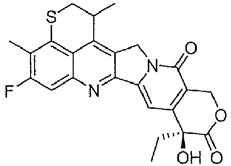 | 453.2 |
| **16** | 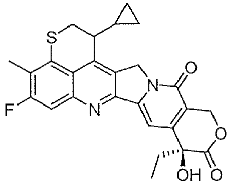 | 479.2 | **17** | 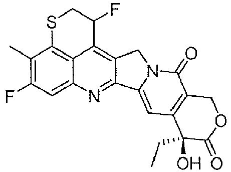 | 457.1 |
| **18** | 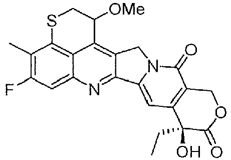 | 469.2 | **19** | 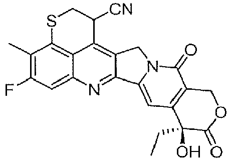 | 464.1 |
| **20** | 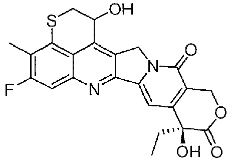 | 455.1 | **21** | 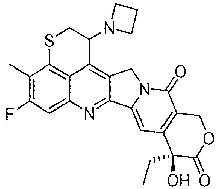 | 494.2 |
| **22** | 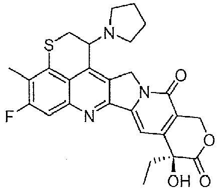 | 508.2 | **23** | 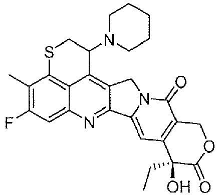 | 522.2 |
| **24** | 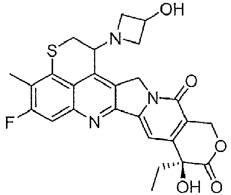 | 510.1 | **25** | 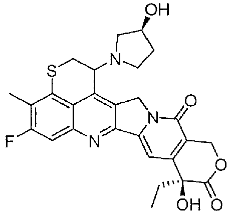 | 524.2 |
| **26** | 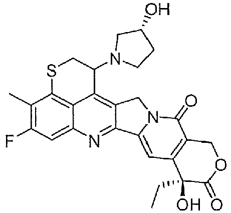 | 524.2 | **27** | 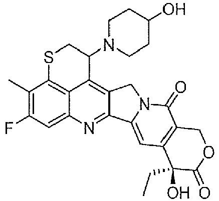 | 538.2 |
| **28** | 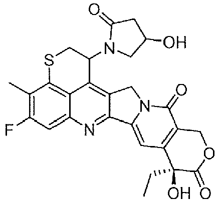 | 538.2 | **29** | 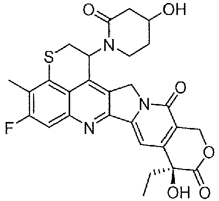 | 552.2 |
| **30** | 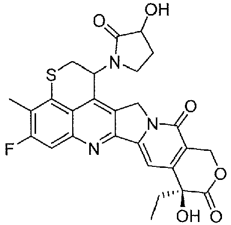 | 538.2 | **31** | 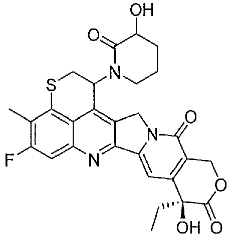 | 552.2 |

### Example 2: Chiral Separation of Compound 1

Compound **1** is a pair of diastereomeric mixture, and two diastereoisomers 1-1 and 1-2 of compound **1** can be obtained by adopting a method of salification and recrystallization or the separation and purification with pre-HPLC.

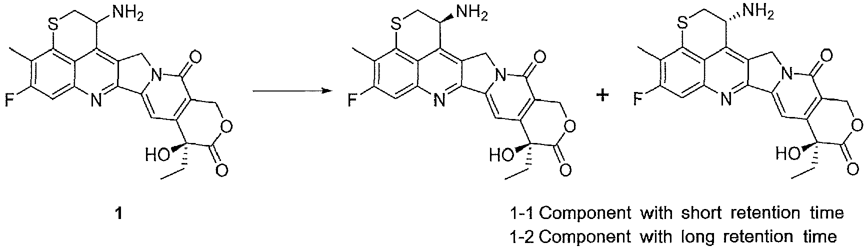

Conditions for chromatography: the preparative liquid chromatograph equipped with Shimadzu LC-20AP; chromatographic column: Waters SunFire Prep C18 OBD (50 × 150 mm, 5 µm); mobile phase: acetonitrile-0.5%o aqueous trifluoroacetic acid solution = 66:34; flow rate: 48.0 mL/min; detection wavelength: 254 nm; injection volume: 3000 µL.

The experimental procedures were as follows: a proper amount of **1** was taken and brought to a certain volume by using a 50% aqueous acetonitrile solution. A test sample solution with a concentration of 25 mg/mL was prepared. The test sample solution was taken and placed into the preparative liquid chromatograph for detection according to the conditions for chromatography of the present invention and then data were recorded.

As a result, the above solution was separated by pre-HPLC to give **1-1** (33 mg) and **1-2** (31 mg). The retention times of the two components were 5.419 minutes and 7.614 minutes, respectively, and the purities thereof were 99.38% and 99.21%, respectively.

Similar to the chiral separation method of compound 1, the compounds listed in the following table can be obtained:

**Table 4. Separation of compounds by Pre-HPLC**

| **No.** | **Structure** | **No.** | **Structure** |
|---|---|---|---|
| **2-1** | 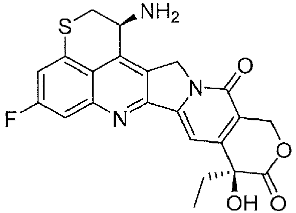 | **2**-**2** | 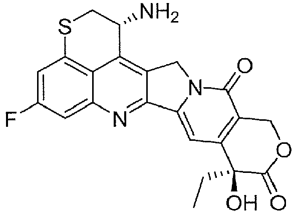 |
| **3-1** | 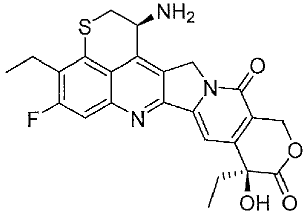 | **3-2** | 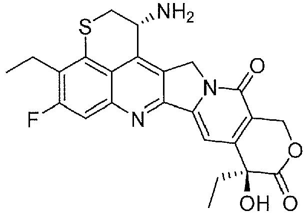 |
| **4-1** | 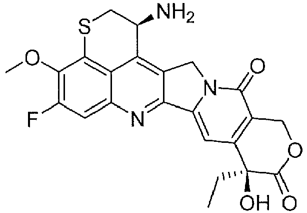 | **4-2** | 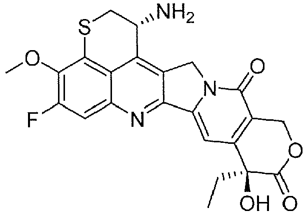 |
| **5-1** | 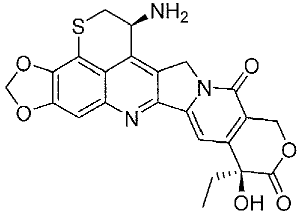 | **5-2** | 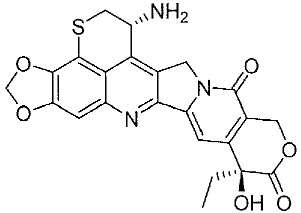 |
| **6-1** | 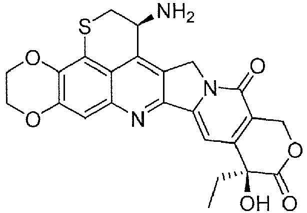 | **6-2** | 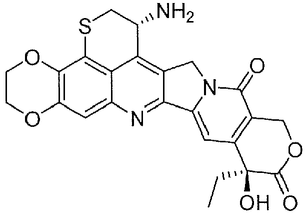 |
| **7-1** | 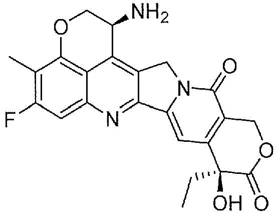 | **7-2** | 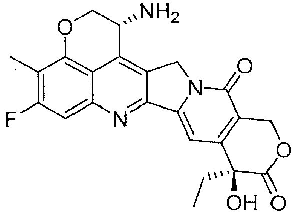 |
| **9-1** | 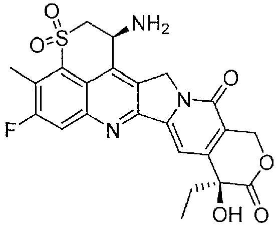 | **9-2** | 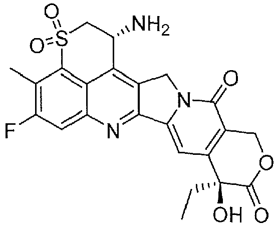 |
| **11-1** | 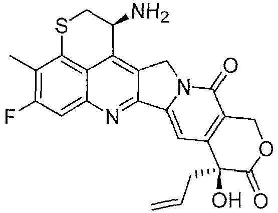 | **11-2** | 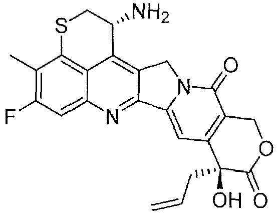 |
| **12-1** | 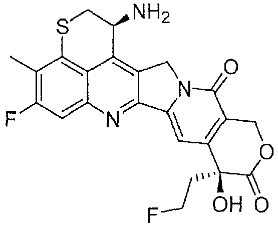 | **12-2** | 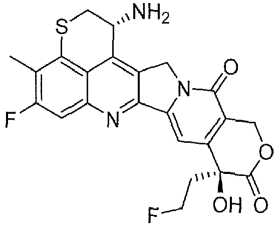 |
| **13-1** | 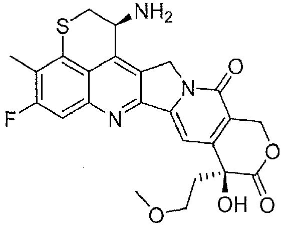 | **13-2** | 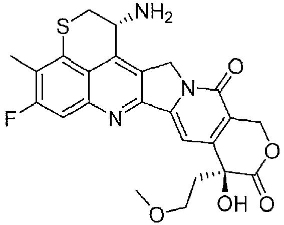 |
| **14-1** | 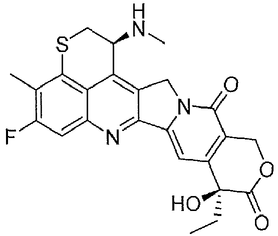 | **14-2** | 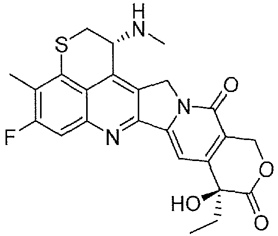 |

### Example 3: Synthesis of Compound 32-1 and Compound 32-2

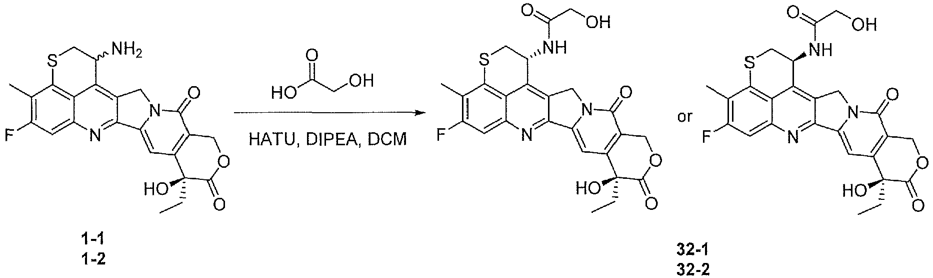

To a 50-mL three-necked flask were added component **1-2** (98 mg, 0.2 mmol, 1 eq) with a long retention time, 2-hydroxyacetic acid (18.4 mg, 0.24 mmol, 1.2 eq) and anhydrous DCM (5 mL), and HATU (84 mg, 0.3 mmol, 1.5 eq) and DIPEA (90.3 mg, 0.7 mmol, 3.5 eq) were added in sequence under an ice bath. After the temperature was maintained for 0.5 hours, the system was added with water (5 mL) for dilution, followed by liquid separation and extraction with DCM (5 mL × 2). The organic phase was washed with saturated saline, dried and concentrated. The residue was subjected to column chromatography (MeOH/DCM = 1/40) to give compound **32-2** (75 mg, 73% yield).

¹H NMR (400 MHz, DMSO-*d*₆) δ: 8.21 (dd, *J* = 13.3, 8.5 Hz, 1H), 7.78 (d, *J=* 10.6 Hz, 1H), 7.31 (d, *J=* 1.2 Hz, 1H), 6.53 (s, 1H), 5.89-5.79 (m, 1H), 5.56-5.49 (m, 1H), 5.43 (s, 2H), 5.38 (s, 1H), 5.31 (d, *J* = 4.8 Hz, 1H), 3.90 (d, *J* = 4.7 Hz, 2H), 3.50 -3.35 (m, 3H), 2.44 (s, 3H), 1.91-1.80 (m, 2H), 0.87 (t, *J=* 6.5 Hz, 3H), LC-MS: 512.2 [M+H]⁺.

Similar to the synthesis of compound **32-2**, a diastereoisomer **32-1** of compound **32-2** can be obtained by using another component **1-1** with a short retention time.

Similar to the synthesis of compound **32-1** and compound **32-2**, the compounds listed in the following table can be obtained by using different intermediates:

**Table 5. List of compounds 33-96**

| **No.** | **Structure** | MS [M+H]⁺ | **No.** | **Structure** | MS [M+H]⁺ |
|---|---|---|---|---|---|
| **33** | 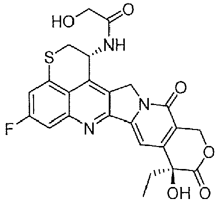 | 498.1 | **34** | 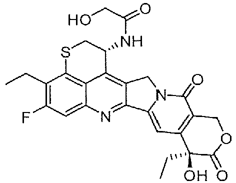 | 526.2 |
| **35** | 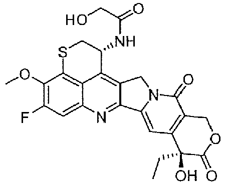 | 528.1 | **36** | 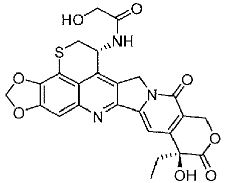 | 524.1 |
| **37** | 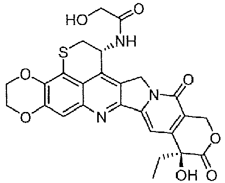 | 538.1 | **38** | 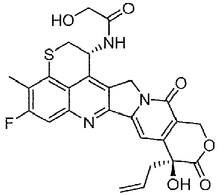 | 524.1 |
| **39** | 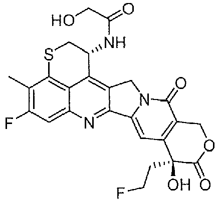 | 530.1 | **40** | 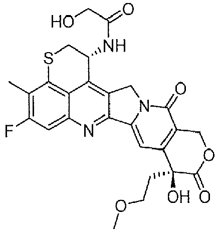 | 542.1 |
| **41** | 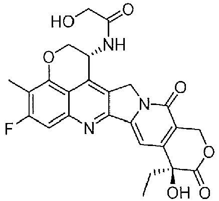 | 496.2 | **42** | 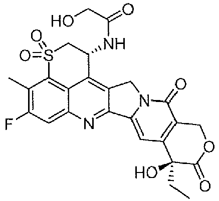 | 544.1 |
| **43** | 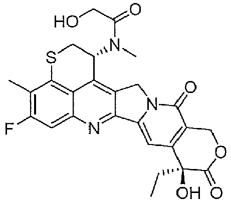 | 526.2 | **44** | 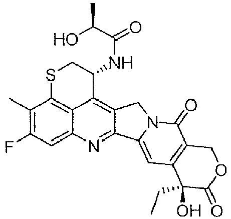 | 526.2 |
| **45** | 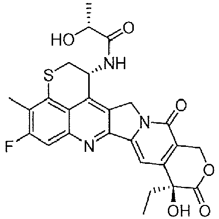 | 526.2 | **46** | 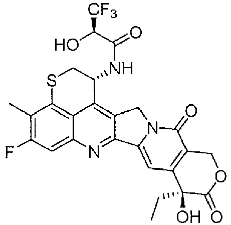 | 580.1 |
| **47** | 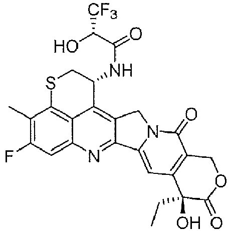 | 580.1 | **48** | 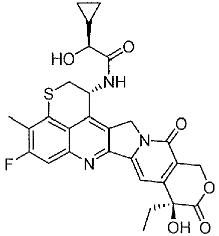 | 552.2 |
| **49** | 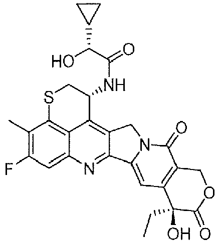 | 552.2 | **50** | 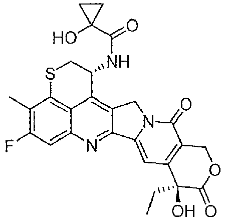 | 538.2 |
| **51** | 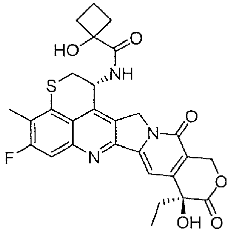 | 552.2 | **52** | 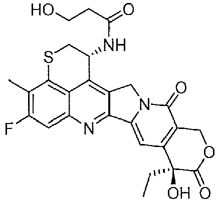 | 526.2 |
| **53** | 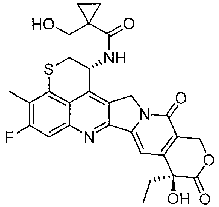 | 552.2 | **54** | 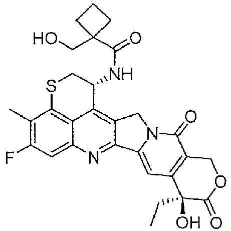 | 566.2 |
| **55** | 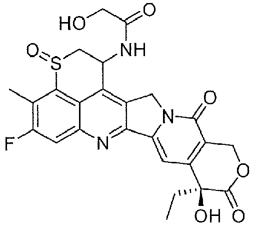 | 528.1 | **56** | 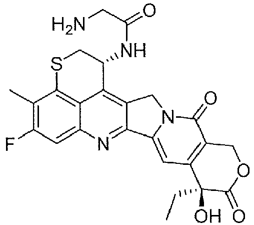 | 511.1 |
| **57** | 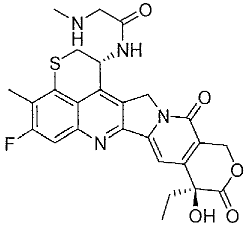 | 525.2 | **58** | 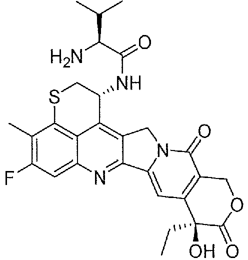 | 553.2 |
| **59** | 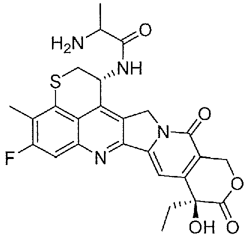 | 525.2 | **60** | 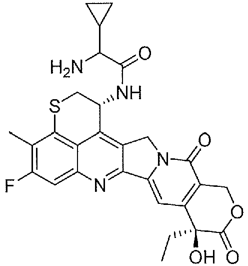 | 551.2 |
| **61** | 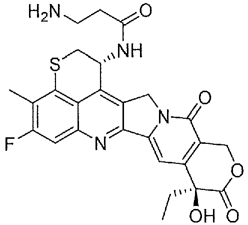 | 525.2 | **62** | 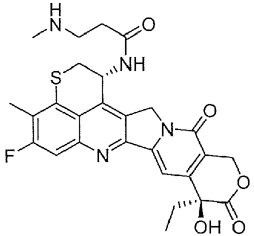 | 539.2 |
| **63** | 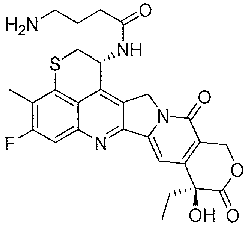 | 539.2 | **64** | 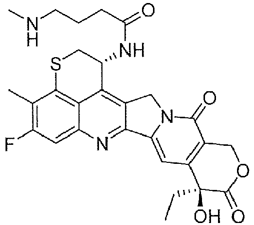 | 553.2 |
| **65** | 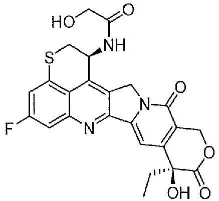 | 498.1 | **66** | 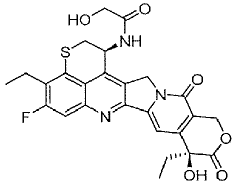 | 526.2 |
| **67** | 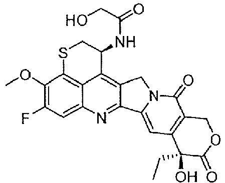 | 528.1 | **68** | 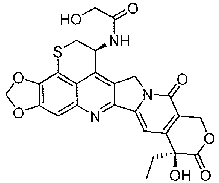 | 524.1 |
| **69** | 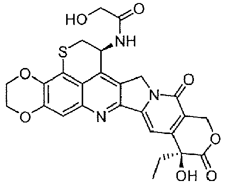 | 538.1 | **70** | 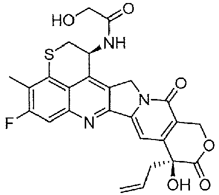 | 524.1 |
| **71** | 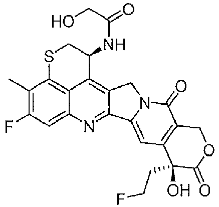 | 530.1 | **72** | 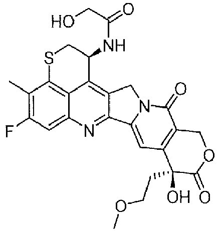 | 542.1 |
| **73** | 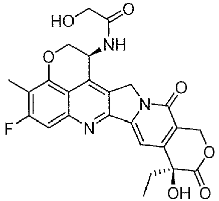 | 496.2 | **74** | 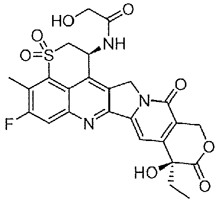 | 544.1 |
| **75** | 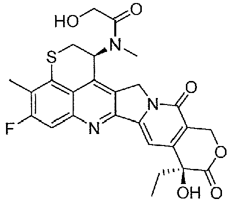 | 526.2 | **76** | 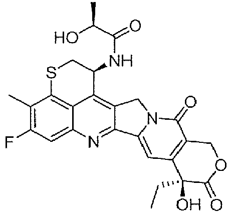 | 526.2 |
| **77** | 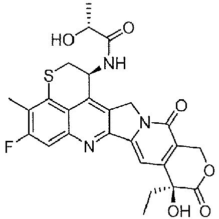 | 526.2 | **78** | 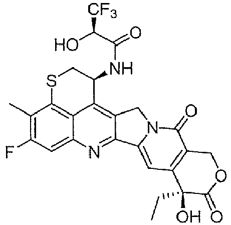 | 580.1 |
| **79** | 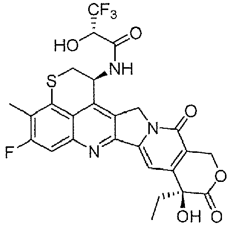 | 580.1 | **80** | 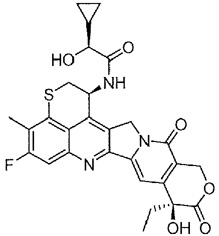 | 552.2 |
| **81** | 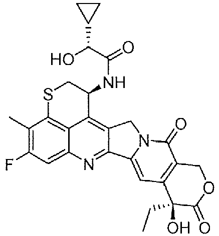 | 552.2 | **82** | 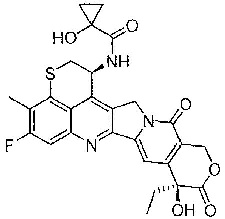 | 538.2 |
| **83** | 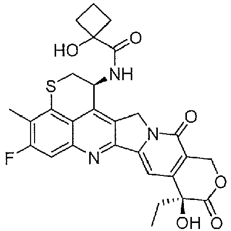 | 552.2 | **84** | 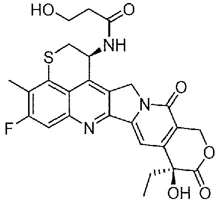 | 526.2 |
| **85** | 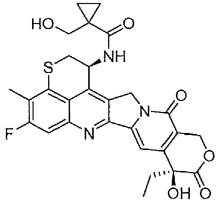 | 552.2 | **86** | 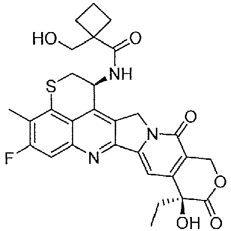 | 566.2 |
| **87** | 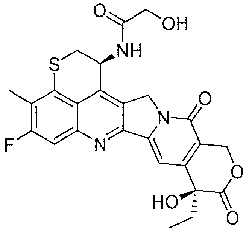 | 512.2 | **88** | 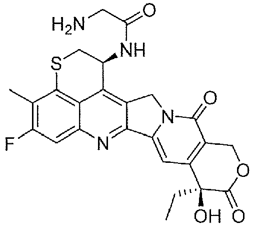 | 511.1 |
| **89** | 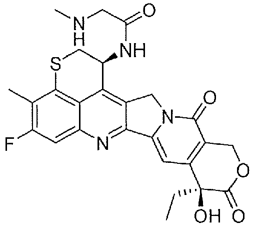 | 525.2 | **90** | 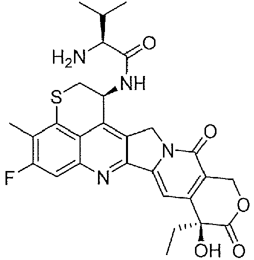 | 553.2 |
| **91** | 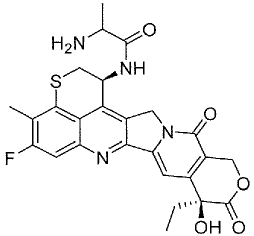 | 525.2 | **92** | 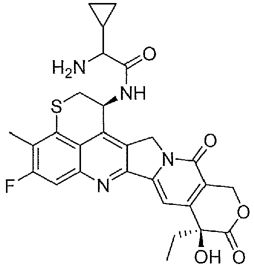 | 551.2 |
| **93** | 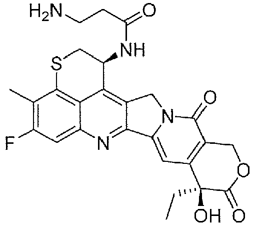 | 525.2 | **94** | 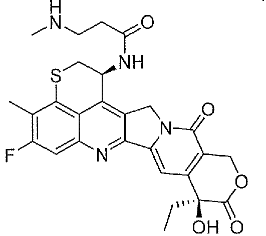 | 539.2 |
| **95** | 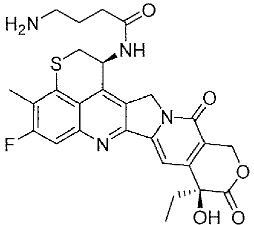 | 539.2 | **96** | 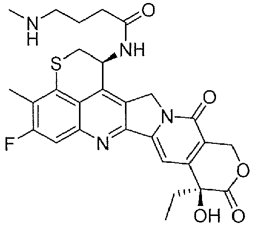 | 553.2 |

### Example 4: Preparation of Antibody-Drug Conjugate (ADC-1)

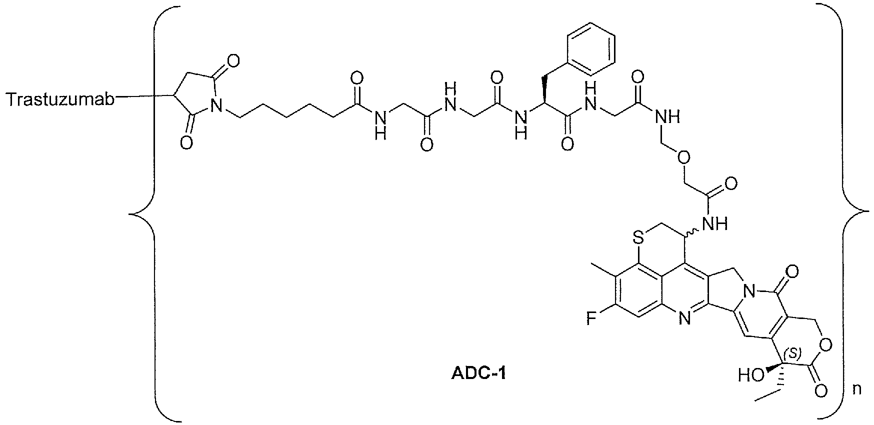

### Step 1: Preparation of compound L-D-1

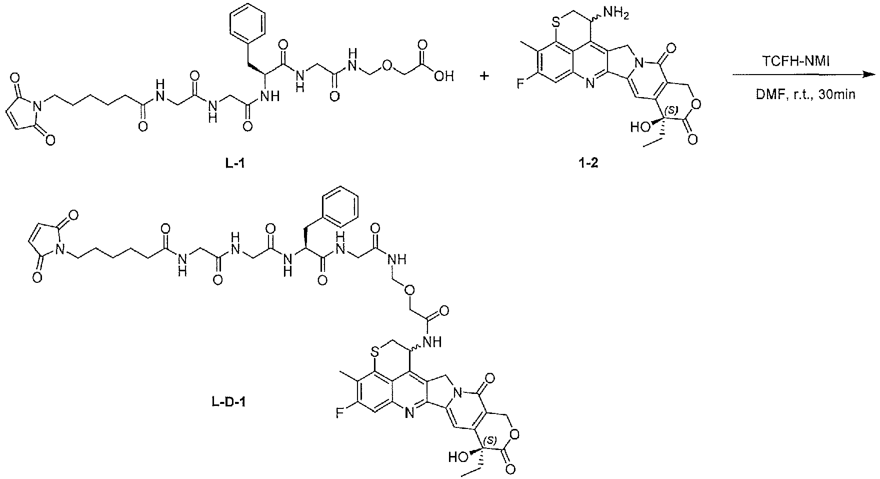

To a 50-mL single-necked flask were added **L-1** (76 mg, 0.12 mmol, 1.0 eq), component compound **1-2** (55 mg, 0.12 mmol, 1.0 eq) with a long retention time, NMI (50.6 mg, 0.62 mmol, 5.0 eq) and DMF (2 mL), and the system was stirred well and cooled to 0 °C. The reaction solution was added with TCFH (41.5 mg, 0.15 mmol, 1.2 eq) and stirred for 30 minutes, followed by detection by LC-MS. After the reaction was completed, the reaction solution was purified by reversed-phase C18 column chromatography (MeCN/water = 0-60%), and fractions of the target substance were lyophilized to give a yellow solid **L-D-1** (80 mg, 61.5% yield).

¹H-NMR (400 MHz, DMSO-*d*₆) δ: 8.56 (t, *J* = 6.4 Hz, 1H), 8.30 (dd, *J* = 14.0, 8.3 Hz, 2H), 8.11 (d, *J* = 7.9 Hz, 1H), 8.06 (t, *J* = 5.7 Hz, 1H), 7.99 (t, *J* = 5.7 Hz, 1H), 7.76 (d, *J* = 10.7 Hz, 1H), 7.31 (d, *J* = 2.8 Hz, 1H), 7.27-7.12 (m, 5H), 6.98 (s, 2H), 6.54 (d, *J* = 1.8 Hz, 1H), 5.84-5.83 (m, 1H), 5.49-5.31 (m, 3H), 5.27-5.21 (m, 1H), 4.66-4.53 (m, 2H), 4.48-4.41 (m, 1H), 3.98 (s, 2H), 3.75-3.54 (m, 6H), 3.42 (s, 2H), 3.36-3.35 (m, 2H), 3.04-2.98 (m, 1H), 2.80-2.74 (m, 1H), 2.42 (s, 3H), 2.08 (t, *J* = 7.4, 2H), 1.93-1.79 (m, 2H), 1.50-1.41 (dd, *J* = 13.2, 5.9 Hz, 4H), 1.25-1.12 (m, 2H), 0.88-0.84 (m, 3H), LC-MS: 1052.1 [M+H]⁺, 1050.1 [M-H]⁻.

### Step 2: Preparation of ADC-1

To an aqueous PBS buffer (0.05 PBS buffer at pH = 6.5; 2.5 mL, 9.96 mg/mL, 0.168 nmol) of antibody trastuzumab was added a prepared aqueous solution of tris(2-carboxyethyl)phosphine (10 mM, 0.082 mL) at 37 °C, and the system was placed in a water bath shaker, and shaken and reacted at 37 °C for 3 hours before the reaction was stopped; the reaction solution was cooled to 25 °C under a water bath and diluted to 5.0 mg/mL.

Compound **L-D-1** (2.02 nmol) was dissolved in DMSO (0.10 mL) and added to 2.0 mL of the above solution, and the system was placed in a water bath shaker, and shaken and reacted at 25 °C for 3 hours before the reaction was stopped. The reaction solution was desalted and purified through a Sephadex G25 gel column (elution phase: 0.05 M PBS buffer at pH 6.5, containing 0.001 M EDTA) to give a PBS buffer (5.0 mg/mL, 1.1 mL) of ADC, which was frozen and stored at 4 °C. Mean calculated by UV-HPLC: n = 7.2.

### Example 5: Preparation of Antibody-Drug Conjugate (ADC-2)

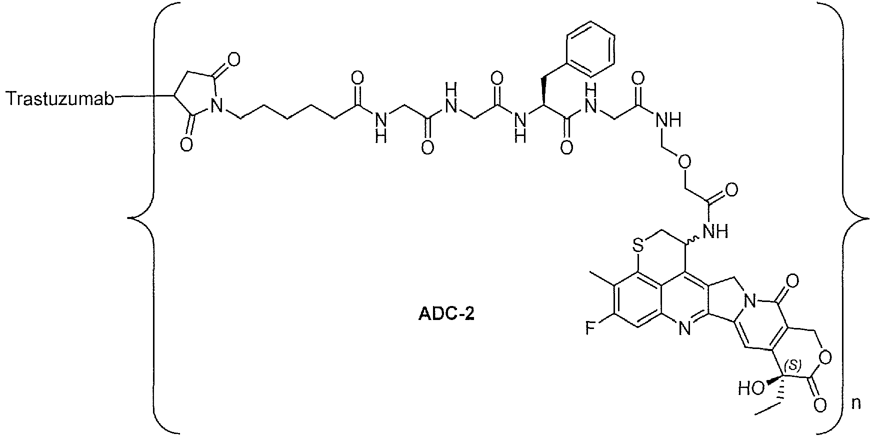

### Step 1: Preparation of compound L-D-2

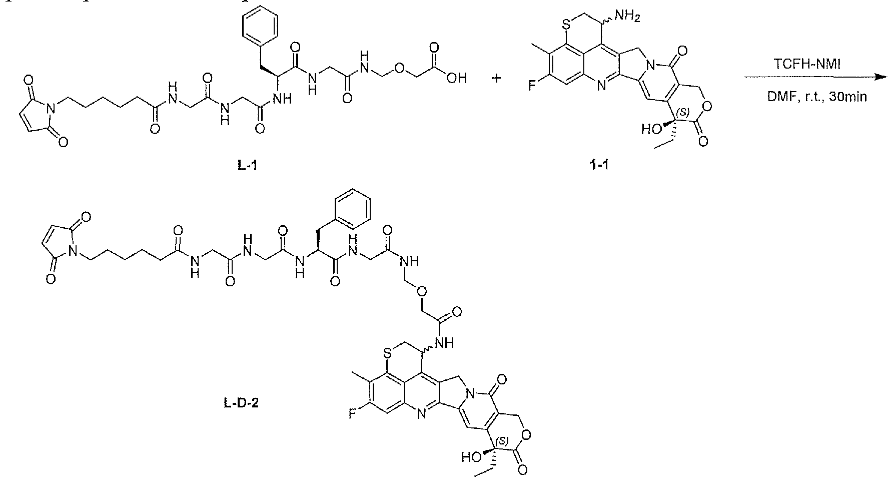

To a 50-mL single-necked flask were added **L-1** (76 mg, 0.12 mmol, 1.0 eq), component compound **1-1** (55 mg, 0.12 mmol, 1.0 eq) with a short retention time, NMI (50.6 mg, 0.62 mmol, 5.0 eq) and DMF (2 mL), and the system was stirred well and cooled to 0 °C. The reaction solution was added with TCFH (41.5 mg, 0.15 mmol, 1.2 eq) and stirred for 30 minutes, followed by detection by LC-MS. After the reaction was completed, the reaction solution was purified by reversed-phase C 18 column chromatography (MeCN/water = 0-60%), and fractions of the target substance were lyophilized to give a yellow solid **L-D-2** (80 mg, 61.5% yield).

¹H-NMR (400 MHz, DMSO-*d*₆) ¹H-NMR (400 MHz, DMSO-*d*₆) δ: 8.56 (t, *J* = 6.4 Hz, 1H), 8.30 (dd, *J* = 14.0, 8.3 Hz, 2H), 8.11 (d, *J* = 7.9 Hz, 1H), 8.06 (t, *J* = 5.7 Hz, 1H), 7.99 (t, *J* = 5.7 Hz, 1H), 7.76 (d, *J* = 10.7 Hz, 1H), 7.31 (d, *J* = 2.8 Hz, 1H), 7.30-7.16 (m, 5H), 6.98 (s, 2H), 6.54 (d, *J* = 1.8 Hz, 1H), 5.90-5.86 (m, 1H), 5.46-5.30 (m, 3H), 5.26-5.21 (m, 1H), 4.68-4.53 (m, 2H), 4.46-4.41 (m, 1H), 3.98 (s, 2H), 3.75-3.54 (m, 6H), 3.42 (s, 2H), 3.36-3.35 (m, 2H), 3.04-2.98 (m, 1H), 2.80-2.74 (m, 1H), 2.42 (s, 3H), 2.08 (t, *J* = *7.4,* 2H), 1.93-1.79 (m, 2H), 1.50-1.41 (dd, *J* = 13.2, 5.9 Hz, 4H), 1.25-1.12 (m, 2H), 0.88-0.84 (m, 3H), LC-MS: 1052.1 [M+H]⁺, 1050.1 [M-H]⁻.

### Step 2: Preparation of ADC-2

To an aqueous PBS buffer (0.05 PBS buffer at pH = 6.5; 2.5 mL, 9.96 mg/mL, 0.168 nmol) of antibody trastuzumab was added a prepared aqueous solution of tris(2-carboxyethyl)phosphine (10 mM, 0.082 mL) at 37 °C, and the system was placed in a water bath shaker, and shaken and reacted at 37 °C for 3 hours before the reaction was stopped; the reaction solution was cooled to 25 °C under a water bath and diluted to 5.0 mg/mL.

Compound **L-D-2** (2.02 nmol) was dissolved in DMSO (0.10 mL) and added to 2.0 mL of the above solution, and the system was placed in a water bath shaker, and shaken and reacted at 25 °C for 3 hours before the reaction was stopped. The reaction solution was desalted and purified through a Sephadex G25 gel column (elution phase: 0.05 M PBS buffer at pH 6.5, containing 0.001 M EDTA) to give a PBS buffer (5.0 mg/mL, 1.1 mL) ofADC, which was frozen and stored at 4 °C. Mean calculated by UV-HPLC: n = 7.2.

### Example 6: Preparation of Antibody-Drug Conjugates (ADC-3 and ADC-4)

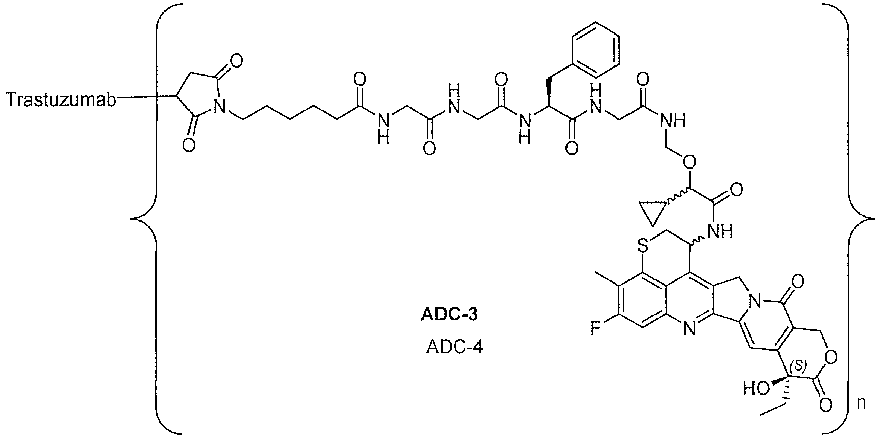

### Step 1: Preparation of compounds L-D-3 and L-D-4

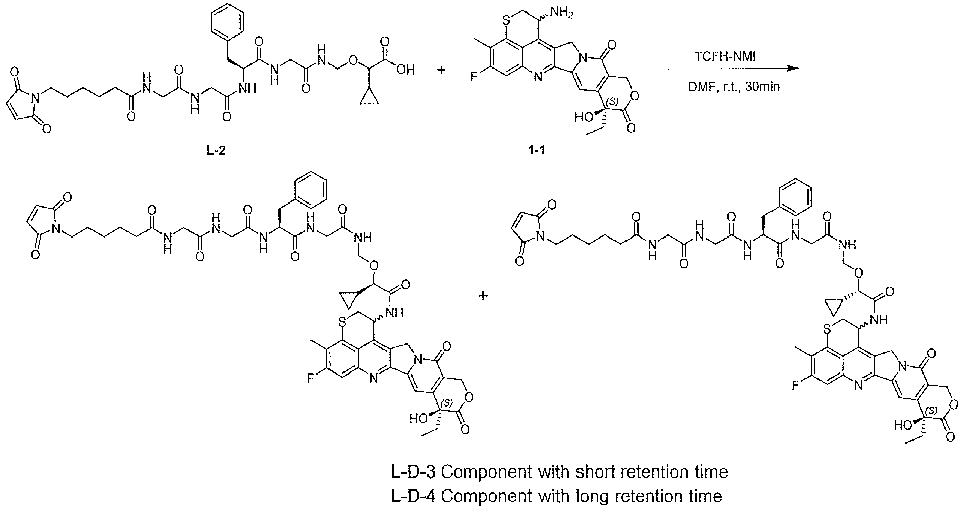

To a 50-mL single-necked flask were added **L-2** (80 mg, 0.12 mmol, 1.0 eq), component compound **1-1** (55 mg, 0.12 mmol, 1.0 eq) with a short retention time, NMI (50.6 mg, 0.62 mmol, 5.0 eq) and DMF (2 mL), and the system was stirred well and cooled to 0 °C. The reaction solution was added with TCFH (41.5 mg, 0.15 mmol, 1.2 eq) and stirred for 30 minutes, followed by detection by LC-MS. After the reaction was completed, the reaction solution was purified by reversed-phase C18 column chromatography to give two fractions, with the fraction with a short retention time being L-D-3 and the fraction with a long retention time being L-D-4. The fractions of the target substance were lyophilized to give yellow solids **L-D-3** (20 mg) and **L-D-4** (25 mg), LC-MS: 1092.4 [M+H]⁺.

Conditions for chromatography: the semi-preparative liquid chromatograph U3000 from Thermo Fisher; chromatographic column: Welch Ultimate XB-Phenyl; mobile phase: acetonitrile containing 0.1% formic acid-0.1% aqueous formic acid = 50:50; flow rate: 30.0 mL/min; detection wavelength: 370 nm; injection amount: 100 µL.

The experimental procedures were as follows: a proper amount of a mixture of **L-D-3** and **L-D-4** was taken and dissolved in DMF. A test sample solution with a concentration of 10 mg/mL was prepared. The test sample solution was taken and placed into the preparative liquid chromatograph for detection according to the conditions for chromatography of the present invention and then data were recorded. The injection was carried out for multiple times.

As a result, **L-D-3** and **L-D-4** were separated by pre-HPLC, where the retention times of the two components were 5.29 minutes and 5.87 minutes, respectively, and the purities thereof were 99.38% and 99.21%, respectively.

### Step 2: Preparation of ADC-3 and ADC-4

To an aqueous PBS buffer (0.05 PBS buffer at pH = 6.5; 2.5 mL, 9.96 mg/mL, 0.168 nmol) of antibody trastuzumab was added a prepared aqueous solution of tris(2-carboxyethyl)phosphine (10 mM, 0.082 mL) at 37 °C, and the system was placed in a water bath shaker, and shaken and reacted at 37 °C for 3 hours before the reaction was stopped; the reaction solution was cooled to 25 °C under a water bath and diluted to 5.0 mg/mL. Two aliquots were prepared in parallel. Compound **L-D-3** (2.0 nmol) was dissolved in DMSO (0.10 mL) and added to 2.0 mL of the above solution, and the system was placed in a water bath shaker, and shaken and reacted at 25 °C for 3 hours before the reaction was stopped. The reaction solution was desalted and purified through a Sephadex G25 gel column (elution phase: 0.05 M PBS buffer at pH 6.5, containing 0.001 M EDTA) to give a PBS buffer (5.0 mg/mL, 1 mL) of ADC, which was frozen and stored at 4 °C. Mean calculated by UV-HPLC: n = 7.3.

**ADC-4** was prepared by using compound **L-D-4** in the same manner, where n = 7.3.

### Example 7: Preparation of Antibody-Drug Conjugates (ADC-5 and ADC-6)

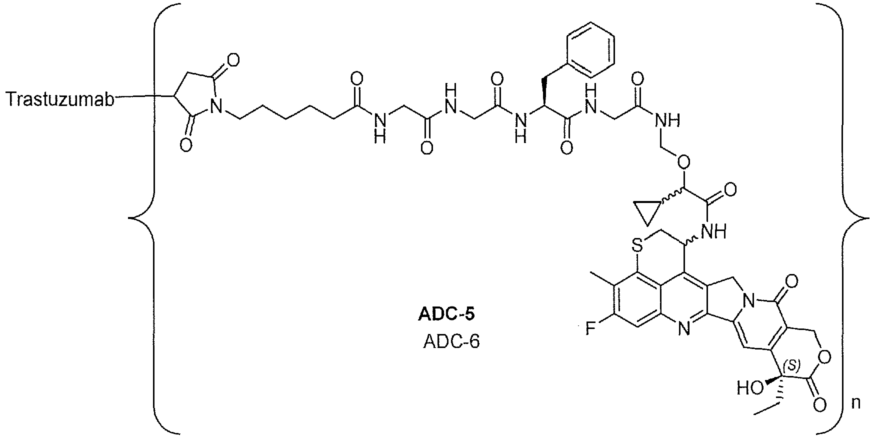

### Step 1: Preparation of compounds L-D-5 and L-D-6

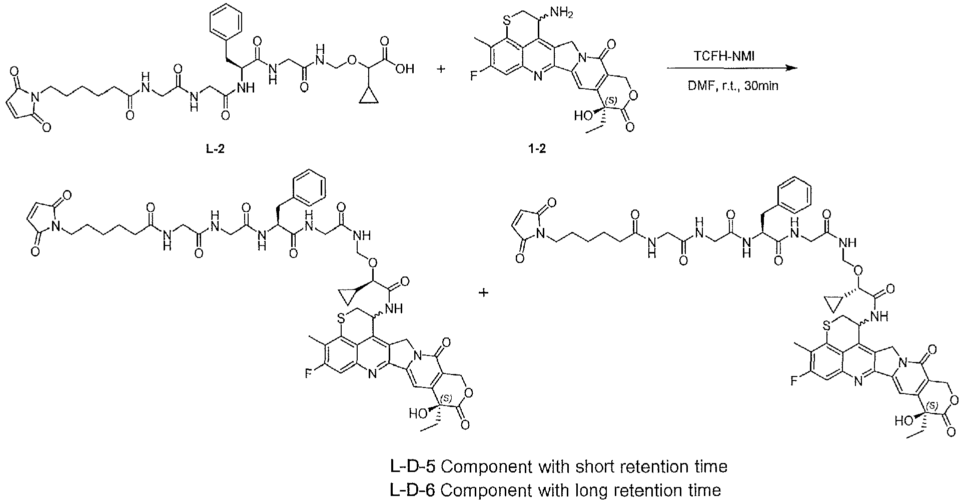

To a 50-mL single-necked flask were added **L-2** (80 mg, 0.12 mmol, 1.0 eq), component compound **1-2** (55 mg, 0.12 mmol, 1.0 eq) with a long retention time, NMI (50.6 mg, 0.62 mmol, 5.0 eq) and DMF (2 mL), and the system was stirred well and cooled to 0 °C. The reaction solution was added with TCFH (41.5 mg, 0.15 mmol, 1.2 eq) and stirred for 30 minutes, followed by detection by LC-MS. After the reaction was completed, the reaction solution was purified by reversed-phase C18 column chromatography to give two fractions, with the fraction with a short retention time being L-D-5 and the fraction with a long retention time being L-D-6. The fractions of the target substance were lyophilized to give yellow solids **L-D-5** (20 mg) and **L-D-6** (23 mg). LC-MS: 1092.4 [M+H]⁺.

Conditions for chromatography: the semi-preparative liquid chromatograph U3000 from Thermo Fisher; chromatographic column: Welch Ultimate XB-Phenyl; mobile phase: acetonitrile containing 0.1% formic acid-0.1% aqueous formic acid = 50:50; flow rate: 30.0 mL/min; detection wavelength: 370 nm; injection amount: 100 µL.

The experimental procedures were as follows: a proper amount of a mixture of **L-D-5** and **L-D-6** was taken and dissolved in DMF. A test sample solution with a concentration of 10 mg/mL was prepared. The test sample solution was taken and placed into the preparative liquid chromatograph for detection according to the conditions for chromatography of the present invention and then data were recorded. The injection was carried out for multiple times.

As a result, **L-D-5** and **L-D-6** were separated by pre-HPLC, where the retention times of the two components were 6.04 minutes and 6.48 minutes, respectively, and the purities thereof were 98.58% and 99.13%, respectively.

### Step 2: Preparation of ADC-5 and ADC-6

To an aqueous PBS buffer (0.05 PBS buffer at pH = 6.5; 2.5 mL, 9.96 mg/mL, 0.168 nmol) of antibody trastuzumab was added a prepared aqueous solution of tris(2-carboxyethyl)phosphine (10 mM, 0.082 mL) at 37 °C, and the system was placed in a water bath shaker, and shaken and reacted at 37 °C for 3 hours before the reaction was stopped; the reaction solution was cooled to 25 °C under a water bath and diluted to 5.0 mg/mL. Two aliquots were prepared in parallel. Compound **L-D-5** (2.0 nmol) was dissolved in DMSO (0.10 mL) and added to 2.0 mL of the above solution, and the system was placed in a water bath shaker, and shaken and reacted at 25 °C for 3 hours before the reaction was stopped. The reaction solution was desalted and purified through a Sephadex G25 gel column (elution phase: 0.05 M PBS buffer at pH 6.5, containing 0.001 M EDTA) to give a PBS buffer (5.0 mg/mL, 1.1 mL) ofADC, which was frozen and stored at 4 °C. Mean calculated by UV-HPLC: n = 7.3.

**ADC-6** was prepared by using compound **L-D-6** in the same manner, where n = 7.3.

### Example 8: Other ADCs

Other compounds similar to **L-D-1, L-D-2, L-D-3, L-D-4, L-D-5** or **L-D-6** (the camptothecin derivative of the present application is a small molecule toxin) can be prepared in the same manner. **L-D-1, L-D-2, L-D-3, L-D-4, L-D-5** or **L-D-6** and similar compounds can be further combined with antibody trastuzumab or other similar antibodies to prepare an antibody-drug conjugate comprising the camptothecin derivative of the present application as a small molecule toxin.

### Example 9: Assay for Antiproliferative Activity against SK-BR-3 Cells

The activity of the antibody-drug conjugate of the present invention can be determined by the assay for the *in-vitro* antiproliferative activity against SK-BR-3 cells of a camptothecin derivative as a small molecule toxin.

SK-BR-3 cells were seeded into a 384-well plate (Fisher 142762) at 3000 cells per well. The next day, serially diluted compounds were added, and 72 hours after the addition, CellTiter-Lumi (Beyotime C0068XL) was added to measure the ATP content in the cells. The growth of the cells was evaluated, and relative IC₅₀ values of the compounds against cell growth was calculated. The screening results are shown in Table 6.

**Table 6. Antiproliferative activity of the compounds of the present invention against SK-BR-3 cells**

| No. | IC₅₀ (nM) | No. | IC₅₀ (nM) |
|---|---|---|---|
| **1** | 2.98 | **1-1** | 5.81 |
| **1-2** | 1.73 | **3-2** | 2.85 |
| **4-2** | 2.74 | **7-2** | 2.68 |
| **14-2** | 4.13 | **20** | 5.83 |
| **32** | 4.30 | **34** | 5.84 |
| **35** | 6.53 | **39** | 4.78 |
| **41** | 6.47 | **44** | 6.29 |
| **48** | 6.73 | **49** | 7.32 |
| **Exatecan** | 5.35 | **Deruxtecan** | 13.61 |
| **Topotecan** | 58.30 | | |

Compared with exatecan, deruxtecan and topotecan which is a camptothecin medicament on the market, the compounds of the present invention have strong *in-vitro* antiproliferative activity against SK-BR-3 cells, and particularly when X in general formula (1) is S or O, have strong antiproliferative activity against cells. For example, compound **1-2** is 2-fold more active than exatecan, and compound **32** is 3-fold more active than deruxtecan. In particular, the compound of general formula (1), containing easily attached groups such as OH or NH₂ in the side chains and having strong cell activity, is suitable to be used as a small molecule toxin of ADCs.

### Example 10: In-Vitro Anti-Tumor Activity of Antibody-Drug Conjugates of the Present Invention

SK-BR-3 cells with high expression of HER2 were selected as cell strains for *in-vitro* activity detection in the experiment and were used for evaluating the dose-effect relationship of the antibody-drug conjugates (ADCs) of the present invention on cell killing. The plating density for each type of cells was initially selected to be 1500-2000 cells/well, and the assay for cell cytotoxicity was performed after 12 hours; the ADCs were added at 10 nM as the starting concentration for a 3-10 fold serial dilution to give a final concentration, the killing effect was then observed for 144 hours, CellTiter-Glo@ Luminescent Cell Viability Assay was used for chemiluminescent staining, and IC₅₀ was calculated after the fluorescence data were read. From the results of the activity test, all of the ADCs showed certain anti-tumor activity, and the activity of part of the ADCs was greater than that of DS-8201a.

**Table 7. Antiproliferative activity of the antibody-drug conjugates of the present invention against SK-BR-3 cells**

| Sample | IC₅₀ (nM) |
|---|---|
| ADC-1 | 0.26 |
| ADC-2 | 0.13 |
| ADC-3 | 0.08 |
| ADC-4 | 0.06 |
| ADC-5 | 0.02 |
| ADC-6 | 0.10 |
| Exatecan | 0.73 |
| DS-8201a | 0.09 |

From the results of the activity test, all of the ADCs showed certain anti-tumor activity, and the activity of part of the ADCs was greater than that of DS-8201a.

### Example 11: In-Vivo Anti-Tumor Activity of Antibody-Drug Conjugates of the Present Invention

Human gastric carcinoma cells (NCI-N87) dissolved in 100 µL of PBS solution were injected subcutaneously into the right sides of the necks or dorsa of female Balb/c nude mice aged 6-8 weeks. When the average tumor volume was about 150-200 mm³, the 32 nude mice were randomly divided into 4 groups according to the tumor size, with 8 animals in each group, and injection administration was performed via tail veins, where 01 was blank control group, 02 was DS-8201a (4.5 mg/kg) group, 03 was ADC-1 (4.5 mg/kg) group, and 04 was ADC-2 (4.5 mg/kg) group. The weight and the tumor volume of the experimental animals were measured twice a week and the survival state of the animals in the experimental process was observed, wherein the specific results of the change in the tumor volume of each group are shown in FIG. 1.

As can be seen from FIG. 1, both of the ADC samples of the present invention showed *in-vivo* anti-tumor activity comparable to DS-8201a.

Although specific embodiments of the present invention have been described above, it will be appreciated by those skilled in the art that these embodiments are merely illustrative and that many changes or modifications can be made to these embodiments without departing from the principles and spirit of the present invention. The scope of protection of the present invention is therefore defined by the appended claims.

## Claims

1. A camptothecin derivative compound of general formula (1) or optical isomers, crystalline forms, pharmaceutically acceptable salts, hydrates or solvates thereof: 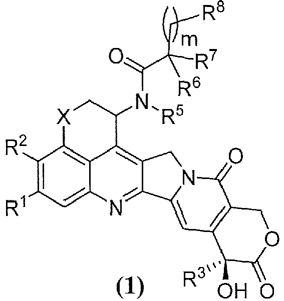 wherein, in general formula (1):
m is an integer of 0, 1 or 2;
X is selected from -O-, -S-, -S(O)-, -S(O₂)-, and -N(R⁴)-;
R¹ and R² are independently selected from H, halogen, OH, C₁₋₆ alkyl, C₁₋₆ alkoxy, C₁₋₆ haloalkyl, C₃₋₆ cycloalkyl, NH₂, NO₂, and CN, or R¹ and R², together with the phenyl ring attached thereto, form 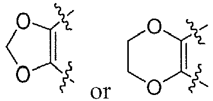 by cyclization;
R³ is selected from C₁₋₆ alkyl, C₂₋₆ alkenyl, C₁₋₃ alkoxy-substituted C₁₋₃ alkyl, and C₁₋₆ haloalkyl;
R⁴ is selected from H, C₁₋₆ alkyl and C₁₋₆ haloalkyl;
R⁵ is selected from H, C₁₋₆ alkyl and C₃₋₆ cycloalkyl;
R⁶ and R⁷ are independently selected from H, C₁₋₆ alkyl, C₁₋₆ haloalkyl, and C₃₋₆ cycloalkyl; or
R⁶ and R⁷, together with the carbon atom attached thereto, form C₃₋₆ cycloalkyl or 4-7 membered heterocycloalkyl by cyclization; or R⁶ and R⁵ are linked to form a 5-7 membered lactam ring, and R⁷ is selected from H, C₁₋₆ alkyl, C₁₋₆ haloalkyl, and C₃₋₆ cycloalkyl;
R⁸ is selected from OH and NR⁹R¹⁰, and R⁹ and R¹⁰ are independently selected from H, C₁₋₆ alkyl and C₃₋₆ cycloalkyl; or R⁹ and R¹⁰, together with the N atom attached thereto, form 4-7 membered heterocycloalkyl by cyclization, and the 4-7 membered heterocycloalkyl is unsubstituted or substituted with 1-3 groups selected from the following: C₁₋₆ alkyl, halogen, OH, CN, and NH₂.

2. The camptothecin derivative compound of formula (1) or the optical isomers, the crystalline forms, the pharmaceutically acceptable salts, the hydrates or the solvates thereof according to claim 1, wherein in the formula (1), R⁸ is OH.

3. The camptothecin derivative compound of formula (1) or the optical isomers, the crystalline forms, the pharmaceutically acceptable salts, the hydrates or the solvates thereof according to any one of claims 1 to 2, wherein in the formula (1), R¹ and R² are independently selected from H, halogen, OH, Me, Et, OMe, OEt, CF₃, NH₂, NO₂, and CN; or R¹ and R², together with the phenyl ring attached thereto, form 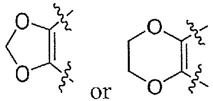 by cyclization.

4. The camptothecin derivative compound of formula (1) or the optical isomers, the crystalline forms, the pharmaceutically acceptable salts, the hydrates or the solvates thereof according to any one of claims 1 to 3, wherein in the formula (1), R³ is selected from Me, Et,

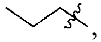

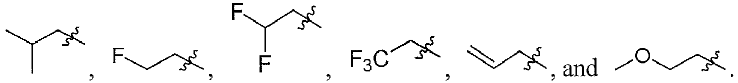

5. The camptothecin derivative compound of formula (1) or the optical isomers, the crystalline forms, the pharmaceutically acceptable salts, the hydrates or the solvates thereof according to any one of claims 1 to 4, wherein in the formula (1), X is selected from -O-, -S-, -S(O)-, -S(O₂)-, -N(H)-, and -N(Me)-.

6. The camptothecin derivative compound of formula (1) or the optical isomers, the crystalline forms, the pharmaceutically acceptable salts, the hydrates or the solvates thereof according to any one of claims 1 to 5, wherein in the formula (1), R⁵ is selected from H, Me, Et, and

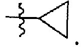

7. The camptothecin derivative compound of formula (1) or the optical isomers, the crystalline forms, the pharmaceutically acceptable salts, the hydrates or the solvates thereof according to any one of claims 1 to 6, wherein in the formula (1), R⁶ and Rare independently selected from H, Me, Et, CHF₂, CF₃, CH₂CF₃, 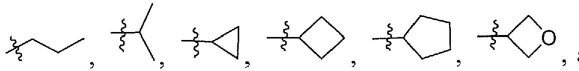 and

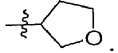

8. The camptothecin derivative compound of formula (1) or the optical isomers, the crystalline forms, the pharmaceutically acceptable salts, the hydrates or the solvates thereof according to claim 1, wherein in the formula (1), 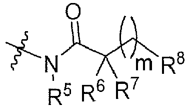 is selected from 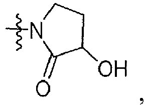 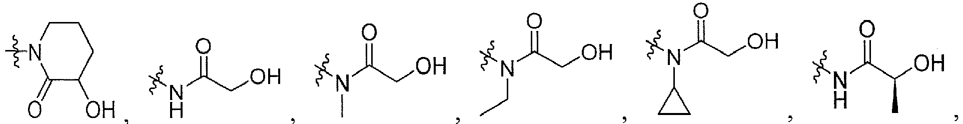 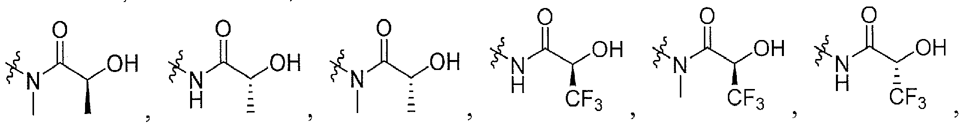 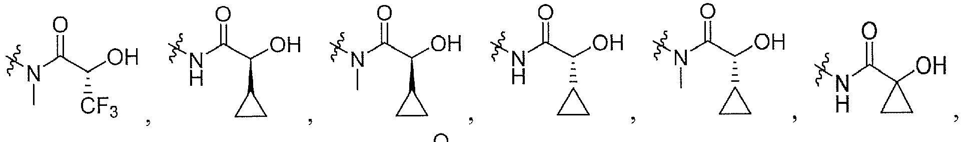 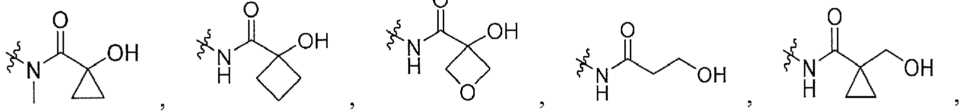 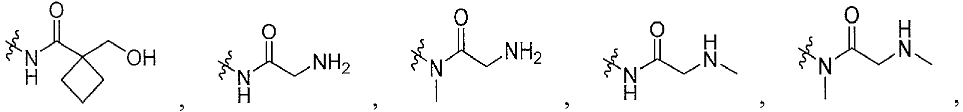 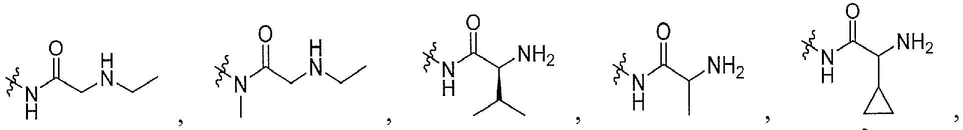 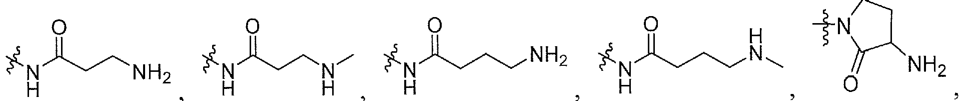 and

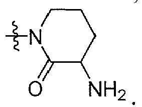

9. The camptothecin derivative compound of formula (1) or the optical isomers, the crystalline forms, the pharmaceutically acceptable salts, the hydrates or the solvates thereof according to claim 2, wherein in the formula (1), R⁸ is OH, and 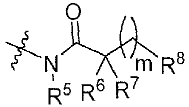 is selected from

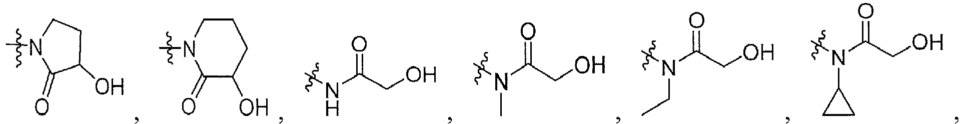

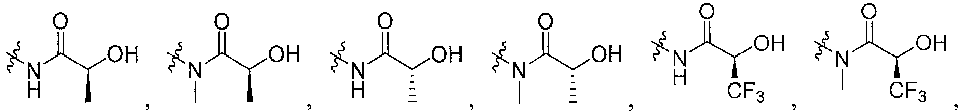

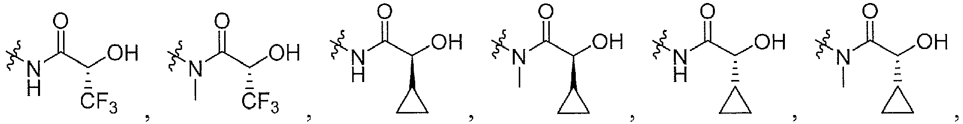

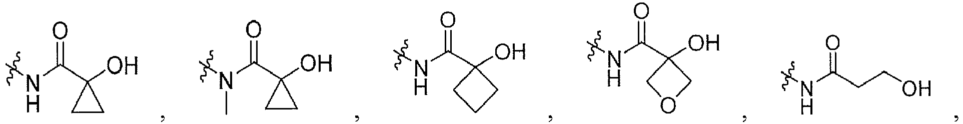

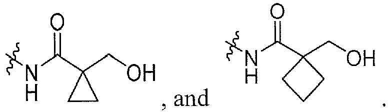

10. The camptothecin derivative compound of formula (1) or the optical isomers, the crystalline forms, the pharmaceutically acceptable salts, the hydrates or the solvates thereof according to any one of claims 1 to 9, wherein the compound has one of the following structures:

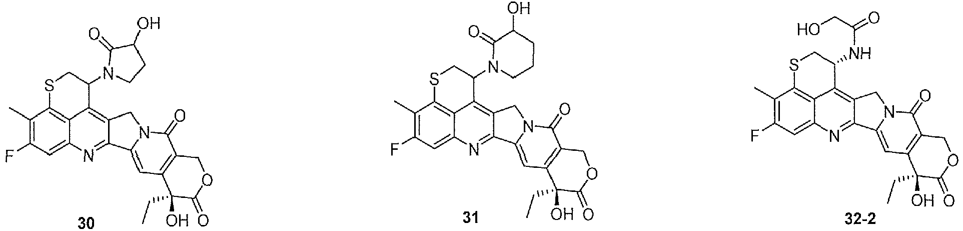

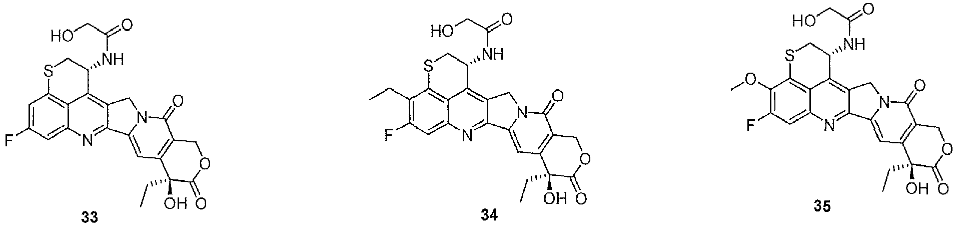

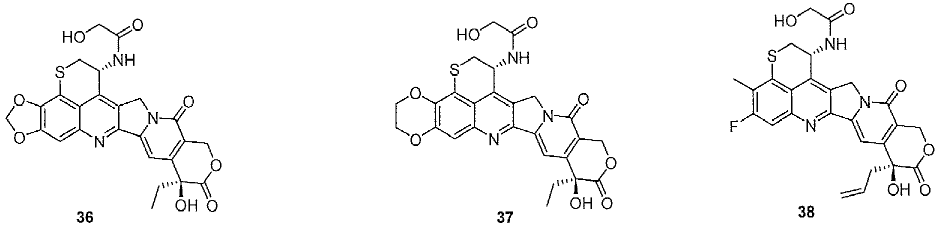

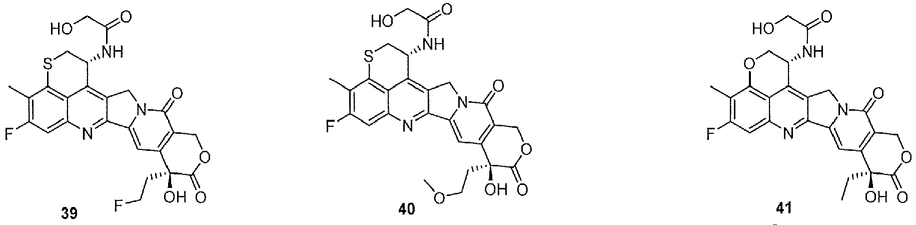

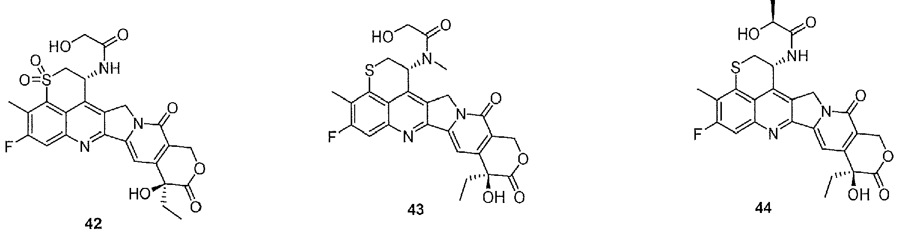

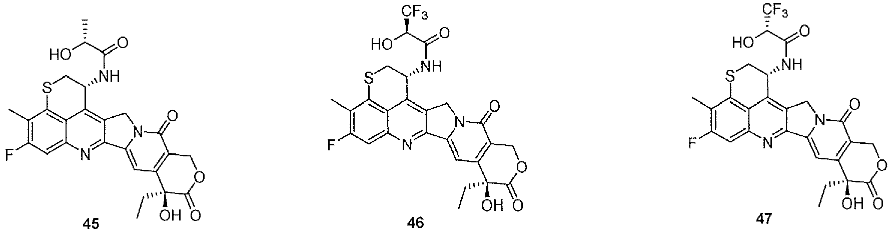

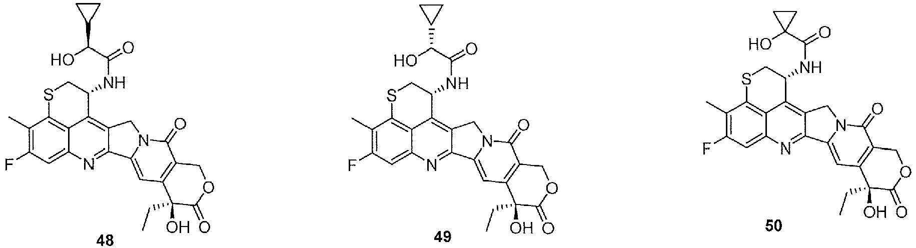

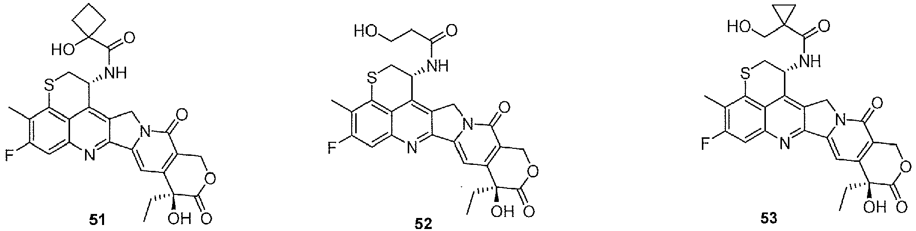

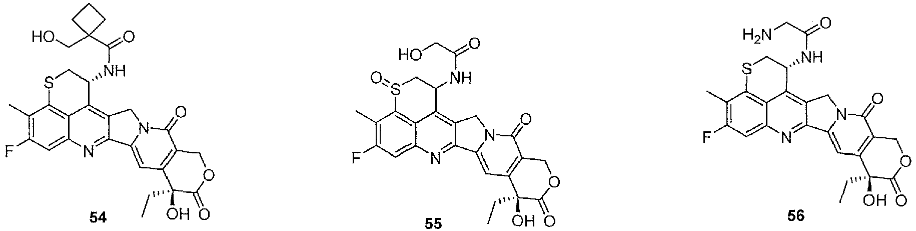

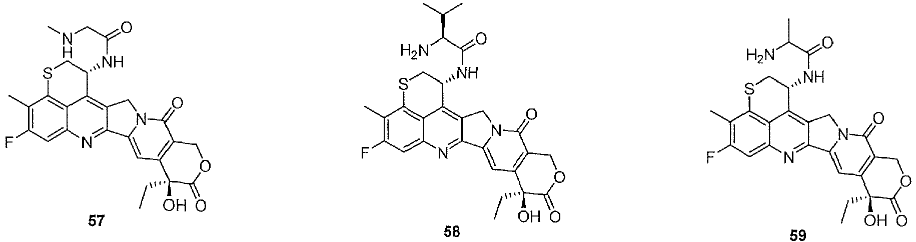

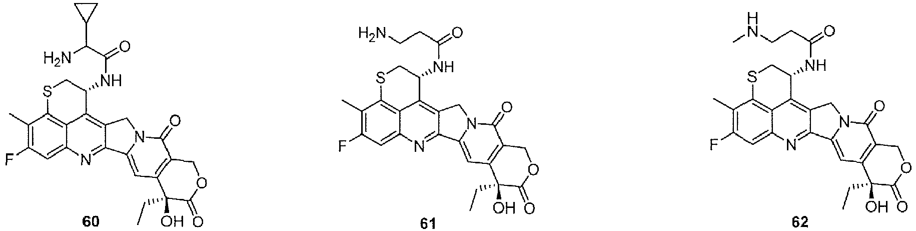

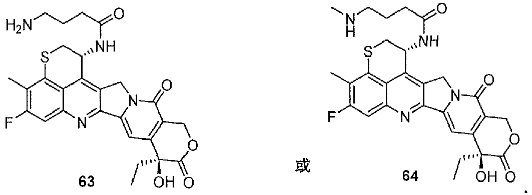

11. The camptothecin derivative compound of formula (1) or the optical isomers, the crystalline forms, the pharmaceutically acceptable salts, the hydrates or the solvates thereof according to any one of claims 1 to 9, wherein the compound has one of the following structures:

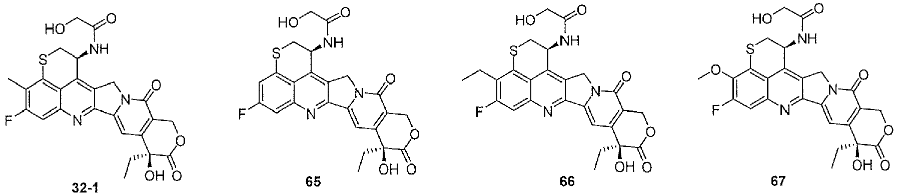

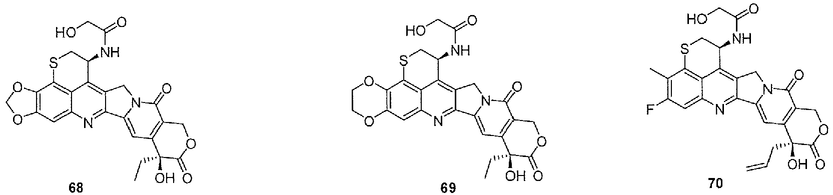

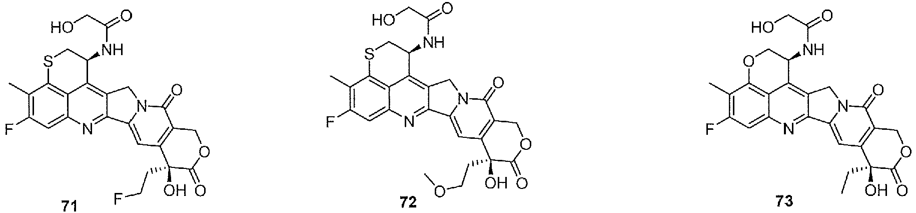

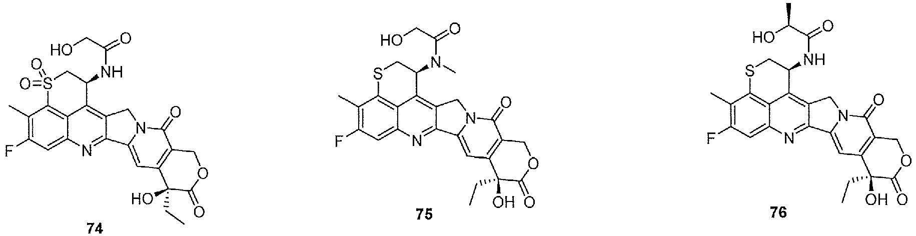

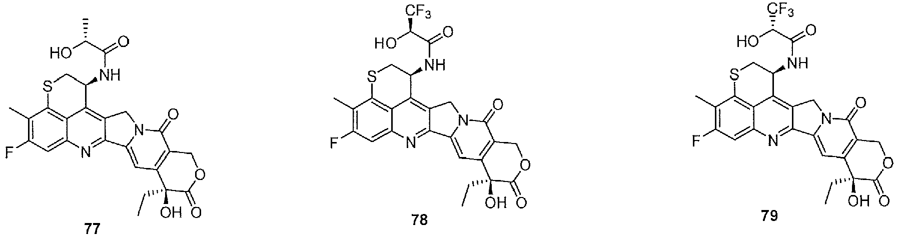

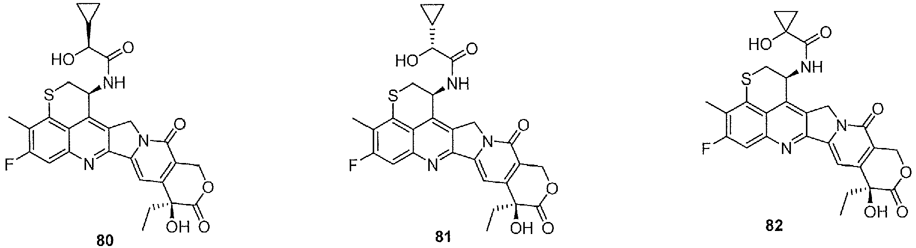

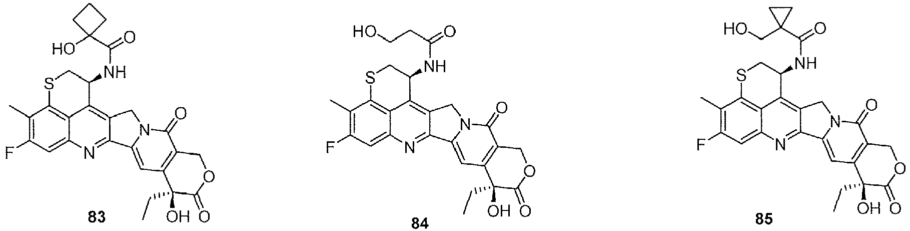

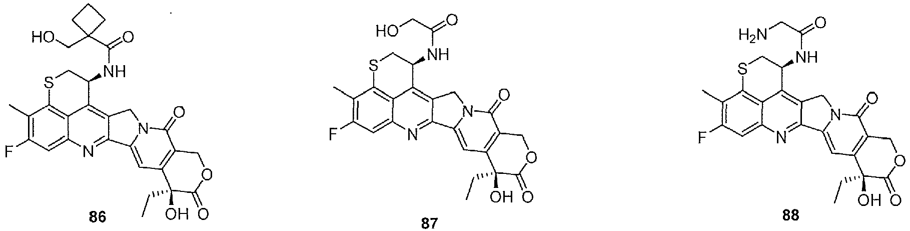

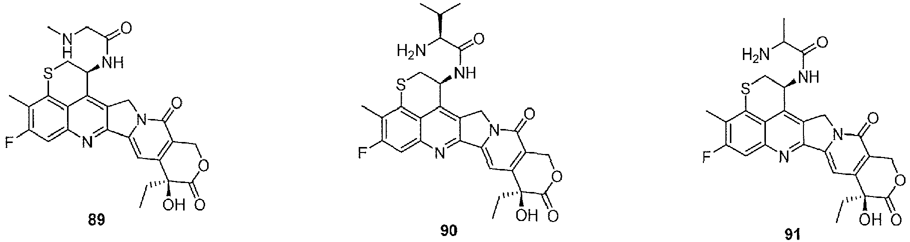

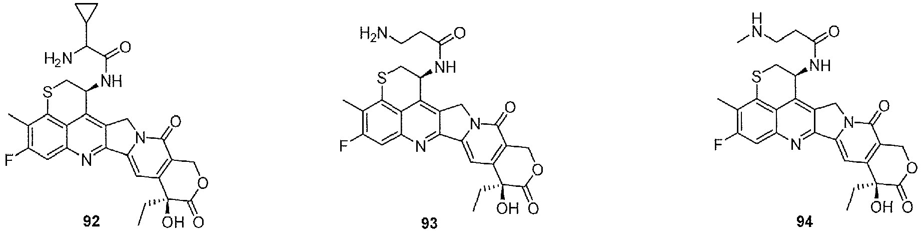

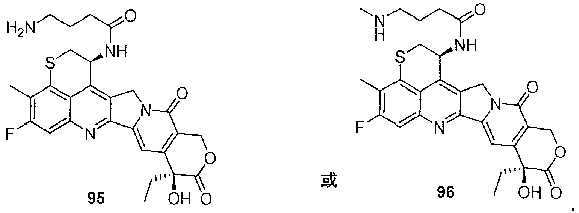

12. A camptothecin derivative compound or optical isomers, crystalline forms, pharmaceutically acceptable salts, hydrates or solvates thereof, wherein the compound has one of the following structures:

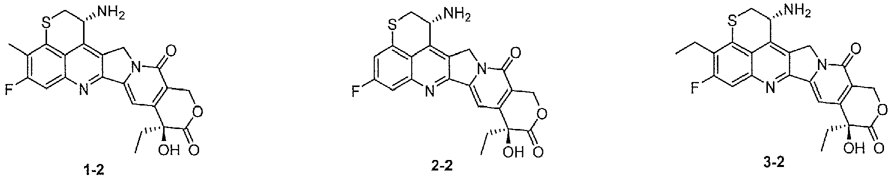

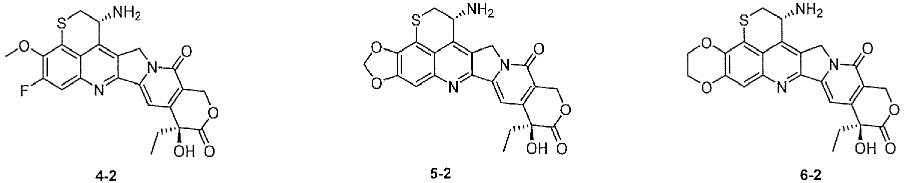

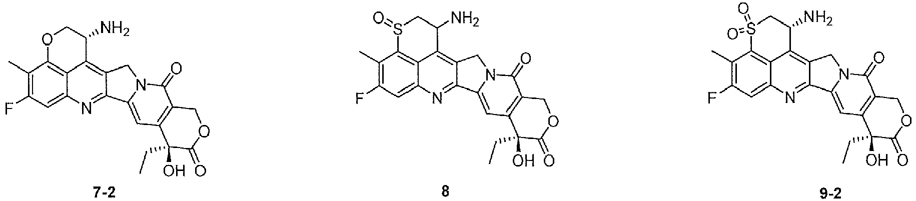

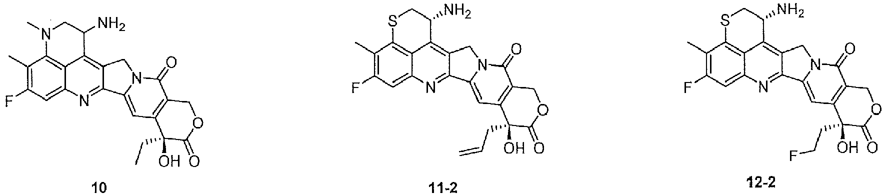

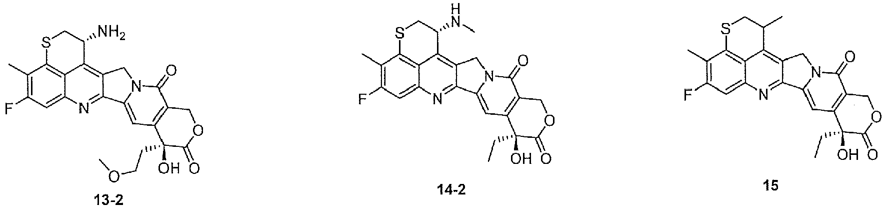

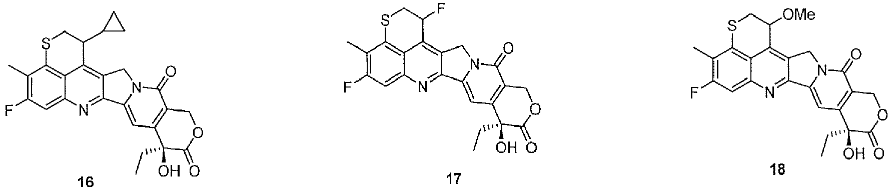

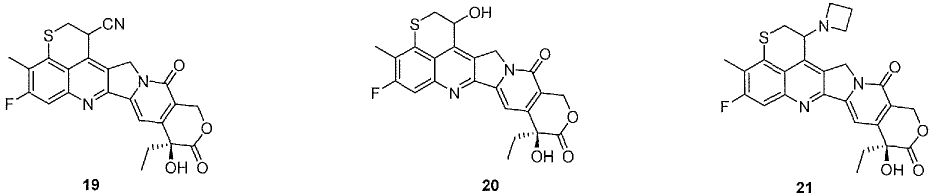

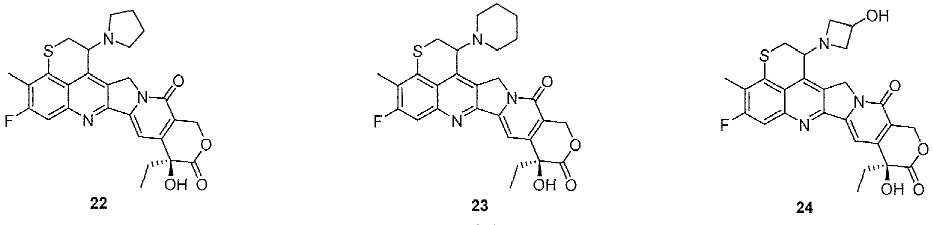

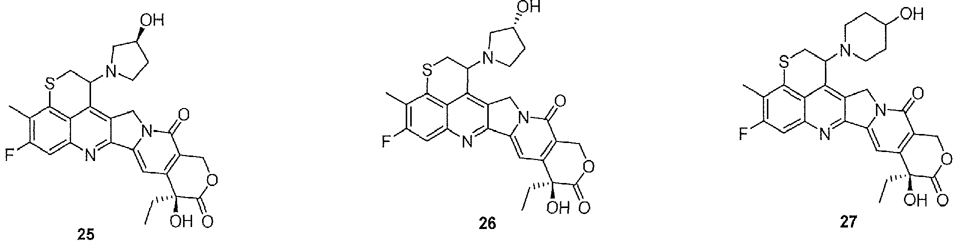

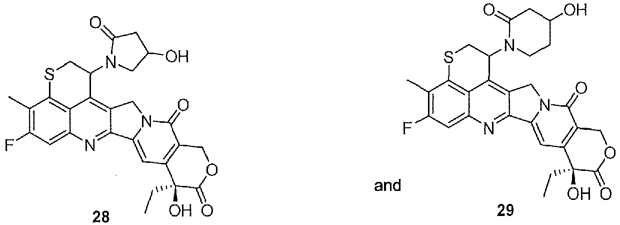

13. A camptothecin derivative compound or optical isomers, crystalline forms, pharmaceutically acceptable salts, hydrates or solvates thereof, wherein the compound has one of the following structures:

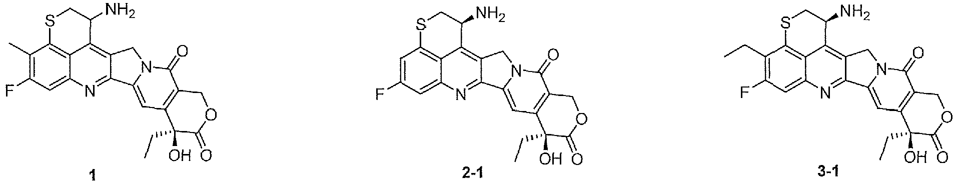

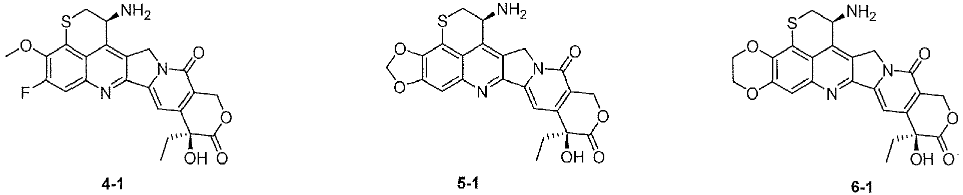

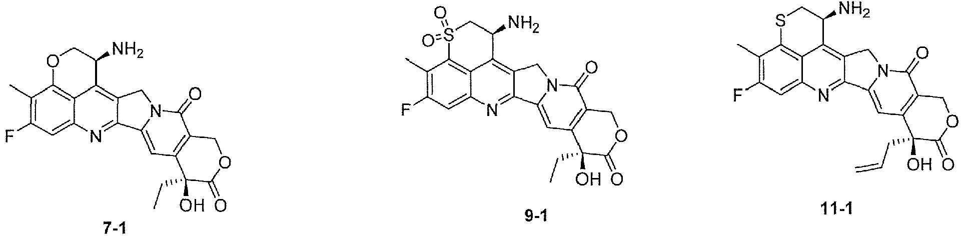

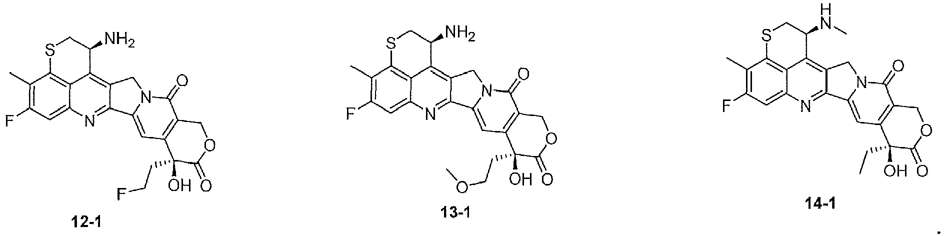

14. Use of the camptothecin derivative compound of general formula (1) or the optical isomers, the crystalline forms, the pharmaceutically acceptable salts, the hydrates or the solvates thereof according to any one of claims 1 to 13 as a small molecule toxin in the preparation of an antibody-drug conjugate.

15. A pharmaceutical composition, comprising a pharmaceutically acceptable excipient or carrier, and the camptothecin derivative compound of general formula (1) or the optical isomers, the crystalline forms, the pharmaceutically acceptable salts, the hydrates or the solvates thereof according to any one of claims 1 to 13 as an active ingredient.

16. Use of the camptothecin derivative compound of general formula (1) or the optical isomers, the crystalline forms, the pharmaceutically acceptable salts, the hydrates or the solvates thereof according to any one of claims 1 to 13, or the pharmaceutical composition according to claim 15 in the preparation of a medicament for treating cancer diseases.

17. An antibody-drug conjugate, comprising an antibody, a small molecule toxin, and a linker, wherein the small molecule toxin is the camptothecin derivative compound of general formula (1) or the optical isomers, the crystalline forms, the pharmaceutically acceptable salts, the hydrates or the solvates thereof according to any one of claims 1 to 13, and the linker links the antibody and the small molecule toxin via a covalent bond.

18. The antibody-drug conjugate according to claim 17, wherein the antibody-drug conjugate has one of the following structures: 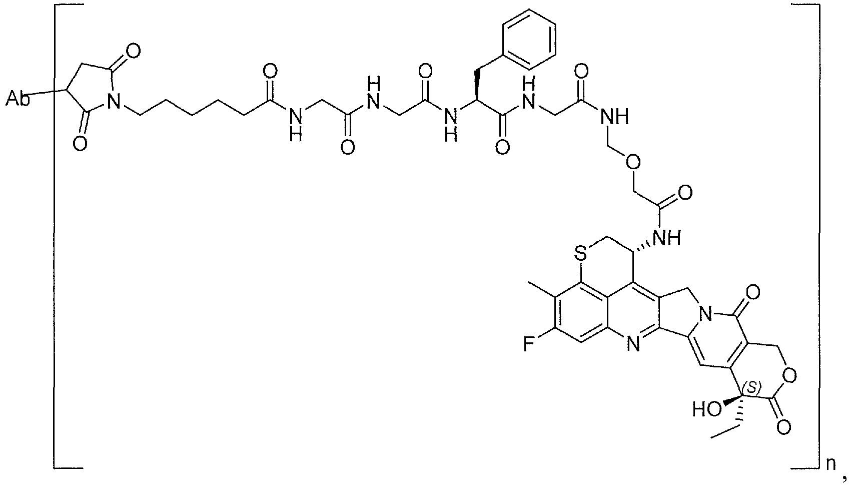 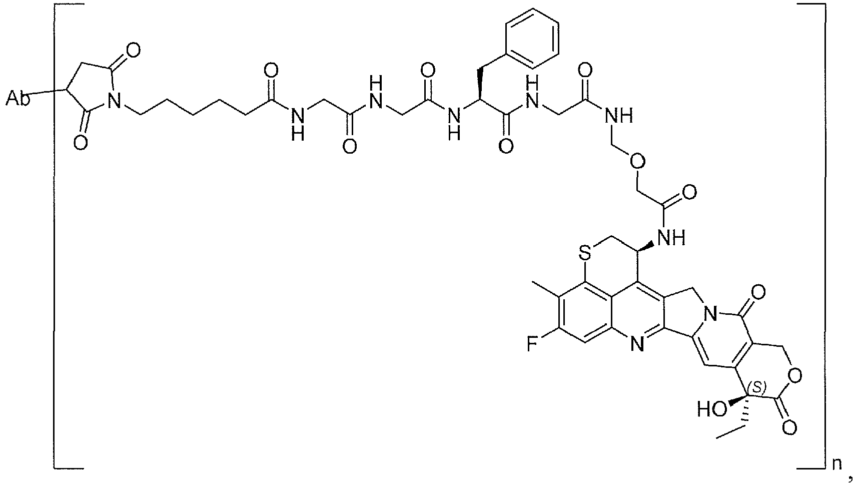 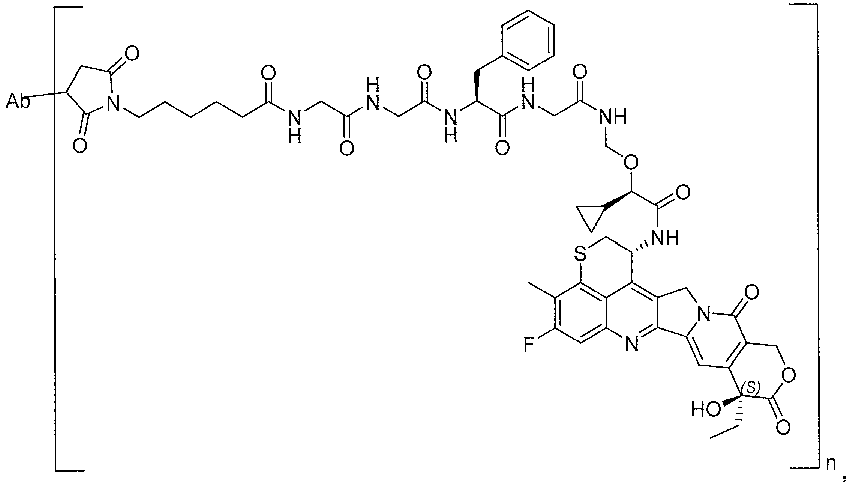 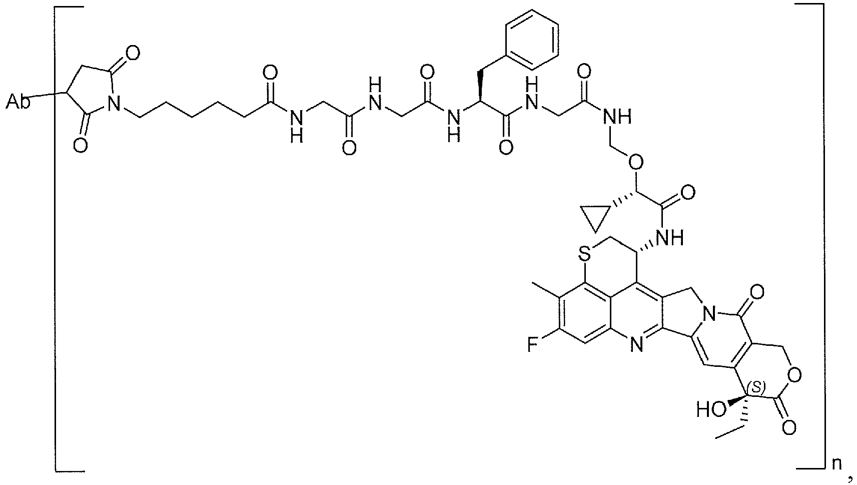 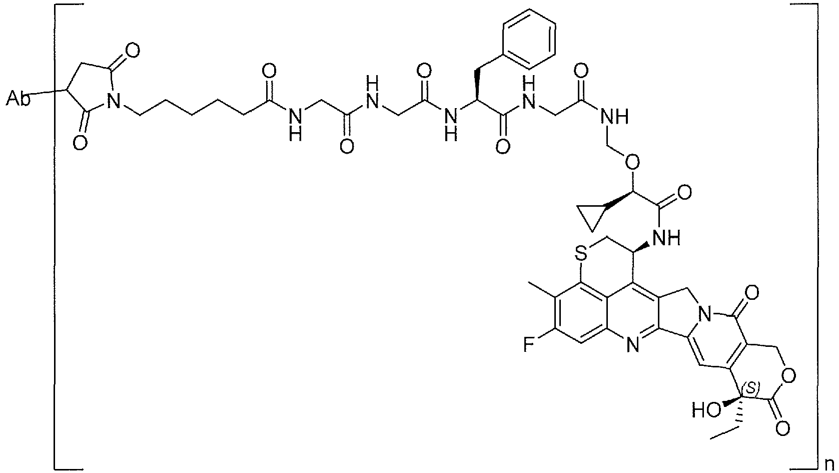 and 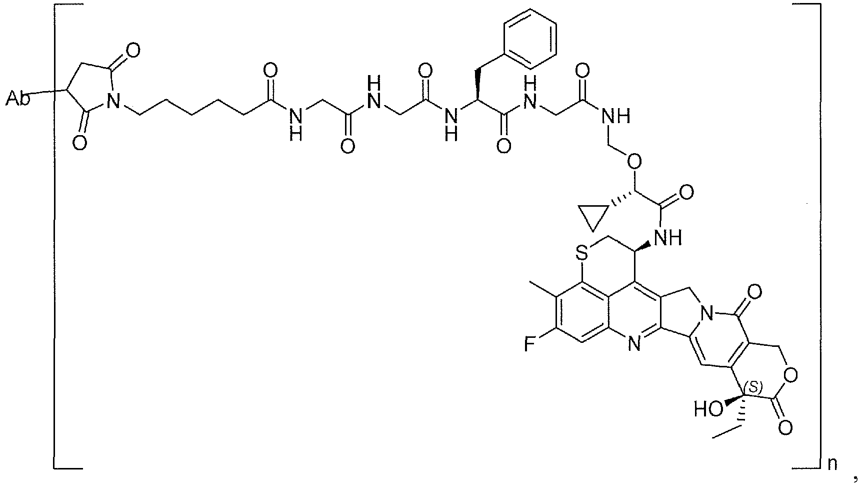 wherein
Ab represents a monoclonal antibody, preferably an anti-her2 antibody, and more preferably trastuzumab; n is a number from 2 to 8, preferably from 4 to 8, and more preferably from 7 to 8.
